# EUROPEAN PATENT APPLICATION

(11) **EP 4 624 464 A1**
(43) Date of publication of application: **01.10.2025**
(21) Application number: 23893974.8
(22) Date of filing: 23.11.2023
(51) Int. Cl.: C07D 401/10, C07D 417/10, C07D 413/10, C07D 471/06, A61K 31/4709, A61K 31/444, A61P 9/12

(54) **PYRIDINE POLYCYCLIC COMPOUND INHIBITOR, PREPARATION METHOD THEREFOR AND USE THEREOF**

(30) Priority: 23.11.2022 CN 202211477961; 11.04.2023 CN 202310384096; 26.05.2023 CN 202310611392
(71) Applicant: Shanghai Hansoh Biomedical Co., Ltd., Shanghai 201203 (CN); Jiangsu Hansoh Pharmaceutical Group Co., Ltd., Lianyungang, Jiangsu 222069 (CN)
(72) Inventor: SU, Yidong, Shanghai 201203 (CN); MAO, Xiaofeng, Shanghai 201203 (CN); YU, Wensheng, Shanghai 201203 (CN); WANG, Jun, Shanghai 201203 (CN); LI, Kailong, Shanghai 201203 (CN)
(74) Representative: Murgitroyd & Company
(86) International application number: PCT/CN2023/133689
(87) International publication number: WO 2024/109885

(57) **Abstract**

The present invention relates to a pyridine polycyclic compound inhibitor, a preparation method therefor and a use thereof. **In** particular, the present invention relates to a compound represented by formula (I), a preparation method therefor, a pharmaceutical composition comprising the compound, and a use of the compound in a medication for treating chronic kidney disease, kidney or cardiac fibrosis, diabetic nephropathy, congestive heart failure, hypertension, primary aldosteronism, and Cushing syndrome, wherein the definitions of the substituents in formula (I) are the same as those in the description.

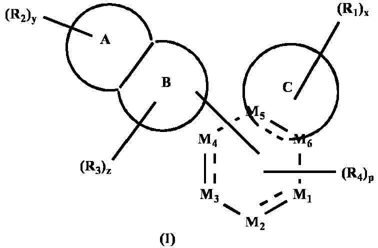

## Description

The present application claims the priority of Chinese Patent Application 2022114779612 filed on November 23, 2022. The above-mentioned Chinese patent application is incorporated in the present application by reference in its entirety.

### Technical Field

The present invention belongs to the field of biomedicine, and specifically relates to a pyridine polycyclic compound inhibitor, a preparation method therefor, and a use thereof.

### Background Art

Aldosterone, a steroid hormone secreted from adrenal gland, binds and activates mineralocorticoid receptor (MR). In primary cells of distal renal tubules and renal collecting ducts, MR activation results in sodium and water retention accompanied with potassium excretion, leading to plasma volume expansion and elevated blood pressure (BP). Renin-angiotensin-aldosterone system (RAAS), as an endocrine system, regulates human blood pressure and body fluid balance. As current antihypertensive drugs, angiotensin-converting enzyme inhibitors (ACEi), angiotensin II receptor antagonists (ARBs), and mineralocorticoid receptor antagonists (MRAs) regulate blood pressure by inhibiting this pathway. Patients on long-term use of ACEi or ARB drugs develop "aldosterone breakthrough". The aldosterone level increases after a brief decrease, leading to damage to the target organ. At present, among aldosterone inhibitors currently available on the market, only spironolactone resutls in hyperkalemia. Excessive aldosterone measured in the circulation is known as primary aldosteronism (PA), which occurs when the renin-angiotensin-aldosterone system (RAAS) dysregulates aldosterone production. PA was first found in patients with adrenal adenoma. Recent evidences have shown that the prevalence of obesity-associated PA is increasing. PA is a common cause of secondary hypertension. The prevalence thereof is 14-21% of that of patients with refractory hypertension (RHTN). The refractory hypertension means that the blood pressure is still higher than the target blood pressure 140/90 mm Hg despite the use of three antihypertensive drugs (calcium channel blockers, angiotensin-converting enzyme inhibitors, angiotensin receptor blockers, and diuretics). Refractory hypertension is a high-risk disease, which has a high comorbidity rate with diabetes, chronic nephropathy, ischemic heart disease, and cerebrovascular diseases.

CYP11B2 is a gene encoding aldosterone synthase and is highly homologous to the sequence of CYP11B1 gene encoding cortisol synthase. Developing inhibitors with a high selectivity for CYP11B2 to inhibit aldosterone synthesis is the main direction for treating refractory hypertension and primary aldosteronism.

### Summary of the Invention

An object of the present invention is to provide a compound represented by general formula (I), or a stereoisomer or a pharmaceutically acceptable salt thereof: 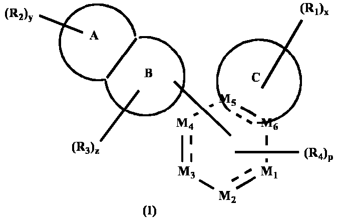 wherein: 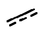
is a single bond or a double bond;
ring A is phenyl, 4- to 7-membered heterocyclyl, 5- to 6-membered heteroaryl, or absent;
ring B is 4- to 7-membered heterocyclyl, 5- to 6-membered heteroaryl, or phenyl;
ring C is cycloalkyl, heteroaryl, heterocyclyl, or absent;
M₁, M₂, M₄, M₅, and M₆ are each independently selected from N, NH, or CH;
M₃ is a bond, N, or CH;
each R₁ is independently selected from cycloalkyl, heterocyclyl, cycloalkyloxy, heterocyclyloxy, cycloalkylamino, heterocyclylamino, cycloalkylthio, heterocyclylthio, -C(O)(CH₂)ₙR_{b}, -NRₐC(O)(CH₂)ₙR_{b}, -O(CH₂)ₙR_{b}, -NH(CH₂)ₙR_{b},-S(CH₂)ₙR_{b}, -S(O)₂(CH₂)ₙR_{b}, -S(O)(NH)(CH₂)ₙR_{b}, -NRₐS(O)₂(CH₂)ₙR_{b},-NRₐS(O)(NH)(CH₂)ₙR_{b}, or -NS(O)(CH₂)ₙRₐR_{b}, wherein optionally the cycloalkyl, heterocyclyl, cycloalkyloxy, heterocyclyloxy, cycloalkylamino, heterocyclylamino, cycloalkylthio, or heterocyclylthio is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, hydroxyalkyl, - C(O)(CH₂)ₙR_{b}, -S(O)₂(CH₂)ₙR_{b}, -S(O)(NH)(CH₂)ₙR_{b}, -S(O)₂(CH₂)ₙR_{b}, or - NRₐS(O)(NH)(CH₂)ₙR_{b};
or any two R₁, together with adjacent carbon atoms, form 3- to 8-membered cycloalkyl or 4- to 7-membered heterocyclyl, wherein optionally the 3- to 8-membered cycloalkyl or 4- to 7-membered heterocyclyl is further substituted with oxo, -C(O)R_{b}, -NRₐC(O)R_{b}, -S(O)₂R_{b}, -S(O)(NH)R_{b}, -NRₐS(O)₂R_{b}, or-NRₐS(O)(NH)R_{b};
Rₐ or R_{b} is each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, aryl, heteroaryl, or heterocyclyl, wherein optionally the cycloalkyl, aryl, heteroaryl, or heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, or -S(O)₂alkyl;
R₂, R₃, or R₄ is each independently selected from hydrogen, deuterium, oxo, halogen, amino, hydroxyl, cyano, nitro, alkyl, alkenyl, alkynyl, deuteroalkyl, haloalkyl, alkoxy, haloalkoxy, hydroxyalkyl, cycloalkyl, heterocyclyl, aryl, or heteroaryl, wherein the alkyl, alkenyl, alkynyl, deuteroalkyl, haloalkyl, alkoxy, haloalkoxy, hydroxyalkyl, cycloalkyl, heterocyclyl, aryl, heteroaryl,-NRₐC(O)(CH₂)ₙR_{b}, or -C(O)NRₐ(CH₂)ₙR_{b} can be optionally further substituted;
or R₂ and R₃, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
or any two R₂, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
or R₂ and R₄, together with adjacent atoms, form 5- to 14-membered cycloalkyl, 5- to 14-membered heteroaryl, or 5-14-membered heterocyclyl, wherein optionally the 5- to 14-membered cycloalkyl, 5- to 14-membered heteroaryl, or 5-14-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
p, x, y, and z are each independently selected from 1, 2, 3, or 4; and
n is selected from 0, 1, 2, or 3;
when 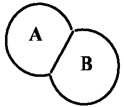 is 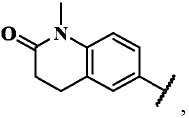 M₁ is N, and ring C is 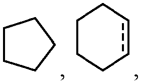 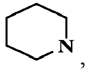 R₁ is not -NRₐC(O)R_{b} and -NRₐS(O)₂R_{b}; and
when M₁ is N, ring C is absent or is 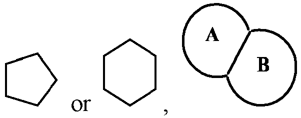 is not

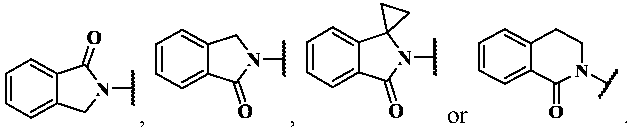

In certain embodiments of the present invention, a compound represented by general formula (I), or a stereoisomer or a pharmaceutically acceptable salt thereof is provided: 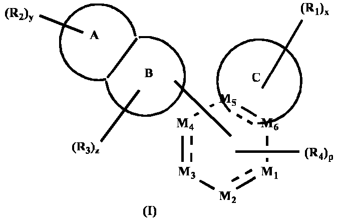 wherein: 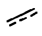
is a single bond or a double bond;
ring A is phenyl, 4- to 7-membered heterocyclyl, 5- to 6-membered heteroaryl, or absent; when ring A is absent, R₂ is connected to ring B;
ring B is 4- to 7-membered heterocyclyl, 5- to 6-membered heteroaryl, or phenyl;
ring C is cycloalkyl, heteroaryl, heterocyclyl, or absent; when ring C is absent, R₁ is connected to 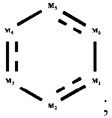
M₁, M₂, M₄, M₅, and M₆ are each independently selected from N, NH, or CH;
M₃ is a bond, N, or CH;
each R₁ is independently selected from cycloalkyl, heterocyclyl, cycloalkyloxy, heterocyclyloxy, cycloalkylamino, heterocyclylamino, cycloalkylthio, heterocyclylthio, -C(O)(CH₂)ₙR_{b}, -NRₐC(O)(CH₂)ₙR_{b},-S(O)₂(CH₂)ₙR_{b}, -S(O)(NH)(CH₂)ₙR_{b}, -NRₐS(O)₂(CH₂)ₙR_{b}, or-NRₐS(O)(NH)(CH₂)ₙR_{b}, wherein optionally the cycloalkyl, heterocyclyl, cycloalkyloxy, heterocyclyloxy, cycloalkylamino, heterocyclylamino, cycloalkylthio, or heterocyclylthio is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, hydroxyalkyl, -C(O)(CH₂)ₙR_{b}, -S(O)₂(CH₂)ₙR_{b}, -S(O)(NH)(CH₂)ₙR_{b}, -S(O)₂(CH₂)ₙR_{b}, or-NRₐS(O)(NH)(CH₂)ₙR_{b};
or any two R₁, together with adjacent carbon atoms, form 3- to 8-membered cycloalkyl or 4- to 7-membered heterocyclyl, wherein optionally the 3- to 8-membered cycloalkyl or the 4- to 7-membered heterocyclyl is further substituted with -C(O)R_{b}, -NRₐC(O)R_{b}, -S(O)₂R_{b}, -S(O)(NH)R_{b}, -NRₐS(O)₂R_{b}, or-NRₐS(O)(NH)R_{b};
Rₐ or R_{b} is each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, aryl, heteroaryl, or heterocyclyl;
R₂, R₃, or R₄ is each independently selected from hydrogen, deuterium, oxo, halogen, amino, hydroxyl, cyano, nitro, alkyl, alkenyl, alkynyl, deuteroalkyl, haloalkyl, alkoxy, haloalkoxy, hydroxyalkyl, cycloalkyl, heterocyclyl, aryl, or heteroaryl, wherein the alkyl, alkenyl, alkynyl, deuteroalkyl, haloalkyl, alkoxy, haloalkoxy, hydroxyalkyl, cycloalkyl, heterocyclyl, aryl, heteroaryl, - NRₐC(O)(CH₂)ₙR_{b}, or -C(O)NRₐ(CH₂)ₙR_{b} can be optionally further substituted;
or R₂ and R₃, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
or any two R₂, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
p, x, y, and z are each independently selected from 1, 2, 3, or 4; and
n is selected from 0, 1, 2, or 3.

In a preferred embodiment of the present invention, ring A is not selected from 5- to 10-membered heterocyclyl containing 1-3 moieties selected from C(O), N, O, S, SO₂, or SONH, or 5- to 10-membered heteroaryl.

In a preferred embodiment of the present invention, ring A is 5- to 7-membered heterocyclyl containing 1-3 moieties selected from C(O), N, O, S, SO₂, or SONH, or 5- to 6-membered heteroaryl, preferably 5- to 7-membered heterocyclyl containing 2-3 heteroatoms selected from N, O, or S, with ring A containing at least one oxygen atom.

In a preferred embodiment of the present invention, ring A is absent.

In a preferred embodiment of the present invention, ring B is selected from phenyl, 5- to 7-membered heterocyclyl containing 1-3 moieties selected from C(O), N, O, or S, or 5- to 6-membered heteroaryl.

In a preferred embodiment of the present invention, 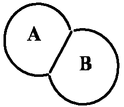 is selected from

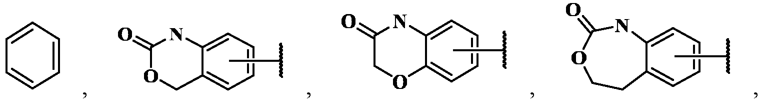

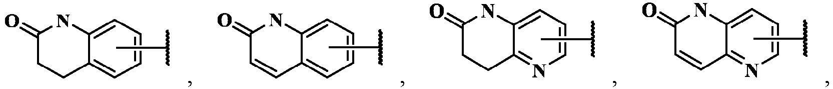

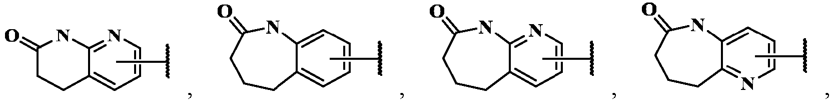

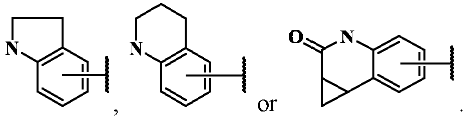

In a preferred embodiment of the present invention, ring C is selected from 3-to 10-membered cycloalkyl, or 4- to 10-membered heterocyclyl containing 1-3 moieties selected from C(O), N, O, S, SO₂, or SONH.

In a preferred embodiment of the present invention, ring C is selected from 5-to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, and 7- to 10-membered bicyclic heterocyclyl containing 1-3 moieties selected from C(O), N, O, S, SO₂, or SONH.

In a preferred embodiment of the present invention, ring C is selected from the following groups:

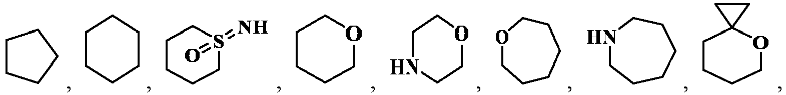

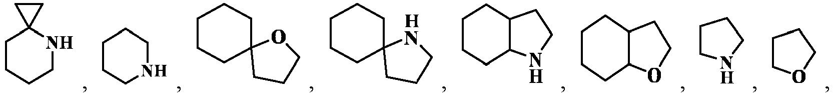

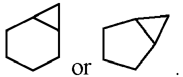

In a preferred embodiment of the present invention, ring C is absent.

In a preferred embodiment of the present invention, each R₁ is independently selected from 3- to 10-membered cycloalkyl, 4- to 10-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, 4- to 8-membered heterocyclylthio, -C(O)(CH₂)ₙR_{b},-NRₐC(O)(CH₂)ₙR_{b}, -O(CH₂)ₙR_{d}, -NH(CH₂)ₙR_{b}, -S(CH₂)ₙR_{b}, -S(O)₂(CH₂)ₙR_{b},-S(O)(NH)(CH₂)ₙR_{b}, -NRₐS(O)₂(CH₂)ₙR_{b}, or -NRₐS(O)(NH)(CH₂)ₙR_{b}, wherein optionally the 3- to 10-membered cycloalkyl, 4- to 10-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, or 4- to 8-membered heterocyclylthio is further substituted with one or more substituents selected from oxo, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋₆hydroxyalkyl, -C(O)(CH₂)ₙR_{b}, -S(O)₂(CH₂)ₙR_{b},-S(O)(NH)(CH₂)ₙR_{b}, -S(O)₂(CH₂)ₙR_{b}, or -NRₐS(O)(NH)(CH₂)ₙR_{b};

Rₐ or R_{b} is each independently selected from hydrogen, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋₆hydroxyalkyl, 3- to 8-membered cycloalkyl, 5- to 8-membered heteroaryl containing 1-3 moieties selected from C(O), N, O, or S, or 4- to 8-membered heterocyclyl containing 1-3 moieties selected from C(O), N, O, or S, wherein optionally the 3- to 8-membered cycloalkyl, 5- to 8-membered heteroaryl containing 1-3 moieties selected from C(O), N, O, or S, or 4-to 8-membered heterocyclyl containing 1-3 moieties selected from C(O), N, O, or S is further substituted with one or more substituents selected from oxo, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋₆hydroxyalkyl, or -SO₂-C₁₋₆alkyl.

In a preferred embodiment of the present invention, Rₐ or R_{b} is each independently selected from hydrogen, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋₆hydroxyalkyl, 3- to 8-membered cycloalkyl, 5- to 8-membered heteroaryl containing 1-3 moieties selected from C(O), N, O, or S, or 4- to 8-membered heterocyclyl containing 1-3 moieties selected from C(O), N, O, or S, wherein optionally the 3- to 8-membered cycloalkyl, 5- to 8-membered heteroaryl containing 1-3 moieties selected from C(O), N, O, or S, or 4- to 8-membered heterocyclyl containing 1-3 moieties selected from C(O), N, O, or S is further substituted with one or more substituents selected from oxo, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋₆hydroxyalkyl, 3- to 6-membered cycloalkyl, or -SO₂-C₁₋₆alkyl.

In a preferred embodiment of the present invention, each R₁ is independently selected from 3- to 10-membered cycloalkyl, 4- to 10-membered heterocyclyl containing 1-3 moieties selected from N, O, S, SO₂, or SONH, 3- to 8-membered cycloalkyloxy, 4- to 8-membered heterocyclyloxy containing 1-3 moieties selected from N, O, S, SO₂, or SONH, -C(O)(CH₂)ₙR_{b}, -NRₐC(O)(CH₂)ₙR_{b}, -O(CH₂)ₙR_{d},-S(O)₂(CH₂)ₙR_{b}, -S(O)(NH)(CH₂)ₙR_{b}, -NRₐS(O)₂(CH₂)ₙR_{b}, or - NRₐS(O)(NH)(CH₂)ₙR_{b}, wherein optionally the 3- to 10-membered cycloalkyl, 4- to 10-membered heterocyclyl containing 1-3 moieties selected from N, O, S, SO₂, or SONH, 3- to 8-membered cycloalkyloxy, or 4- to 8-membered heterocyclyloxy containing 1-3 moieties selected from N, O, S, SO₂, or SONH is further substituted with one or more substituents selected from oxo, -C(O)(CH₂)ₙR_{b}, -S(O)₂(CH₂)ₙR_{b},-S(O)(NH)(CH₂)ₙR_{b}, -S(O)₂(CH₂)ₙR_{b}, or -NRₐS(O)(NH)(CH₂)ₙR_{b}; and
Rₐ or R_{b} is each independently selected from hydrogen, deuterium, C₁₋₃alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 5- to 8-membered heteroaryl containing 1-3 heteroatoms selected from N, O, or S or the 4-to 8-membered heterocyclyl containing 1-3 moieties selected from C(O), N, O, or S is further substituted with one or more substituents selected from oxo, deuterium, halogen, amino, hydroxyl, cyano, nitro, C₁₋₃alkyl, C₂₋₄alkenyl, C₂₋₄alkynyl, C₁₋₃deuteroalkyl, C₁₋₃haloalkyl, C₁₋₃alkoxy, C₁₋₃haloalkoxy, C₁₋₃hydroxyalkyl, or -SO₂₋C₁₋₃alkyl.

In a preferred embodiment of the present invention, each R₁ is independently selected from 3- to 8-membered cycloalkyl, 4- to 8-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, 4- to 8-membered heterocyclylthio, -C(O)(CH₂)ₙR_{b}, - NRₐC(O)(CH₂)ₙR_{b}, -S(O)₂(CH₂)ₙR_{b}, -S(O)(NH)(CH₂)ₙR_{b}, -NRₐS(O)₂(CH₂)ₙR_{b}, or - NRₐS(O)(NH)(CH₂)ₙR_{b}, wherein optionally the 3- to 8-membered cycloalkyl, 4- to 8-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, or 4- to 8-membered heterocyclylthio is further substituted with one or more substituents selected from oxo, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋6hydroxyalkyl, -C(O)(CH₂)ₙR_{b}, -S(O)₂(CH₂)ₙR_{b}, -S(O)(NH)(CH2)nRb,-S(O)₂(CH₂)ₙR_{b}, or -NRₐS(O)(NH)(CH₂)ₙR_{b}; and
Ra or Rb is each independently selected from hydrogen, deuterium, halogen, amino, hydroxyl, cyano, nitro, C1-6alkyl, C2-6alkenyl, C2-6alkynyl, C1-6deuteroalkyl, C1-6haloalkyl, C1-6alkoxy, C1-6haloalkoxy, C1-6hydroxyalkyl, 3-to 8-membered cycloalkyl, 5- to 8-membered heteroaryl containing 1-3 moieties selected from C(O), N, O, or S, or 4- to 8-membered heterocyclyl containing 1-3 moieties selected from C(O), N, O, or S.

In a preferred embodiment of the present invention, each R₁ is independently selected from 3- to 8-membered cycloalkyl, 4- to 8-membered heterocyclyl containing 1-3 moieties selected from N, O, S, SO₂, or SONH, 3- to 8-membered cycloalkyloxy, 3- to 8-membered cycloalkylamino, 4- to 8-membered heterocyclyloxy containing 1-3 moieties selected from N, O, S, SO₂, or SONH, 4-to 8-membered heterocyclylamino containing 1-3 moieties selected from N, O, S, SO₂, or SONH, -C(O)(CH₂)ₙR_{b}, -NRₐC(O)(CH₂)ₙR_{b}, -O(CH₂)ₙR_{b}, -S(O)₂(CH₂)ₙR_{b}, -S(O)(NH)(CH₂)ₙR_{b}, -NRₐS(O)₂(CH₂)ₙR_{b}, or -NRₐS(O)(NH)(CH₂)ₙR_{b}, wherein optionally the 3- to 8-membered cycloalkyl, 4- to 8-membered heterocyclyl containing 1-3 moieties selected from N, O, S, SO₂, or SONH, 3- to 8-membered cycloalkyloxy, 3- to 8-membered cycloalkylamino, 4- to 8-membered heterocyclyloxy containing 1-3 moieties selected from N, O, S, SO₂, or SONH, or 4- to 8-membered heterocyclylamino containing 1-3 moieties selected from N, O, S, SO₂, or SONH is further substituted with one or more substituents selected from oxo, -C(O)(CH₂)ₙR_{b}, -S(O)₂(CH₂)ₙR_{b}, -S(O)(NH)(CH₂)ₙR_{b}, -S(O)₂(CH₂)ₙR_{b}, or-NRₐS(O)(NH)(CH₂)ₙR_{b}; and
Rₐ or R_{b} is each independently selected from hydrogen, deuterium, C₁₋₃alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 5- to 8-membered heteroaryl containing 1-3 heteroatoms selected from N, O, or S, or 4- to 8-membered heterocyclyl containing 1-3 moieties selected from C(O), N, O, or S is further substituted with one or more substituents selected from oxo, deuterium, halogen, amino, hydroxyl, cyano, nitro, C₁₋₃alkyl, C₂₋₄alkenyl, C₂₋₄alkynyl, C₁₋₃deuteroalkyl, C₁₋₃haloalkyl, C₁₋₃alkoxy, C₁₋₃haloalkoxy, C₁₋₃hydroxyalkyl, 3- to 6-membered cycloalkyl, or -SO₂-C₁₋₃alkyl.

Alternatively, Rₐ or R_{b} is each independently selected from halogen, hydroxyl, or cyano.

In a preferred embodiment of the present invention, each R₁ is independently selected from 3- to 8-membered cycloalkyl, 4- to 8-membered heterocyclyl containing 1-3 moieties selected from N, O, S, SO₂, or SONH, 3- to 8-membered cycloalkyloxy, 4- to 8-membered heterocyclyloxy containing 1-3 moieties selected from N, O, S, SO₂, or SONH, -C(O)(CH₂)ₙR_{b}, -NRₐC(O)(CH₂)ₙR_{b}, -S(O)₂(CH₂)ₙR_{b}, -S(O)(NH)(CH₂)ₙR_{b}, -NRₐS(O)₂(CH₂)ₙR_{b}, or -NRₐS(O)(NH)(CH₂)ₙR_{b}, wherein optionally the 3- to 8-membered cycloalkyl, 4- to 8-membered heterocyclyl containing 1-3 moieties selected from N, O, S, SO₂, or SONH, 3- to 8-membered cycloalkyloxy, or 4- to 8-membered heterocyclyloxy containing 1-3 moieties selected from N, O, S, SO₂, or SONH is further substituted with one or more substituents selected from oxo, -C(O)(CH₂)ₙR_{b}, -S(O)₂(CH₂)ₙR_{b},-S(O)(NH)(CH₂)ₙR_{b}, -S(O)₂(CH₂)ₙR_{b}, or -NRₐS(O)(NH)(CH₂)ₙR_{b}; and
Ra or Rb is each independently selected from hydrogen, deuterium, C1-3alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S.

In a preferred embodiment of the present invention, R₂, R₃, or R₄ is each independently selected from hydrogen, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋₆deuteroalkoxy, C₁₋₆hydroxyalkyl, 3- to 8-membered cycloalkyl, 3- to 8-membered cycloalkyloxy, 3- to 8-membered cycloalkylamino, C₆₋₁₀aryl, 5-to 6-membered heteroaryl containing 1-3 heteroatoms selected from N, O, or S or 4-to 8-membered heterocyclyl containing 1-3 moieties selected from C(O), N, O, or S, -NRₐR_{b}, -NRₐC(O)R_{b}, or - C(O)NRₐR_{b}.

In a preferred embodiment of the present invention, R₂, R₃, or R₄ is each independently selected from hydrogen, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋₆hydroxyalkyl, 3- to 8-membered cycloalkyl, or 4- to 8-membered heterocyclyl containing 1-3 moieties selected from C(O), N, O, or S,-NRₐC(O)R_{b}, or -C(O)NRₐR_{b}; and
Rₐ or R_{b} is each independently selected from hydrogen, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋₆hydroxyalkyl, 3- to 8-membered cycloalkyl, 5- to 8-membered heteroaryl containing 1-3 moieties selected from C(O), N, O, or S, or 4- to 8-membered heterocyclyl containing 1-3 moieties selected from C(O), N, O, or S.

Alternatively, Rₐ or R_{b} is each independently selected from halogen, hydroxyl, or cyano.

In a preferred embodiment of the present invention, R₂, R₃, or R₄ is each independently selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, or C₃₋₆cycloalkyloxy.

In a preferred embodiment of the present invention, R₂, R₃, or R₄ is each independently selected from hydrogen, deuterium, halogen, C₁₋₃alkyl, C₁₋₃deuteroalkyl, C₁₋₃haloalkyl, C₁₋₃alkoxy, C₁₋₃haloalkoxy, or C₁₋₃hydroxyalkyl.

In a preferred embodiment of the present invention, any two R₂, together with adjacent atoms, form 3- to 8-membered cycloalkyl, 5- to 6-membered heteroaryl containing 1-3 heteroatoms selected from N, O, or S, or 4- to 7-membered heterocyclyl containing 1-3 heteroatoms selected from N, O, or S; and optionally the 3- to 8-membered cycloalkyl, 5- to 6-membered heteroaryl containing 1-3 heteroatoms selected from N, O, or S, or 4- to 7-membered heterocyclyl containing 1-3 heteroatoms selected from N, O, or S is further substituted with one or more substituents selected from oxo, deuterium, halogen, amino, hydroxyl, cyano, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, or C₁₋₆hydroxyalkyl.

In a preferred embodiment of the present invention, any two R₁, together with adjacent atoms, form 3- to 8-membered cycloalkyl, or 4- to 7-membered heterocyclyl containing 1-3 heteroatoms selected from N, O, or S; and optionally the 3- to 8-membered cycloalkyl, or 4- to 7-membered heterocyclyl containing 1-3 heteroatoms selected from N, O, or S is further substituted with one or more substituents selected from oxo, -C(O)R_{b}, -NRₐC(O)R_{b}, -S(O)₂R_{b}, -S(O)(NH)R_{b},-NRₐS(O)₂R_{b}, or -NRₐS(O)(NH)R_{b}.

In a preferred embodiment of the present invention, R₂ and R₃, together with adjacent atoms, form 3- to 8-membered cycloalkyl, 5- to 6-membered heteroaryl containing 1-3 heteroatoms selected from N, O, or S, or 4- to 7-membered heterocyclyl containing 1-3 heteroatoms selected from N, O, or S; and optionally the 3- to 8-membered cycloalkyl, 5- to 6-membered heteroaryl containing 1-3 heteroatoms selected from N, O, or S, or 4- to 7-membered heterocyclyl containing 1-3 heteroatoms selected from N, O, or S is further substituted with one or more substituents selected from deuterium, halogen, amino, hydroxyl, cyano, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, or C₁₋₆hydroxyalkyl.

In a preferred embodiment of the present invention, R₂ and R₄, together with adjacent atoms, form 6- to 12-membered cycloalkyl, 6- to 12-membered heteroaryl containing 1-4 moieties selected from N, O, or S, or 6- to 12-membered heterocyclyl containing 1-4 moieties selected from N, O, or S; and optionally the 6- to 12-membered cycloalkyl, 6- to 12-membered heteroaryl containing 1-4 moieties selected from N, O, or S, or 6- to 12-membered heterocyclyl containing 1-4 moieties selected from N, O, or S is further substituted with one or more substituents selected from deuterium, halogen, amino, hydroxyl, cyano, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, or C₁₋₆hydroxyalkyl.

In a more preferred embodiment of the present invention, the general formula (I) is further a compound represented by general formula (II-a) or (II-c), or a stereoisomer or a pharmaceutically acceptable salt thereof: 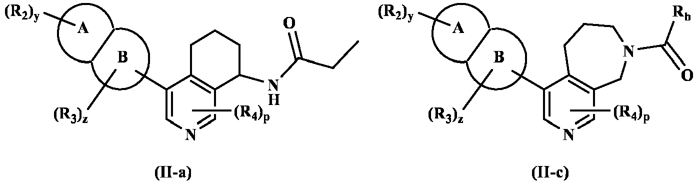 wherein,
ring A is 5- to 7-membered heterocyclyl containing 1-3 moieties selected from C(O), N, O, S, SO₂, or SONH, or absent;
ring B is selected from phenyl, or 5- to 6-membered heteroaryl containing 1-3 heteroatoms selected from N, O, or S;
R₂, R₃, or R₄ is each independently selected from hydrogen, deuterium, halogen, C₁₋₃alkyl, C₁₋₃deuteroalkyl, C₁₋₃haloalkyl, C₁₋₃alkoxy, C₁₋₃haloalkoxy, or C₁₋₃hydroxyalkyl, -NRₐC(O)R_{b}, or -C(O)NRₐR_{b};
Rₐ or R_{b} is each independently selected from hydrogen, deuterium, C₁₋₃alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S;
or any two R₂, together with adjacent atoms, form 3- to 8-membered cycloalkyl, 5- to 6-membered heteroaryl containing 1-3 heteroatoms selected from N, O, or S, or 4- to 7-membered heterocyclyl containing 1-3 heteroatoms selected from N, O, or S, wherein optionally the 3- to 8-membered cycloalkyl, 5- to 6-membered heteroaryl containing 1-3 heteroatoms selected from N, O, or S, or 4- to 7-membered heterocyclyl containing 1-3 heteroatoms selected from N, O, or S is further substituted with one or more substituents selected from deuterium, halogen, amino, hydroxyl, cyano, C₁₋₃alkyl, C₁₋₃deuteroalkyl, C₁₋₃haloalkyl, C₁₋₃alkoxy, C₁₋₃haloalkoxy, or C₁₋₃hydroxyalkyl;
or R₂ and R₃, together with adjacent atoms, form 3- to 8-membered cycloalkyl, or 4- to 7-membered heterocyclyl containing 1-3 heteroatoms selected from N, O, or S; optionally the 3- to 8-membered cycloalkyl, or 4- to 7-membered heterocyclyl containing 1-3 heteroatoms selected from N, O, or S is further substituted with one or more substituents selected from deuterium, halogen, amino, hydroxyl, cyano, C₁₋₃alkyl, C₁₋₃deuteroalkyl, C₁₋₃haloalkyl, C₁₋₃alkoxy, C₁₋₃haloalkoxy, or C₁₋₃hydroxyalkyl;
or R₂ and R₄, together with adjacent atoms, form 6- to 12-membered cycloalkyl, 6- to 12-membered heteroaryl containing 1-4 moieties selected from N, O, or S, or 6- to 12-membered heterocyclyl containing 1-4 moieties selected from N, O, or S; and optionally the 6- to 12-membered cycloalkyl, 6- to 12-membered heteroaryl containing 1-4 moieties selected from N, O, or S, or 6- to 12-membered heterocyclyl containing 1-4 moieties selected from N, O, or S is further substituted with one or more substituents selected from deuterium, halogen, amino, hydroxyl, cyano, C₁₋₃alkyl, C₁₋₃deuteroalkyl, C₁₋₃haloalkyl, C₁₋₃alkoxy, C₁₋₃haloalkoxy, or C₁₋₃hydroxyalkyl.

In a more preferred embodiment of the present invention, the general formula (II-a) is further a compound represented by general formula (II-a-1) or (II-a-2), or a stereoisomer or a pharmaceutically acceptable salt thereof:

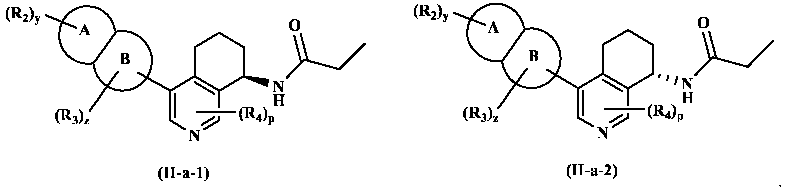

In a more preferred embodiment of the present invention, the general formula (II-c) is further a compound represented by general formula (II-c-1) or (II-c-2), or a stereoisomer or a pharmaceutically acceptable salt thereof:

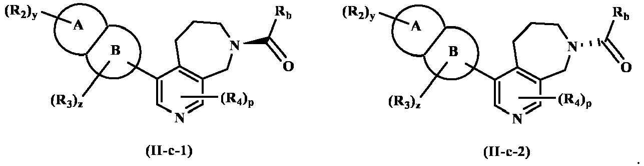

In a more preferred embodiment of the present invention, the general formula (I) is further a compound represented by general formula (II-b), or a stereoisomer or a pharmaceutically acceptable salt thereof: 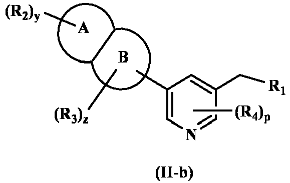 wherein,
ring A is absent or is 5- to 7-membered heterocyclyl containing 1-3 moieties selected from C(O), N, O, S, SO₂, or SONH, or absent;
ring B is selected from phenyl, or 5- to 6-membered heteroaryl containing 1-3 heteroatoms selected from N, O, or S;
R₁ is selected from 3- to 8-membered cycloalkyl, 4- to 8-membered heterocyclyl containing 1-3 moieties selected from N, O, S, SO₂, or SONH, 3- to 8-membered cycloalkyloxy, 4- to 8-membered heterocyclyloxy containing 1-3 moieties selected from N, O, S, SO₂, or SONH, -C(O)(CH₂)ₙR_{b}, -NR₂C(O)(CH₂)ₙR_{b}, -O(CH₂)ₙR_{b}, -S(O)₂(CH₂)ₙR_{b}, -S(O)(NH)(CH₂)ₙR_{b}, -NRₐS(O)₂(CH₂)ₙR_{b}, or-NRₐS(O)(NH)(CH₂)ₙR_{b}, wherein optionally the 3- to 8-membered cycloalkyl, 4- to 8-membered heterocyclyl containing 1-3 moieties selected from N, O, S, SO₂, or SONH, 3- to 8-membered cycloalkyloxy, or 4- to 8-membered heterocyclyloxy containing 1-3 moieties selected from N, O, S, SO₂, or SONH is further substituted with one or more substituents selected from oxo, -C(O)(CH₂)ₙR_{b}, -S(O)₂(CH₂)ₐR_{b},-S(O)(NH)(CH₂)ₙR_{b}, -S(O)₂(CH₂)ₙR_{b}, or -NRₐS(O)(NH)(CH₂)ₙR_{b};
R₂, R₃, or R₄ is each independently selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, -NRₐC(O)R_{b}, or -C(O)NRₐR_{b};
Rₐ or R_{b} is each independently selected from hydrogen, deuterium, C₁₋₃alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 5- to 8-membered heteroaryl containing 1-3 heteroatoms selected from N, O, or S or the 4-to 8-membered heterocyclyl containing 1-3 moieties selected from C(O), N, O, or S is further substituted with one or more substituents selected from oxo, deuterium, halogen, amino, hydroxyl, cyano, nitro, C₁₋₃alkyl, C₂₋₄alkenyl, C₂₋₄alkynyl, C₁₋₃deuteroalkyl, C₁₋₃haloalkyl, C₁₋₃alkoxy, C₁₋₃haloalkoxy, C₁₋₃hydroxyalkyl, or -SO₂-C₁₋₃alkyl;
or any two R₂, together with adjacent atoms, form 3- to 8-membered cycloalkyl, 5- to 6-membered heteroaryl containing 1-3 heteroatoms selected from N, O, or S, or 4- to 7-membered heterocyclyl containing 1-3 heteroatoms selected from N, O, or S, wherein optionally the 3- to 8-membered cycloalkyl, 5- to 6-membered heteroaryl containing 1-3 heteroatoms selected from N, O, or S, or 4- to 7-membered heterocyclyl containing 1-3 heteroatoms selected from N, O, or S is further substituted with one or more substituents selected from deuterium, halogen, amino, hydroxyl, cyano, C₁₋₃alkyl, C₁₋₃deuteroalkyl, C₁₋₃haloalkyl, C₁₋₃alkoxy, C₁₋₃haloalkoxy, or C₁₋₃hydroxyalkyl;
or R₂ and R₃, together with adjacent atoms, form 3- to 8-membered cycloalkyl, or 4- to 7-membered heterocyclyl containing 1-3 heteroatoms selected from N, O, or S; optionally the 3- to 8-membered cycloalkyl, or 4- to 7-membered heterocyclyl containing 1-3 heteroatoms selected from N, O, or S is further substituted with one or more substituents selected from deuterium, halogen, amino, hydroxyl, cyano, C₁₋₃alkyl, C₁₋₃deuteroalkyl, C₁₋₃haloalkyl, C₁₋₃alkoxy, C₁₋₃haloalkoxy, or C₁₋₃hydroxyalkyl;
or R₂ and R₄, together with adjacent atoms, form 6- to 12-membered cycloalkyl, 6- to 12-membered heteroaryl containing 1-4 moieties selected from N, O, or S, or 6- to 12-membered heterocyclyl containing 1-4 moieties selected from N, O, or S; and optionally the 6- to 12-membered cycloalkyl, 6- to 12-membered heteroaryl containing 1-4 moieties selected from N, O, or S, or 6- to 12-membered heterocyclyl containing 1-4 moieties selected from N, O, or S is further substituted with one or more substituents selected from deuterium, halogen, amino, hydroxyl, cyano, C₁₋₃alkyl, C₁₋₃deuteroalkyl, C₁₋₃haloalkyl, C₁₋₃alkoxy, C₁₋₃haloalkoxy, or C₁₋₃hydroxyalkyl.

In a more preferred embodiment of the present invention, the general formula (I) is further a compound represented by general formula (III-a)-(III-d), or a stereoisomer or a pharmaceutically acceptable salt thereof: 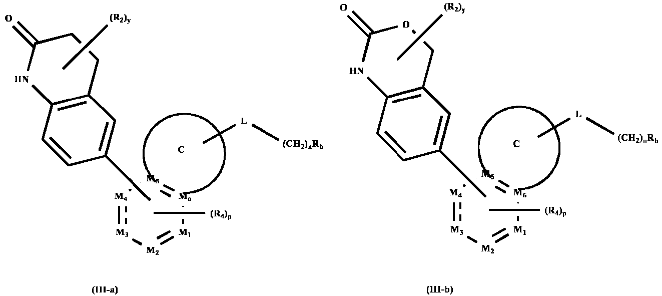 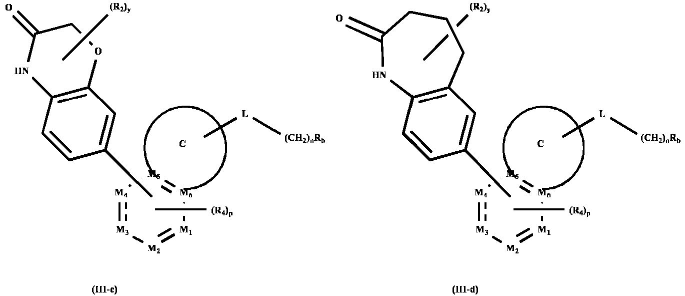
ring C is selected from 5- to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, and 7- to 10-membered bicyclic heterocyclyl containing 1-3 moieties selected from C(O), N, O, S, SO₂, or SONH;
or ring C is absent;
L is selected from a bond, -O-, -R_{c}C(O)-, -R_{c}S(O)NH-, or -R_{c}S(O)₂, preferably -NHC(O)-;
R_{c} is selected from a bond, NH, 3- to 8-membered cycloalkyl, 4- to 8-membered heterocyclyl containing 1-3 moieties selected from N, O, S, SO₂, or SONH, 3- to 8-membered cycloalkyloxy, or 4- to 8-membered heterocyclyloxy containing 1-3 moieties selected from N, O, S, SO₂, or SONH;
R₂, R₃, or R₄ is each independently selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, or C₃₋₆cycloalkyl;
R_{b} is selected from hydrogen, deuterium, halogen, hydroxyl, cyano, C₁₋₃alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂, or SONH; and
y is 1, 2, or 3.

In a more preferred embodiment of the present invention, the general formula (I) is further a compound represented by general formula (III') or general formula (IV'), or a stereoisomer or a pharmaceutically acceptable salt thereof: 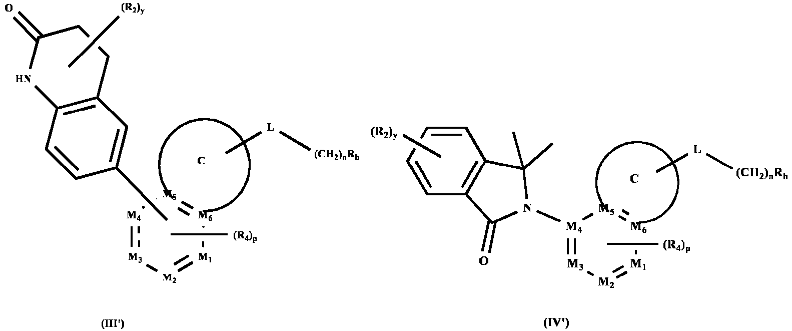
ring C is selected from 5- to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, and 7- to 10-membered bicyclic heterocyclyl containing 1-3 moieties selected from C(O), N, O, S, SO₂, or SONH;
or ring C is absent;
L is selected from a bond, -O-, -R_{c}C(O)-, -R_{c}S(O)NH-, or -R_{c}S(O)₂;
R_{c} is selected from a bond, NH, 3- to 8-membered cycloalkyl, 4- to 8-membered heterocyclyl containing 1-3 moieties selected from N, O, S, SO₂, or SONH, 3- to 8-membered cycloalkyloxy, or 4- to 8-membered heterocyclyloxy containing 1-3 moieties selected from N, O, S, SO₂, or SONH;
R₂ or R₄ is each independently selected from hydrogen, deuterium, halogen, C₁₋₃alkyl, C₁₋₃deuteroalkyl, C₁₋₃haloalkyl, C₁₋₃alkoxy, C₁₋₃haloalkoxy, or C₁₋₃hydroxyalkyl;
R_{b} is selected from hydrogen, deuterium, C₁₋₃alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂, or SONH; and
y is 1, 2, or 3.

In a further preferred embodiment of the present invention, 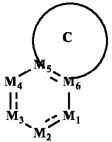 is selected from the following groups:

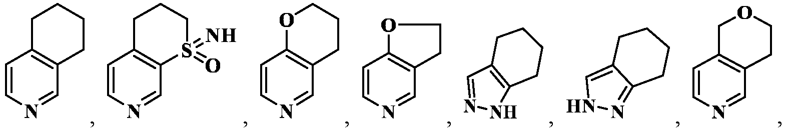

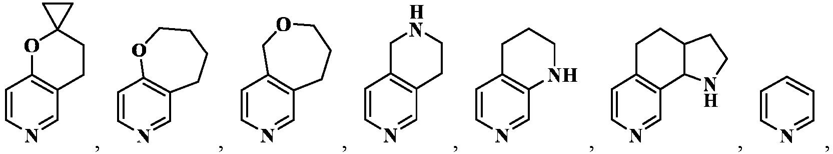

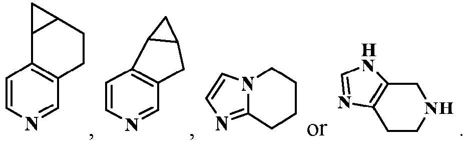

In a more preferred embodiment of the present invention, in a more preferred embodiment of the present invention, the general formula (I) is further a compound represented by general formula (IV), or a stereoisomer or a pharmaceutically acceptable salt thereof: 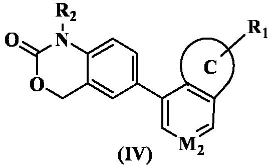 wherein:
M₂ is selected from N or CH;
ring C is selected from 5- to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, 7- to 10-membered bicyclic heterocyclyl containing 1-3 moieties selected from C(O), N, O, S, SO₂, or SONH, or absent;
R₁ is selected from -C(O)R_{b}, -NRₐC(O)R_{b}, -OR_{b}, -S(O)₂R_{b}, -S(O)(NH)R_{b},-NRₐS(O)₂R_{b}, -NRₐS(O)(NH)R_{b}, or 4- to 8-membered heterocyclyl, wherein the 4-to 8-membered nitrogen-containing heterocyclyl is optionally further substituted with -C(O)R_{b} or -S(O)₂R_{b};
Rₐ or R_{b} is each independently selected from hydrogen, deuterium, halogen, hydroxyl, cyano, C₁₋₃alkyl, C₁₋₃deuteroalkyl, C₁₋₃haloalkyl, C₁₋₃alkoxy, C₁₋₃haloalkoxy, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S; and
R₂ is selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, or C₃₋₆cycloalkyloxy.

In a more preferred embodiment of the present invention, the general formula (I) is further a compound represented by general formula (VI), or a stereoisomer or a pharmaceutically acceptable salt thereof: 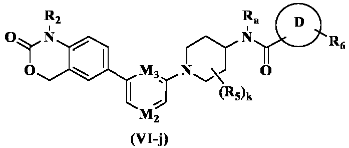 wherein:
L is selected from NRₐ, O, or S;
L₁ is selected from C(O) or S(O)₂;
Rₐ is selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, or C₃₋₆cycloalkyloxy;
M₂ is selected from N or CH;
M₃ is selected from N or CH;
R₂ is selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, or C₃₋₆cycloalkyloxy;
or any two R₂, together with adjacent atoms, form 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S;
each R_{b} is independently selected from hydrogen, deuterium, C₁₋₃alkyl, C₁₋₃deuteroalkyl, C₁₋₃haloalkyl, C₁₋₃alkoxy, C₁₋₃haloalkoxy, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S is further substituted with halogen, hydroxyl, amino, CN, C₁₋₃alkyl, C₁₋₃deuteroalkyl, C₁₋₃haloalkyl, C₁₋₃alkoxy, C₁₋₃haloalkoxy, C₁₋₃hydroxyalkyl, or 3- to 6-membered cycloalkyl;
each R₅ is independently selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, or C₃₋₆cycloalkyloxy;
R₆ is selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, or C₃₋₆cycloalkyl;
or any two R₅, together with adjacent carbon atoms, form 3- to 8-membered cycloalkyl or 3- to 8-membered heterocyclyl;
ring D is selected from 3- to 8-membered cycloalkyl or 4- to 8-membered heterocyclyl, preferably 4- to 6-membered nitrogen-containing heterocyclyl;
q, r, s, and t are each independently selected from 1 or 2; and
k is independently selected from 1, 2, or 3.

In a more preferred embodiment of the present invention, R₂ is selected from methyl or methoxy, and y is 1.

In a more preferred embodiment of the present invention, M₂ is N, and M₃ is CH.

In a more preferred embodiment of the present invention, R_{b} is selected from C₁₋₃alkyl, 3- to 6-membered cycloalkyl, or 5- to 6-membered heteroaryl containing 1-3 heteroatoms selected from N, O, or S, wherein optionally the 3- to 6-membered cycloalkyl or the 5- to 6-membered heteroaryl containing 1-3 heteroatoms selected from N, O, or S is further substituted with halogen, hydroxyl, CN, or C₁₋₃alkyl.

In a more preferred embodiment of the present invention, q, r, s, and t are all 1.

In another aspect, the present invention further relates to a pharmaceutical composition comprising a therapeutically effective dose of any compound represented by the general formula, or a stereoisomer or a pharmaceutically acceptable salt thereof, and one or more pharmaceutically acceptable carriers, diluents, or excipients.

In certain embodiments of the present invention, the weight percentage of the compound, or the stereoisomer or the pharmaceutically acceptable salt thereof in the pharmaceutical composition is 0.1-95%, preferably 5-70%, e.g., 70%, 65%, 60%, 55%, 50%, 45%, 40%, 35%, 30%, 25%, 20%, 15%, 10%, or 5% on a free base basis.

In certain embodiments of the present invention, the pharmaceutical composition is selected from tablets, capsules, a liquid preparation, or an injection, preferably further comprises a filler, optionally further comprises a disintegrant, or further comprises one or more of a glidant or a lubricant.

In certain embodiments of the present invention, the pharmaceutical composition is an immediate-release preparation or a sustained-release preparation.

In certain embodiments of the present invention, the unit dose of the compound, or the stereoisomer or the pharmaceutically acceptable salt thereof in the pharmaceutical composition is 1-1000 mg, preferably 1-500 mg, or preferably 1 mg, 2 mg, 3 mg, 5 mg, 10 mg, 20 mg, 40 mg, 50 mg, 60 mg, 80 mg, 100 mg, 200 mg, 300 mg, 400 mg, or 500 mg on a free base basis.

In certain embodiments of the present invention, the compound, or the stereoisomer or the pharmaceutically acceptable salt thereof can be administered by any convenient method, for example, by oral, parenteral, buccal, sublingual, nasal, rectal, intrathecal or transdermal administration, and the pharmaceutical composition can be adjusted accordingly.

In certain embodiments of the present invention, the compound, or the stereoisomer or the pharmaceutically acceptable salt thereof can be formulated into a liquid or solid preparation, such as a syrup, a suspension, an emulsion, tablets, capsules, powder, granules, or a lozenge.

In another aspect, the present invention further relates to the use of any compound represented by the general formula, or the stereoisomer or the pharmaceutically acceptable salt thereof, or the pharmaceutical composition in the preparation of a medicament for treating a disease related to CYP11B2.

The present invention further relates to the use of the compound represented by the general formula, or the stereoisomer or the pharmaceutically acceptable salt thereof, or the pharmaceutical composition thereof in the preparation of a medicament for treating or preventing chronic kidney disease, kidney or cardiac fibrosis, diabetic nephropathy, congestive heart failure, hypertension, primary aldosteronism, and Cushing's syndrome.

In a preferred embodiment of the present invention, the hypertension is refractory hypertension.

In some embodiments, the EC₅₀ value of the compound of the present invention for CYP11B2 is between 0.0001 µM and 50 µM; preferably, the EC₅₀ value of the compound for CYP11B2 is between 0.0001 µM and 10 µM; more preferably, the EC₅₀ value of the compound for CYP11B2 is between 0.0001 µM and 1 µM; and further preferably, the EC₅₀ value of the compound for CYP11B2 is between 0.0001 µM and 0.1 µM.

In addition, the compound of the present invention has a relatively good selectivity for CYP11B1, and the selectivity is greater than 50, preferably greater than 100, more preferably greater than 200, further preferably greater than 500.

In another aspect, the present invention provides a method for preparing a compound represented by general formula (I), or a stereoisomer or a pharmaceutically acceptable salt thereof, comprising the following steps: 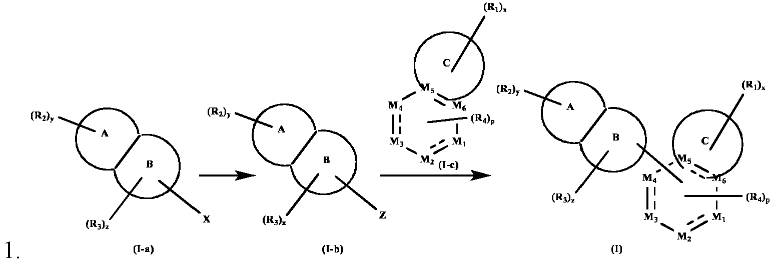
reacting compound (I-a) with a boron compound to prepare compound (I-b), or a stereoisomer or a pharmaceutically acceptable salt thereof, and performing a coupling reaction with compound (I-c), or a stereoisomer or a pharmaceutically acceptable salt thereof to prepare the compound represented by general formula (I), or the stereoisomer or the pharmaceutically acceptable salt thereof, wherein
a catalyst for the coupling reaction is palladium reagent, preferably palladium acetate, tetrakis(triphenylphosphine)palladium, bis(dibenzylideneacetone)palladiu m, tris(dibenzylideneacetone)dipalladium, or [1,1'-bis(diphenylphosphino)ferrocen e]palladium dichloride;
X is halogen, preferably bromine;
Z is a boron-containing compound, preferably dioxaborolanyl; and
ring A, ring B, R₁, R₂, R₃, R₄, M₁, M₂, M₃, M₄, M₅, M₆, x, y, z, and p are as defined for general formula (I).
Preferably, the present invention provides a method for preparing a compound represented by general formula (IV), or a stereoisomer or a pharmaceutically acceptable salt thereof, comprising the following steps: 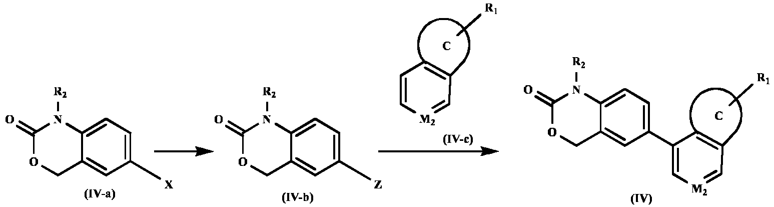
reacting compound (IV-a) with a borane to prepare compound (IV-b), or a stereoisomer or a pharmaceutically acceptable salt thereof, and performing a coupling reaction with compound (IV-c), or a stereoisomer or a pharmaceutically acceptable salt thereof to prepare the compound represented by general formula (IV), or the stereoisomer or the pharmaceutically acceptable salt thereof, wherein
a catalyst for the coupling reaction is palladium reagent, preferably palladium acetate, tetrakis(triphenylphosphine)palladium, bis(dibenzylideneacetone)palladiu m, tris(dibenzylideneacetone)dipalladium, or [1,1'-bis(diphenylphosphino)ferrocen e]palladium dichloride;
X is halogen, preferably bromine;
Z is boranyl, preferably dioxaborolanyl; and
ring C, R₁, R₂, and M₂ are as defined for general formula (IV).

### Detailed Description of the Invention

Unless stated to the contrary, the terms used in the description and claims have the following meanings.

The term "alkyl" refers to a saturated aliphatic hydrocarbon group, which is a linear or branched group containing 1 to 20 carbon atoms, preferably alkyl containing 1 to 8 carbon atoms, more preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferably, the alkyl is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl can be substituted or unsubstituted, and when substituted, the substituent can be substituted at any available connection point, and the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group. In the present invention, alkyl is preferably methyl, ethyl, isopropyl, tert-butyl, haloalkyl, deuteroalkyl, alkoxy-substituted alkyl, and hydroxyl-substituted alkyl.

The term "cycloalkyl" refers to a saturated or partially unsaturated monocyclic or polycyclic cyclic hydrocarbon substituent. The cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and polycyclic cycloalkyl includes spiro, fused and bridged cycloalkyl, preferably cyclopropyl, cyclobutyl, cyclohexyl, cyclopentyl, and cycloheptyl.

The term "spirocycloalkyl" refers to a polycyclic group with 5 to 20 membered monocyclic rings sharing one carbon atom (called a spiro atom). It may contain one or more double bonds, but no ring has a completely conjugated π electron system. Preferably, the group is 6- to 14-membered, and more preferably 7- to 10-membered. According to the number of shared spiro atoms between the rings, the spirocycloalkyl is divided into monospirocycloalkyl, bispirocycloalkyl or polyspirocycloalkyl, preferably monospirocycloalkyl and bispirocycloalkyl. More preferably, the group is a 3-membered/6-membered, 3-membered/5-membered, 4-membered/4-membered, 4-membered/5-membered, 4-membered/6-membered, 5-membered/5-membered or 5-membered/6-membered monospirocycloalkyl. Non-limiting examples of spirocycloalkyl include: 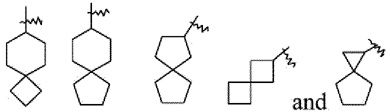 etc.;
and also include spirocycloalkyl in which monospirocycloalkyl and heterocycloalkyl share a spiro atom. Non-limiting examples include: 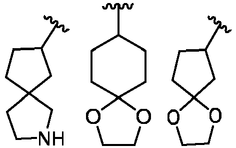 etc.

The term "fused cycloalkyl" refers to a 5- to 20-membered all-carbon polycyclic group in which each ring in the system shares an adjacent pair of carbon atoms with other rings in the system, one or more ring of which may contain one or more double bonds, but no ring has a fully conjugated π electron system. Preferably, the group is 6- to 14-membered, and more preferably 7- to 10-membered. According to the number of constituent rings, it can be divided into bicyclic, tricyclic, tetracyclic or polycyclic fused cycloalkyl, preferably bicyclic or tricyclic, and more preferably 5-membered/5-membered or 5-membered/6-membered bicyclic cycloalkyl. Non-limiting examples of fused cycloalkyl include: 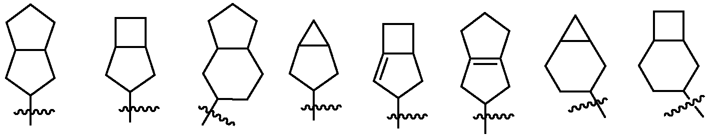 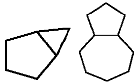 etc.

The term "bridged cycloalkyl" refers to a 5- to 20-membered all-carbon polycyclic group with any two rings sharing two carbon atoms that are not directly connected. It may contain one or more double bonds, but no ring has a fully conjugated π electron system. Preferably, the group is 6- to 14-membered, and more preferably 7- to 10-membered. According to the number of constituent rings, it can be divided into bicyclic, tricyclic, tetracyclic or polycyclic bridged cycloalkyl, preferably bicyclic, tricyclic or tetracyclic, and more preferably bicyclic or tricyclic. Non-limiting examples of bridged cycloalkyl include:

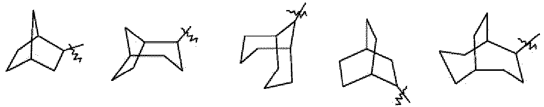

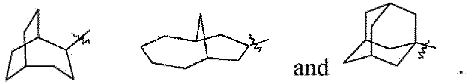

The cycloalkyl ring can be fused to an aryl, heteroaryl or heterocycloalkyl ring, wherein the ring connected to the parent structure is cycloalkyl, and non-limiting examples include indanyl, tetrahydronaphthyl, benzocycloheptanyl, etc. Cycloalkyl can be optionally substituted or unsubstituted. When the cycloalkyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group.

The term "heterocyclyl" refers to a saturated or partially unsaturated monocyclic or polycyclic cyclic hydrocarbon substituent, which comprises 3 to 20 ring atoms, wherein one or more ring atoms are heteroatoms selected from nitrogen, oxygen or S(O)ₘ (wherein m is an integer of 0 to 2), but excluding ring moieties of -O-O-, -O-S- or -S-S-, and the remaining ring atoms are carbon. Preferably, the heterocyclyl comprises 3 to 12 ring atoms, among which 1-4 are heteroatoms; more preferably, the heterocyclyl comprises 3 to 8 ring atoms; most preferably, the heterocyclyl comprises 3 to 8 ring atoms; further preferably, the heterocyclyl is a 3-to -8-membered heterocyclyl group comprising 1-3 nitrogen atoms, which is optionally substituted with 1-2 oxygen atoms, sulfur atoms, or oxo, including nitrogen-containing monocyclic heterocyclyl, nitrogen-containing spiroheterocyclyl, or nitrogen-containing fused heterocyclyl.

Non-limiting examples of monocyclic heterocyclyl include azetidine, pyrrolidinyl, imidazolidinyl, tetrahydrofuryl, tetrahydrothienyl, dihydroimidazolyl, dihydrofuryl, dihydropyrazolyl, dihydropyrrolyl, piperidinyl, piperazinyl, morpholinyl, thiomorpholinyl, homopiperazinyl, azepinyl, 1,4-diazacycloheptyl, pyranyl, etc., preferably pyrrolidinyl, morpholinyl, piperidinyl, azepinyl, 1,4-diazacycloheptyl, and piperazinyl. Polycyclic heterocyclyl includes spiro, fused and bridged heterocyclyl, wherein the spiro, fused and bridged heterocyclyl are optionally connected to other groups via a single bond, or further fused to other cycloalkyl, heterocyclyl, aryl and heteroaryl via any two or more atoms on the ring.

The term "spiroheterocyclyl" refers to 5- to 20-membered polycyclic heterocyclyl with monocyclic rings sharing one atom (called a spiro atom), wherein one or more ring atoms are heteroatoms selected from nitrogen, oxygen, or S(O)ₘ (wherein m is an integer of 0 to 2), and the remaining ring atoms are carbon. The term may contain one or more double bonds, and none of the rings has a fully conjugated π electron system. Preferably, the group is 6- to 14-membered, and more preferably 7- to 10-membered. According to the number of shared spiro atoms between the rings, the spiroheterocyclyl is divided into monospiroheterocyclyl, bispiroheterocyclyl, or polyspiroheterocyclyl, preferably monospiroheterocyclyl and bispiroheterocyclyl. More preferably, it is a 3-membered/5-membered, 3-membered/6-membered, 4-membered/4-membered, 4-membered/5-membered, 4-membered/6-membered, 5-membered/5-membered, or 5-membered/6-membered monospiroheterocyclyl. Non-limiting examples of spiroheterocyclyl include: 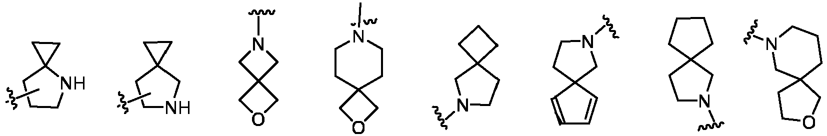 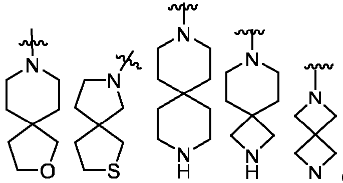 etc.

The term "fused heterocyclyl" refers to 5- to 20-membered polycyclic heterocyclyl with each ring in the system sharing a pair of atoms neighboring other rings in the system, and one or more rings may contain one or more double bonds, and none of the rings has a fully conjugated π electron system, wherein one or more ring atoms are heteroatoms selected from nitrogen, oxygen, or S(O)ₘ (wherein m is an integer of 0 to 2), and the remaining ring atoms are carbon. Preferably, the group is 6- to 14-membered, and more preferably 7- to 10-membered. According to the number of constituent rings, it can be divided into bicyclic, tricyclic, tetracyclic or polycyclic fused heterocyclyl, preferably bicyclic or tricyclic, more preferably 5-membered/5-membered or 5-membered/6-membered bicyclic fused heterocyclyl. Non-limiting examples of fused heterocyclyl include: 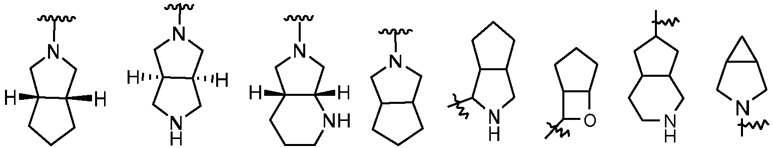 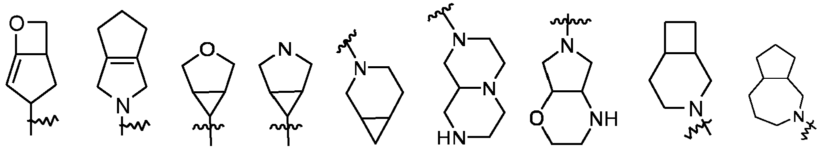 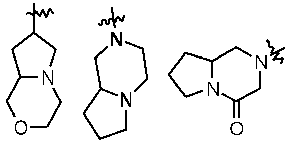 etc.

The term "bridged heterocyclyl" refers to a 5- to 14-membered polycyclic heterocyclyl with any two rings sharing two atoms that are not directly connected. It may contain one or more double bonds, and none of the rings has a fully conjugated π electron system, wherein one or more ring atoms are heteroatoms selected from nitrogen, oxygen, or S(O)ₘ (wherein m is an integer of 0 to 2), and the remaining ring atoms are carbon. Preferably, the group is 6- to 14-membered, and more preferably 7- to 10-membered. According to the number of constituent rings, it can be divided into bicyclic, tricyclic, tetracyclic or polycyclic bridged heterocyclyl, preferably bicyclic, tricyclic or tetracyclic, and more preferably bicyclic or tricyclic. Non-limiting examples of bridged heterocyclyl include: 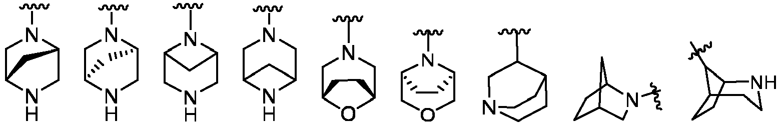 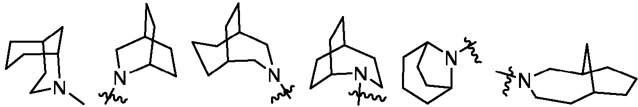 etc.

The heterocyclyl ring can be fused to an aryl, heteroaryl or cycloalkyl ring, wherein the ring connected to the parent structure is heterocyclyl, and the non-limiting examples thereof include: 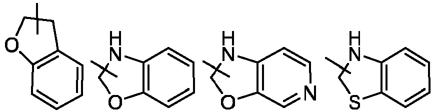 etc.

Heterocyclyl can be optionally substituted or unsubstituted. When the heterocyclyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group.

The term "aryl" refers to a 6- to 14-membered all-carbon monocyclic or fused polycyclic (i.e., rings sharing an adjacent pair of carbon atoms) group having a conjugated π electron system, preferably 6- to 12-membered aryl, such as phenyl and naphthyl, and more preferably phenyl. The aryl ring can be fused to heteroaryl, heterocyclyl or cycloalkyl ring, including benzo 5- to -10-membered heteroaryl, benzo 3- to -8-membered cycloalkyl and benzo 3- to -8-membered heteroalkyl, preferably benzo 5- to -6-membered heteroaryl, benzo 3- to -6-membered cycloalkyl, and benzo 3- to -6-membered heteroalkyl, wherein the heterocyclyl is heterocyclyl containing 1-3 nitrogen atoms, oxygen atoms, or sulfur atoms; or further including a three-membered nitrogen-containing fused ring containing a benzene ring,
wherein the ring connected to the parent structure is an aryl ring, and the non-limiting examples thereof include: 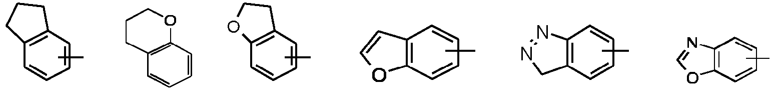 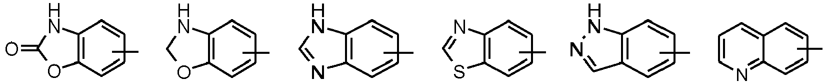 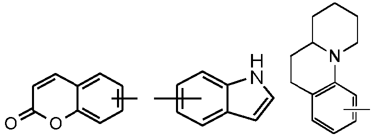 etc.

Aryl can be substituted or unsubstituted. When the aryl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

The term "heteroaryl" refers to a heteroaromatic system containing 1 to 4 heteroatoms and 5 to 14 ring atoms, wherein the heteroatoms are selected from oxygen, sulfur, and nitrogen. The heteroaryl is preferably 5- to 12-membered, more preferably 5-membered or 6-membered, such as imidazolyl, furyl, thienyl, thiazolyl, pyrazolyl, oxazolyl, pyrrolyl, triazolyl, tetrazolyl, pyridyl, pyrimidinyl, thiadiazole, pyrazinyl, etc., preferably triazolyl, thienyl, imidazolyl, pyrazolyl, oxazolyl, pyrimidinyl, or thiazolyl; more preferably, pyrazolyl, pyrrolyl, and oxazolyl. The heteroaryl ring can be fused to an aryl, heterocyclyl, or cycloalkyl ring, wherein the ring connected to the parent structure is heteroaryl ring, and the non-limiting examples thereof include: 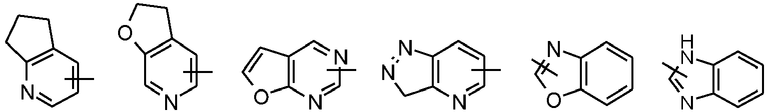 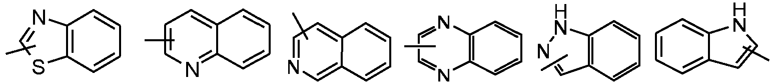 etc.

Heteroaryl can be optionally substituted or unsubstituted. When the heteroaryl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropoxy, cyclobutoxy, cyclopentoxy, and cyclohexoxy. Alkoxy can be optionally substituted or unsubstituted. When the alkoxy is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

"Haloalkyl" refers to alkyl substituted with one or more halogen, wherein the alkyl is defined as above.

"Haloalkoxy" refers to alkoxy substituted with one or more halogen, wherein the alkoxy is defined as above.

"Hydroxyalkyl" refers to alkyl substituted with hydroxyl, wherein the alkyl is defined as above.

"Alkenyl" refers to an alkene group, also known as an olefinic group, which is a linear or branched unsaturated aliphatic hydrocarbon group containing at least one carbon-carbon double bond that can be located at any position within the alkenyl group. Alkenyl is a linear or branched unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), 2 to 4 (C₂₋₄), or 2 to 3 (C₂₋₃) carbon atoms. Non-limiting examples of alkenyl include: 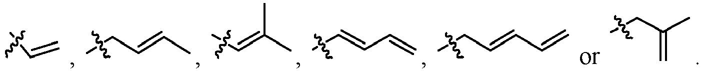 The alkenyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

"Alkynyl" refers to (CH=C-), which contains at least one carbon-carbon triple bond that can be located any position in the alkynyl and contains at least one carbon-carbon double bond that can be located at any position within the alkenyl group. The alkynyl is a linear or branched unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), 2 to 4 (C₂₋₄), or 2 to 3 (C₂₋₃) carbon atoms. Non-limiting examples of alkynyl include: 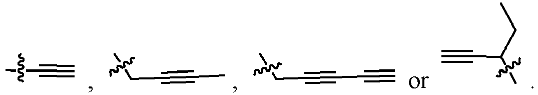 The alkynyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

The term "alkenylcarbonyl" refers to -C(O)-(alkenyl), wherein the alkenyl is defined as above. Non-limiting examples of alkenylcarbonyl include: ethenylcarbonyl, propenylcarbonyl, and butenylcarbonyl. Alkenylcarbonyl can be optionally substituted or unsubstituted. When the alkenylcarbonyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.
" 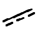 " refers to a single bond or a double bond.
"Hydroxyl" refers to an -OH group.
"Halogen" refers to fluorine, chlorine, bromine, or iodine.
"Amino" refers to -NH₂.
"Cyano" refers to -CN.
"Nitro" refers to -NO₂.
"Carbonyl" refers to -C(O)-.
"Carboxyl" refers to -C(O)OH.

Different terms such as "X is selected from A, B, or C", "X is selected from A, B and C", "X is A, B or C", and "X is A, B and C" all express the same meaning, that is, X can be any one or more of A, B, and C.

The hydrogen atoms of the present invention can be replaced with the isotope deuterium thereof, and any hydrogen atom in the example compounds involved in the present invention can also be replaced with a deuterium atom.

"Optional" or "optionally" means that the event or circumstance subsequently described may but need not to occur, and the description includes the occasion where the event or circumstance occurs or does not occur. For example, "heterocyclyl optionally substituted with alkyl" means the alkyl may be present but be not necessarily present, and the description includes the case where the heterocyclyl is substituted with alkyl and the case where the heterocyclyl is not substituted with alkyl.

"Substituted" means that one or more hydrogen atoms, preferably at most 5, more preferably 1-3 hydrogen atoms in the group are each independently substituted with a corresponding number of substituents. It goes without saying, the substituents may be only in possible chemical positions thereof, and those skilled in the art can determine the possible or impossible substitutions (by experiment or theory) without paying too much effort. For example, the amino group having a free hydrogen or a hydroxyl group may be unstable when combined the carbon atoms having an unsaturated (e.g., olefinic) bond.

"Pharmaceutical composition" denotes a mixture containing one or more compounds described herein or physiologically/pharmaceutically acceptable salts or prodrug thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

"Pharmaceutically acceptable salts" and "pharmaceutical salts" refer to salts of the compounds of the present invention, which are safe and effective when used in mammals, and have appropriate biological activity.

### Detailed Description of Embodiments

### Examples

The structures of compounds are determined by nuclear magnetic resonance (NMR) or/and mass spectrometry (MS). The NMR shift (δ) is given in 10⁻⁶ (ppm). NMR was determined using a Bruker AVANCE-400 nuclear magnetic instrument. The solvents for determination were deuterated dimethyl sulfoxide (DMSO-d6), deuterated chloroform (CDCl₃), and deuterated methanol (CD₃OD), and the internal standard was tetramethylsilane (TMS).

For MS determination, FINNIGAN LCQAd (ESI) mass spectrometer (manufacturer: Thermo, model: Finnigan LCQ advantage MAX) is used.

For HPLC determination, Agilent 1200DAD high-pressure liquid chromatograph instrument (Sunfire C18 150 × 4.6 mm chromatographic column) and Waters 2695-2996 high-pressure liquid chromatograph instrument (Gimini C18 150 × 4.6 mm chromatographic column) are used.

The average kinase inhibition rate and IC₅₀ value were measured using NovoStar microplate reader (BMG Company, Germany).

Yantai Huanghai HSGF254 or Qingdao GF254 silica gel plate is used as a thin layer chromatography silica plate, and the silica gel plate for thin layer chromatography (TLC) has a specification of 0.15 mm-0.2 mm, and the specification when separating and purifying a product by thin layer chromatography is 0.4 mm-0.5 mm.

For column chromatography, Yantai Huanghai silica gel of 200-300 mesh silica gel is generally used as a carrier.

The known starting materials of the present invention can be synthesized by methods known in the art, or can be purchased from ABCR GmbH & Co. KG, Acros Organics, Aldrich Chemical Company, Accela ChemBio Inc., Chembee Chemicals Inc. and other companies.

Unless otherwise specially specified in examples, reactions can all be carried out in an argon atmosphere or a nitrogen atmosphere.

Argon atmosphere or nitrogen atmosphere means that a reaction flask is connected to an argon or nitrogen balloon with a volume of about 1 L.

The hydrogen atmosphere means that a reaction flask is connected to a hydrogen balloon with a volume of about 1 L.

In a pressurized hydrogenation reaction, Parr 3916EKX hydrogenator and Qinglan QL-500 hydrogen generator or HC2-SS hydrogenator are used.

For a hydrogenation reaction, evacuation to a vacuum and filling with hydrogen are usually carried out, and the operation is repeated three times.

For a microwave reaction, CEM Discover-S 908860 microwave reactor is usually used.

Unless otherwise specially specified in examples, a solution refers to an aqueous solution.

Unless otherwise specially specified in examples, the reaction temperature is room temperature, which is 20-30°C.

The progress of a reaction in the examples is monitored by thin layer chromatography (TLC). Developing agent systems used in the reaction include: A: dichloromethane and methanol system, B: n-hexane and ethyl acetate system, and C: petroleum ether and ethyl acetate system, D: acetone, the volume ratio of the solvents is adjusted according to the polarity of the compounds.

Eluent systems for column chromatography and developing agent systems for thin layer chromatography, which are used to purify compounds, include: A: dichloromethane and methanol system, B: n-hexane and ethyl acetate system, and C: dichloromethane and acetone system, wherein the volume ratio between the solvents can be adjusted depending on the polarity of the compound, or can also be adjusted by adding a small amount of basic or acidic reagents such as triethylamine and acetic acid.

### Intermediates

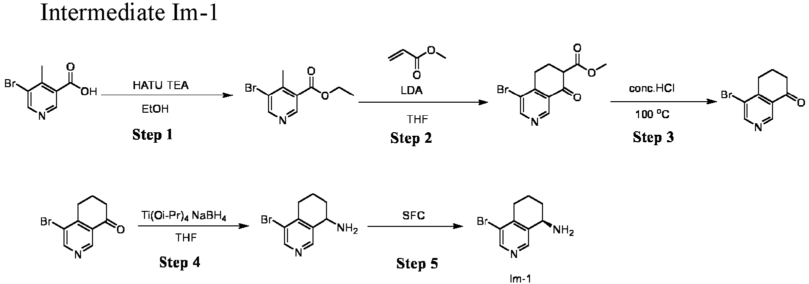

### Step 1. Ethyl 5-bromo-4-methyl-pyridine-3-carboxylate

5-Bromo-4-methyl-pyridine-3-carboxylic acid (20 g, 92.58 mmol) was dissolved in anhydrous N,N-dimethylformamide (200 mL), and anhydrous ethanol (42.65 g, 925.79 mmol, 54.06 mL), HATU (52.39 g, 138.87 mmol), and TEA (28.10 g, 277.74 mmol, 38.74 mL) were separately added thereto. The reaction system was stirred at room temperature 20°C for 16 hours. After the reaction was complete, the reaction was quenched by adding water (50 mL), and the reaction liquid was extracted with ethyl acetate (100 mL x 3), washed with a saturated aqueous sodium chloride solution (100 mL x 2), dried over anhydrous sodium sulfate, filtered, and concentrated to obtain crude ethyl 5-bromo-4-methyl-pyridine-3-carboxylate (21 g, crude).

MS m/z (ESI): 244.0, 246.0 [M+1].

### Step 2. Methyl 4-bromo-8-carbonyl-6,7-dihydro-5H-isoquinoline-7-carboxylate

Ethyl 5-bromo-4-methyl-pyridine-3-carboxylate (7 g, 28.68 mmol) was dissolved in anhydrous tetrahydrofuran (200 mL), lithium diisopropylamide (2 M, 17.21 mL) was dropwise added thereto at -78°C, stirring was maintained at -78°C for 1 hour, and methyl acrylate (6.17 g, 71.70 mmol, 6.46 mL) was then slowly added thereto. The reaction system was naturally warmed to room temperature and stirring was continued for 5 hours. After the reaction was complete, the reaction was quenched by slowly dropwise adding water (20 mL) thereto, extracted with ethyl acetate (50 mL x 3), washed with a saturated aqueous sodium chloride solution (50 mL x 2), dried over anhydrous sodium sulfate, filtered, and concentrated to obtain a crude product. The crude product was purified by a column (PE/EtOAc = 3 : 1, uv = 254 nm) to finally obtain the product methyl 4-bromo-8-carbonyl-6,7-dihydro-5H-isoquinoline-7-carboxylate (4 g, 14.0 mmol, 48.8% yield).

MS m/z (ESI): 284.0, 286.0 [M+1].

### Step 3. 4-Bromo-6,7-dihydro-5H-isoquinolin-8-one

Methyl 4-bromo-8-carbonyl-6,7-dihydro-5H-isoquinoline-7-carboxylate (7 g, 24.64 mmol) was dissolved in hydrochloric acid (6 M) (30 mL). The reaction system was stirred in an oil bath at 100°C for 2 hours. After the reaction was complete, the reaction liquid was cooled to room temperature, adjusted to pH 7-8 with a 6 M sodium hydroxide solution, washed with ethyl acetate (50 mL x 3) and a saturated aqueous sodium chloride solution (50 mL x 2), dried over anhydrous sodium sulfate, filtered, and concentrated to obtain crude 4-bromo-6,7-dihydro-5H-isoquinolin-8-one (4.8 g, 21.23 mmol, 86.17% yield). The crude product was directly used for the next reaction.

MS m/z (ESI): 226.0, 228.0 [M+1].

### Step 4. 4-Bromo-5,6,7,8-tetrahydroisoquinolin-8-amine

4-Bromo-6,7-dihydro-5H-isoquinolin-8-one (7 g, 30.96 mmol) was dissolved in a solution of ammonia in methanol (2 M, 100 mL), and tetraisopropyl titanate (17.60 g, 61.93 mmol, 18.33 mL) was added thereto. After the reaction system was stirred at 20°C for 16 hours, sodium borohydride (1.76 g, 46.45 mmol) was added thereto in portions in an ice-water bath. The reaction system was further stirred for 2 hours at room temperature. After the reaction was complete, the reaction was quenched by adding water (20 mL),
and the reaction liquid was filtered through diatomite, extracted with ethyl acetate (50 mL x 3), washed with a saturated aqueous sodium chloride solution (50 mL x 2), dried over anhydrous sodium sulfate, filtered, and concentrated to obtain a crude product. The crude product was purified by a column (dichloromethane : methanol = 10 : 1, uv = 254 nm) to obtain 4-bromo-5,6,7,8-tetrahydroisoquinolin-8-amine (4.5 g, 19.82 mmol, 63.99% yield).

MS m/z (ESI): 227.0, 229.0 [M+1].

### Step 5. (R)-4-bromo-5,6,7,8-tetrahydroisoquinolin-8-amine

4-Bromo-5,6,7,8-tetrahydroisoquinolin-8-amine (4.5 g, 19.82 mmol) was subjected to the following chiral separation to obtain the products P1 and Im-1, respectively.

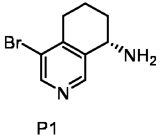

| Column type | Mobile phase | Flow rate | Detection wavelength | Tempe rature | Retention time of P1 | Retention time of Im-1 | Linear gradient |
|---|---|---|---|---|---|---|---|
| CHIRALPA K IB-H, 4.6 × 250 mm, 5 µm | Hexane: EtOH : MeOH = 70 : 15 : 15 | 1.0 ml/min | 214 nm | 35°C | 3.440 min | 3.740 min | Isometric |

MS m/z (ESI): 227.0, 229.0 [M+1].

### Example 1

### N-(4-(1-methyl-2-carbonyl-1,4-dihydro-2H-benzo[d][1,3]oxazin-6-yl)-5,6,7,8-tetrahydroisoquinolin-8-yl)propanamide

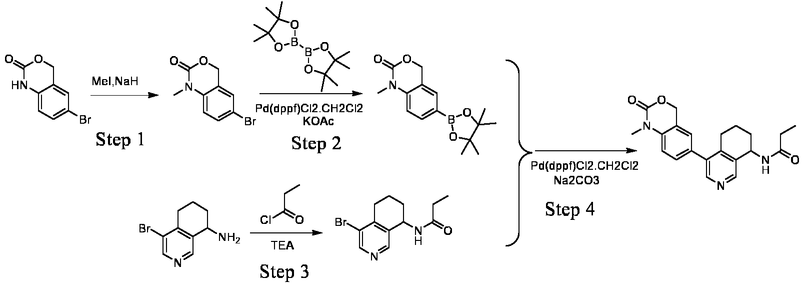

### Step 1

### 6-Bromo-1-methyl-1,4-dihydro-2H-benzo[d][1,3]oxazin-2-one

In a 25 mL reaction flask, 6-bromo-1,4-dihydro-2H-benzo[d][1,3]oxazin-2-one (500 mg, 2.19 mmol) and iodomethane (622.45 mg, 4.39 mmol) were dissolved in tetrahydrofuran (5 mL), sodium hydride (78.92 mg, 3.29 mmol, 60% purity) was then added at 0°C, and the reaction liquid was stirred at 25°C for 10 hours. After the reaction was stopped, the reaction was quenched by adding water (5 mL), and the reaction liquid was extracted with ethyl acetate (5 mL × 2). The combined organic phases were washed with saturated sodium chloride (5 mL), dried over anhydrous sodium sulfate, and filtered, and the resulting residue was purified by silica gel column chromatography with petroleum ether and ethyl acetate as an eluent system to obtain the title product 6-bromo-1-methyl-1,4-dihydro-2H-benzo[d][1,3]oxazin-2-one (410 mg, a yellow solid), yield: 77.2%.

MS m/z (ESI): 242.0, 244.0 [M+1].

### Step 2

### 1-Methyl-6-(4,4,5,5-tetramethyl-1,3,2-dioxaborolan-2-yl)-1,4-dihydro-2H-benzo[d][1,3]oxazin-2-one

6-Bromo-1-methyl-1,4-dihydro-2H-benzo[d][1,3]oxazin-2-one (410 mg, 1.69 mmol), bis(pinacolato)diboron (860.2 mg, 3.39 mmol), palladium acetate (498.67 mg, 5.08 mmol), and [1,1'-bis(diphenylphosphino)ferrocene]palladium dichloride dichloromethane complex (68.65 mg, 84.69 µmol) were dissolved in dioxane (10 mL), and after displacement three times with nitrogen, the reaction liquid was stirred at 90°C for 10 hours. After the reaction was stopped, the reaction liquid was cooled to room temperature, filtered, and concentrated under reduced pressure, and the resulting residue was purified by silica gel column chromatography with petroleum ether and ethyl acetate as an eluent system to obtain the title product 1-methyl-6-(4,4,5,5-tetramethyl-1,3,2-dioxaborolan-2-yl)-1,4-dihydro-2H-benzo[d][1,3]oxazin-2-one (300 mg, yellow solid), yield: 61.2%.

MS m/z (ESI): 290.1 [M+1].

### Step 3

### N-(4-bromo-5,6,7,8-tetrahydroisoquinolin-8-yl)propanamide

In a 25 mL reaction flask, 4-bromo-5,6,7,8-tetrahydroisoquinolin-8-amine (500 mg, 2.20 mmol) and triethylamine (445.58 mg, 4.40 mmol) were dissolved in dichloromethane (5 mL), and propionyl chloride (224.07 mg, 2.42 mmol) was dropwise then added. The reaction liquid was stirred at 25°C for 3 hours. After the reaction was stopped, the reaction was quenched by adding water (5 mL), and the reaction liquid was extracted with dichloromethane (5 mL × 2). The combined organic phases were washed with saturated sodium chloride (5 mL), dried over anhydrous sodium sulfate, and filtered, and the resulting residue was purified by silica gel column chromatography with petroleum ether and ethyl acetate as an eluent system to obtain the title product N-(4-bromo-5,6,7,8-tetrahydroisoquinolin-8-yl)propanamide (500 mg), yield: 80.2%.

MS m/z (ESI): 283.0, 285.0 [M+1].

### Step 4

### N-(4-(1-methyl-2-carbonyl-1,4-dihydro-2H-benzo[d][1,3]oxazin-6-yl)-5,6,7,8-tetrahydroisoquinolin-8-yl)propanamide

N-(4-bromo-5,6,7,8-tetrahydroisoquinolin-8-yl)propanamide (100 mg, 353.15 µmol), 1-methyl-6-(4,4,5,5-tetramethyl-1,3,2-dioxaborolan-2-yl)-1,4-dihydro-2H-benzo[d][1,3]oxazin-2-one (102.11 mg, 353.15 µmol), Na₂CO₃ (112.29 mg, 1.06 mmol), and [1,1'-bis(diphenylphosphino)ferrocene]palladium dichloride dichloromethane complex (14.31 mg, 17.66 µmol) were dissolved in dioxane (5 mL) and water (1 mL), and after displacement three times with nitrogen, the reaction liquid was stirred at 90°C for 10 hours. After the reaction was stopped, the reaction was quenched by adding water (5 mL) and extracted with ethyl acetate (5 mL × 2). The combined organic phases were washed with saturated sodium chloride (5 mL), dried over anhydrous sodium sulfate, and filtered, and the resulting residue was purified by silica gel column chromatography with petroleum ether and ethyl acetate as an eluent system to obtain the title product N-(4-(1-methyl-2-carbonyl-1,4-dihydro-2H-benzo[d][1,3]oxazin-6-yl)-5,6,7,8-tetrahydroisoquinolin-8-yl)propanamide (75 mg), yield: 58.2%.

MS m/z (ESI): 366.2 [M+1].

¹HNMR (400 MHz, DMSO) δ 8.26 (s, 1H), 8.22 (d, 1H), 8.14 (s, 1H), 7.30 (dd, 1H), 7.23 (d, 1H), 7.12 (d, 1H), 5.23 (s, 2H), 5.02 (q, 1H), 3.25 (s, 3H), 2.53 (q, 2H), 2.11-2.05 (m, 2H), 1.84 - 1.61 (m, 4H), 0.98 (t, 3H).

Example 1 was resolved to obtain 1-A and 1-B.

| | | |
|---|---|---|
| 1-A | 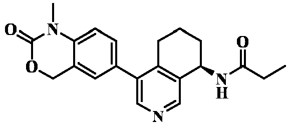 | MS m/z (ESI): 366.2 [M+1]. |
| 1-B | 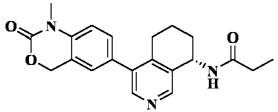 | MS m/z (ESI): 366.2 [M+1]. |

### Example 2

### N-(4-(4-methyl-3-carbonyl-3,4-dihydro-2H-benzo[b][1,4]oxazin-7-yl)-5,6,7,8-tetrahydroisoquinolin-8-yl)propanamide

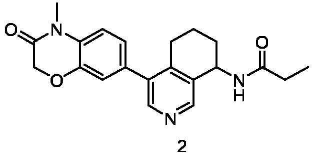

Referring to the synthesis route of Example 1, 6-bromo-1,4-dihydro-2H-benzo[d][1,3]oxazin-2-one was replaced with 7-bromo-2H-benzo[b][1,4]oxazin-3(4H)-one to obtain the title product N-(4-(4-methyl-3-carbonyl-3,4-dihydro-2H-benzo[b][1,4]oxazin-7-yl)-5,6,7,8-tetrahydroisoquinolin-8-yl)propanamide.

MS m/z (ESI): 366.2 [M+1].

¹H NMR (400 MHz, DMSO) δ 8.33 (s, 1H), 8.27 (d, 1H), 8.20 (s, 1H), 7.25 (d, 1H), 7.05 (dd, 1H), 7.01 (d, 1H), 5.09 (q, 1H), 4.71 (s, 2H), 3.32 (s, 3H), 2.60 (t, 2H), 2.23 - 2.08 (m, 2H), 1.95 - 1.60 (m, 4H), 1.05 (t, 3H).

Example 2 was resolved to obtain 2-A and 2-B.

| | | |
|---|---|---|
| 2-A | 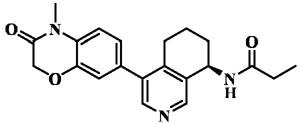 | MS m/z (ESI): 366.2 [M+1]. |
| 2-B | 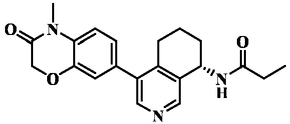 | MS m/z (ESI): 366.2 [M+1]. |

Referring to a similar preparation method to that in Example 1, Examples 3-56 were obtained.

| Example No. | Structure | LCMS (ESI, M+1) |
|---|---|---|
| 3 | 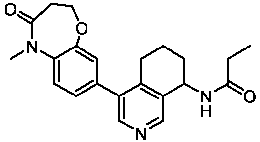 | 380.2 |
| 4 | 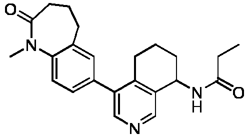 | 378.2 |
| 5 | 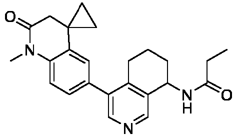 | 390.2 |
| 6 | 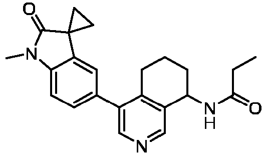 | 376.2 |
| 7 | 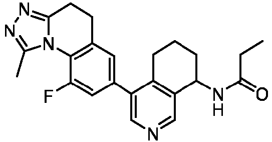 | 406.2 |
| 8 | 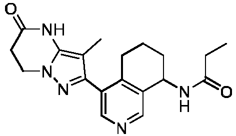 | 354.2 |
| 9 | 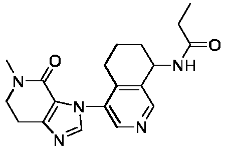 | 354.2 |
| 10 | 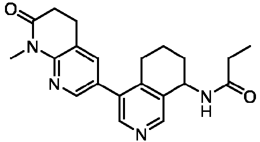 | 365.2 |
| 11 | 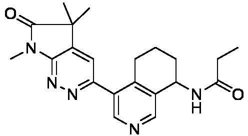 | 380.2 |
| 12 | 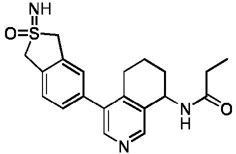 | 370.2 |
| 13 | 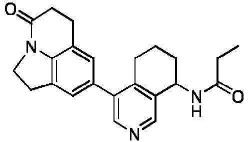 | 376.2 |
| 14 | 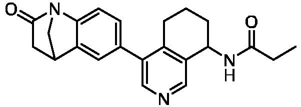 | 362.2 |
| 15 | 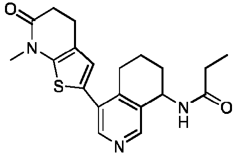 | 370.2 |
| 16 | 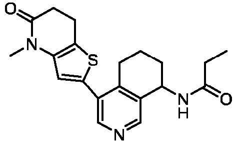 | 370.2 |
| 17 | 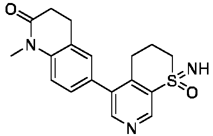 | 342.1 |
| 18 | 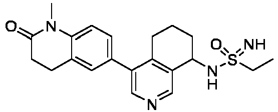 | 399.2 |
| 19 | 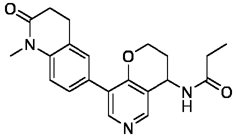 | 366.2 |
| 20 | 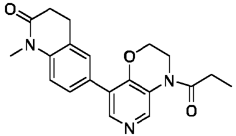 | 352.2 |
| 21 | 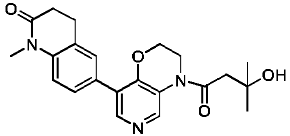 | 396.2 |
| 22 | 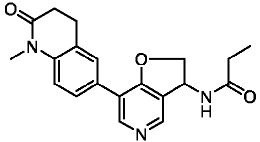 | 352.2 |
| 23 | 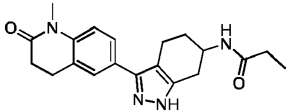 | 353.2 |
| 24 | 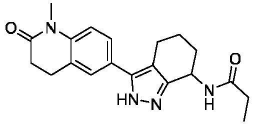 | 353.2 |
| 25 | 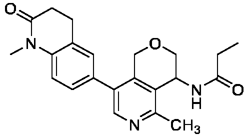 | 380.2 |
| 26 | 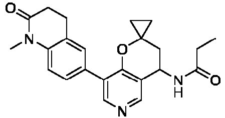 | 392.2 |
| 27 | 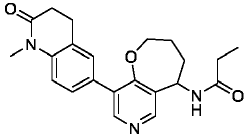 | 380.2 |
| 28 | 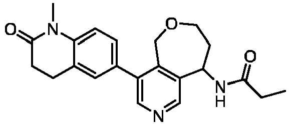 | 380.2 |
| 29 | 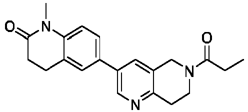 | 350.2 |
| 30 | 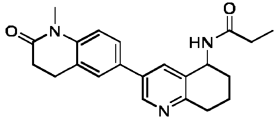 | 364.2 |
| 31 | 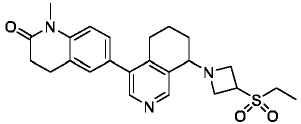 | 440.2 |
| 32 | 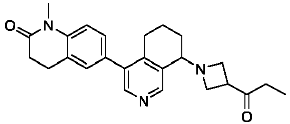 | 404.2 |
| 33 | 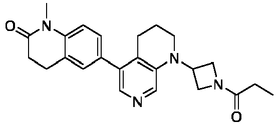 | 405.2 |
| 34 | 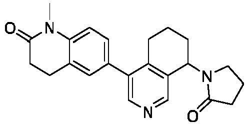 | 376.2 |
| 35 | 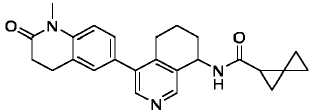 | 402.2 |
| 36 | 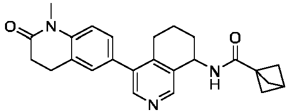 | 402.2 |
| 37 | 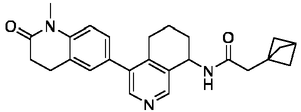 | 416.2 |
| 38 | 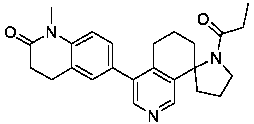 | 404.2 |
| 39 | 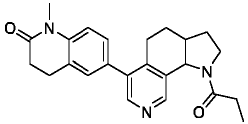 | 390.2 |
| 40 | 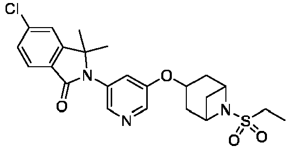 | 476.1 |
| 41 | 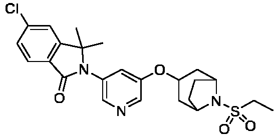 | 490.2 |
| 42 | 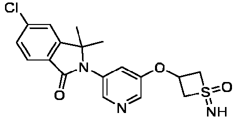 | 392.0 |
| 43 | 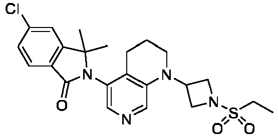 | 475.2 |
| 44 | 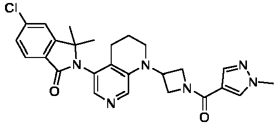 | 491.2 |
| 45 | 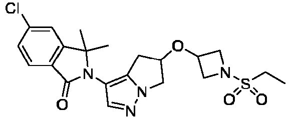 | 465.1 |
| 46 | 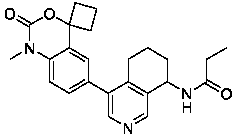 | 406.2 |
| 47 | 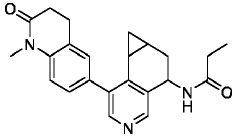 | 376.2 |
| 48 | 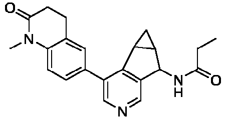 | 362.2 |
| 49 | 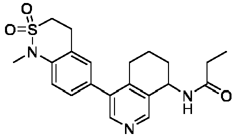 | 400.2 |
| 50 | 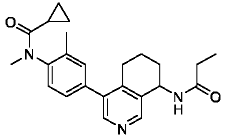 | 392.2 |
| 51 | 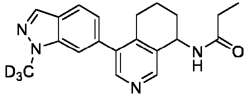 | 338.2 |
| 52 | 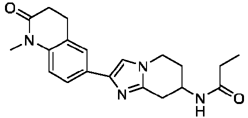 | 353.2 |
| 53 | 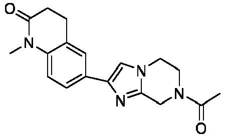 | 325.2 |
| 54 | 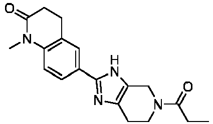 | 339.2 |
| 55 | 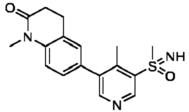 | 330.1 |
| 56 | 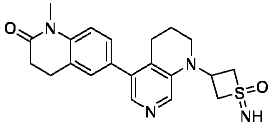 | 397.2 |

### Example 4

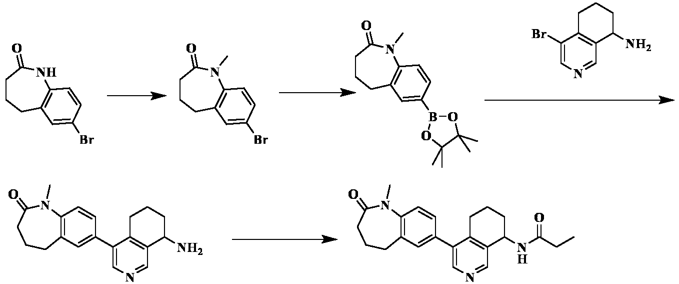

### Steps 1 and 2

### 1-Methyl-7-(4,4,5,5-tetramethyl-1,3,2-dioxaborolan-2-yl)-1,3,4,5-tetrahydro-2H-benzo[b]azepin-2-one.

With 7-bromo-1,3,4,5-tetrahydro-2H-benzo[b]azepin-2-one as a raw material, 1-methyl-7-(4,4,5,5-tetramethyl-1,3,2-dioxaborolan-2-yl)-1,3,4,5-tetrahydro-2H-benzo[b]azepin-2-one was obtained by the same operation to steps 1 and 2 in Example 1.

MS m/z (ESI): 302.2 [M+1].

### Step 3

### N-(4-(1-methyl-2-carbonyl-2,3,4,5-tetrahydro-1H-benzo[b]azepin-7-yl)-5,6,7,8-tetrahydroisoquinolin-8-yl)propanamide

7-(8-Amino-5,6,7,8-tetrahydroisoquinolin-4-yl)-1-methyl-1,3,4,5-tetrahydro-2H-benzo[b]azepin-2-one was obtained by the same operation to step 4 in Example 1.

MS m/z (ESI): 322.2 [M+1].

### Step 4

### N-(4-(1-methyl-2-carbonyl-2,3,4,5-tetrahydro-1H-benzo[b]azepin-7-yl)-5,6,7,8-tetrahydroisoquinolin-8-yl)propanamide

N-(4-(1-methyl-2-carbonyl-2,3,4,5-tetrahydro-1H-benzo[b]azepin-7-yl)-5,6,7,8-tetrahydroisoquinolin-8-yl)propanamide was obtained by the same operation to step 3 in Example 1.

MS m/z (ESI): 378.2 [M+1].

### Example 10

### N-(4-(8-methyl-7-carbonyl-5,6,7,8-tetrahydro-1,8-diazanaphth-3-yl)-5,6,7,8-tetrahydroisoquinolin-8-yl)propanamide

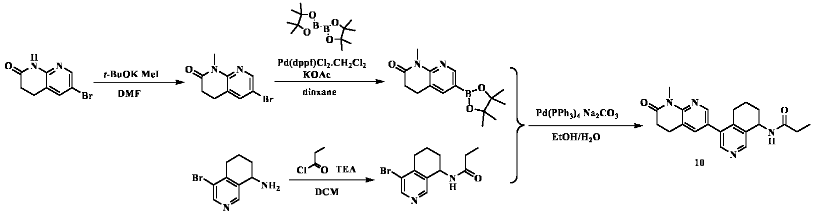

### Step 1

### 6-Bromo-1-methyl-3,4-dihydro-1,8-phthalazin-2(1H)-one

6-Bromo-3,4-dihydro-1,8-phthalazin-2(1H)-one (5 g, 22.02 mmol) was dissolved in N,N-dimethylformamide (100 mL), and in an ice-water bath, potassium tert-butoxide (4.94 g, 44.04 mmol) was slowly added thereto. After stirring for 0.5 hours, iodomethane (4.69 g, 33.03 mmol, 2.06 mL) was dropwise added thereto. The reaction system was further stirred at room temperature 20°C for 5.5 hours. After the reaction was complete, the reaction was quenched by slowly dropwise adding water (10 mL) under ice-water bath condition, and the reaction liquid was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 3 : 1, uv = 254 nm) to finally obtain 6-bromo-1-methyl-3,4-dihydro-1,8-phthalazin-2(1H)-one as a pale yellow solid (4 g, yield 75.3%).

MS m/z (ESI): 241.0, 243.0 [M+1].

### Step 2

### 1-Methyl-6-(4,4,5,5-tetramethyl-1,3,2-dioxaborolan-2-yl)-3,4-dihydro-1,8-phthalazin-2(1H)-one

6-Bromo-1-methyl-3,4-dihydro-1,8-phthalazin-2(1H)-one (2.5 g, 10.37 mmol) was dissolved in dioxane (50 mL), bis(pinacolato)diboron (3.16 g, 12.44 mmol), potassium acetate (2.04 g, 20.74 mmol), and [1,1'-bis(diphenylphosphino)ferrocene]palladium dichloride dichloromethane complex (752.49 mg, 1.04 mmol) were separately added thereto. After displacement multiple times with nitrogen, the reaction system was placed in an oil bath at 100°C and stirred for 16 hours. After the reaction was complete, the reaction was cooled to room temperature, filtered through diatomite, diluted by adding water (10 mL), extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 5 : 1, uv = 254 nm) to finally obtain 1-methyl-6-(4,4,5,5-tetramethyl-1,3,2-dioxaborolan-2-yl)-3,4-dihydro-1,8-phthalazin-2(1H)-one as a white solid (2.4 g, 8.33 mmol, 80.32% yield).

MS m/z (ESI): 289.1 [M+1].

### Step 3

### N-(4-bromo-5,6,7,8-tetrahydroisoquinolin-8-yl)propanamide

In a 25 mL reaction flask, 4-bromo-5,6,7,8-tetrahydroisoquinolin-8-amine (500 mg, 2.20 mmol) and triethylamine (445.58 mg, 4.40 mmol) were dissolved in dichloromethane (5 mL), and propionyl chloride (224.07 mg, 2.42 mmol) was dropwise then added. The reaction liquid was stirred at 25°C for 3 hours. After the reaction was stopped, the reaction was quenched by adding water (5 mL) and extracted with dichloromethane (5 mL × 2). The combined organic phases were washed with saturated sodium chloride (5 mL), dried over anhydrous sodium sulfate, and filtered, and the resulting residue was purified by silica gel column chromatography with petroleum ether and ethyl acetate as an eluent system to obtain the title product N-(4-bromo-5,6,7,8-tetrahydroisoquinolin-8-yl)propanamide (500 mg, a yellow solid), yield: 80.2%.

MS m/z (ESI): 283.0 [M+1].

### Step 4

### N-(4-(8-methyl-7-carbonyl-5,6,7,8-tetrahydro-1,8-diazanaphth-3-yl)-5,6,7,8-tetrahydroisoquinolin-8-yl)propanamide

1-Methyl-6-(4,4,5,5-tetramethyl-1,3,2-dioxaborolan-2-yl)-3,4-dihydro-1,8-phthalazin-2(1H)-one (333.06 mg, 1.16 mmol) and N-(4-bromo-5,6,7,8-tetrahydroisoquinolin-8-yl)propanamide (328 mg, 1.16 mmol) were dissolved in a mixed solvent of H₂O (5 mL) and EtOH (25 mL), and sodium carbonate (246 mg, 2.32 mmol) and tetrakis(triphenylphosphine)palladium (127.21 mg, 110.08 µmol) were sequentially added thereto. After displacement multiple times with nitrogen, the reaction system was placed in an oil bath at 100°C and stirred for 16 hours. After the reaction was complete, the reaction liquid was cooled to room temperature, diluted by adding water (10 mL), extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (DCM/MeOH = 10 : 1, UV = 254 nm) to finally obtain N-(4-(8-methyl-7-carbonyl-5,6,7,8-tetrahydro-1,8-diazanaphth-3-yl)-5,6,7,8-tetrahydroisoquinolin-8-yl)propanamide (200 mg, 58.92% yield).

MS m/z (ESI): 365.1 [M+1].

¹H NMR (400 MHz, DMSO) δ 8.36 (s, 1H), 8.29 (d, 1H), 8.25 (s, 1H), 8.22 (d, 1H), 7.72 (d, 1H), 5.10 (q, 1H), 3.36 (s, 3H), 2.95 (dd, 2H), 2.73 - 2.59 (m, 4H), 2.15 (qd, 2H), 1.96 - 1.60 (m, 4H), 1.05(t, 3H).

Example 10 was resolved to obtain 10-A and 10-B.

| | | |
|---|---|---|
| 10-A | 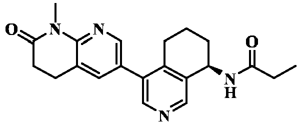 | MS m/z (ESI): 365.1 [M+1]. |
| 10-B | 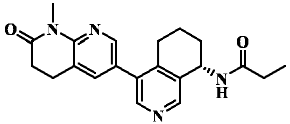 | MS m/z (ESI): 365.1 [M+1]. |

### Example 37

### (R)-2-(bicyclo[1.1.1]pentan-1-yl)-N-(4-(1-methyl-2-carbonyl-1,2,3,4-tetrahydroisoquinolin-6-yl)-5,6,7,8-tetrahydroisoquinolin-8-yl)acetamide

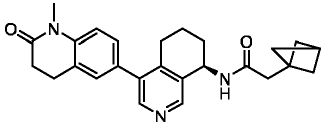

With intermediate Im-1 as a raw material, the product (R)-2-(bicyclo[1.1.1]pentan-1-yl)-N-(4-(1-methyl-2-carbonyl-1,2,3,4-tetrahydroisoquinolin-6-yl)-5,6,7,8-tetrahydroisoquinolin-8-yl)acetamide was obtained according to Example 1.

MS m/z (ESI): 416.2 [M+1].

### Example 48

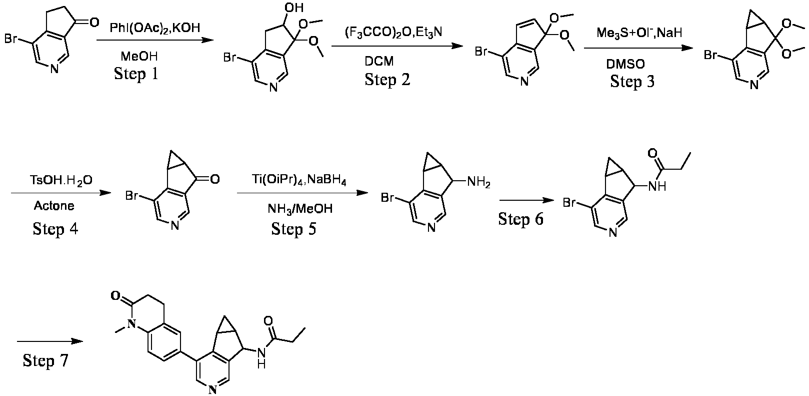

### Step 1

### 4-Bromo-7,7-dimethoxy-6,7-dihydro-5H-cyclopenta[c]pyridin-6-ol

Potassium hydroxide (5.29 g, 94.32 mmol) was dissolved in methanol (50 mL), and under ice-water bath cooling and nitrogen protection, 4-bromo-5,6-dihydro-7H-cyclopenta[c]pyridin-7-one was added. The mixture was reacted with stirring for 5 minutes at 0°C. Iodobenzene acetate (6.08 g, 18.86 mmol) was then added. The mixture was reacted with stirring for 4 hours at 18°C. The reaction liquid was evaporated to dryness at a low temperature, and the reaction was quenched by adding a saturated aqueous sodium chloride solution (100 mL) to the crude product and extracted with ethyl acetate (50 mL × 3). The mixture was separated. The organic phases were combined and washed with a saturated aqueous sodium chloride solution (100 mL × 2), and the organic phase was dried over anhydrous sodium sulfate and concentrated under reduced pressure. The residue was purified by rapid silica gel chromatography (eluted with dichloromethane:methanol = 100: 0 to 95: 5) to obtain the target product 4-bromo-7,7-dimethoxy-6,7-dihydro-5H-cyclopenta[c]pyridin-6-ol (1.5 g, a brown oil), yield: 58.02%.

MS m/z (ESI): 274.0, 276.0 [M+1].

### Step 2

### 4-Bromo-7,7-dimethoxy-7H-cyclopenta[c]pyridine

4-Bromo-7,7-dimethoxy-6,7-dihydro-5H-cyclopenta[c]pyridin-6-ol (1.5 g, 5.47 mmol) and triethylamine (2.77 g, 27.36 mmol, 3.82 mL) were dissolved in dichloromethane (60 mL), and under ice-water bath cooling and nitrogen protection, trifluoroacetic anhydride (2.30 g, 10.94 mmol, 1.52 mL) was added. The mixture was reacted with stirring for 12 hours at 20°C. The reaction was quenched by adding a saturated aqueous sodium bicarbonate solution (150 mL) to the mixture. The organic phase was separated, sequentially washed with a saturated aqueous sodium chloride solution (50 mL × 2), dried over anhydrous sodium sulfate, and concentrated under reduced pressure. The residue was purified by rapid silica gel chromatography (eluted with petroleum ether:ethyl acetate = 100 : 0 to 80 : 20) to obtain the target product 4-bromo-7,7-dimethoxy-7H-cyclopenta[c]pyridine (1.3 g), yield: 92.76%.

MS m/z (ESI): 256.0, 258.0 [M+1].

### Step 3

### 4-Bromo-6,6-dimethoxy-4b,5,5a,6-tetrahydrocyclopropa[3,4]cyclopenta[1,2-c]pyridine

Trimethylsulfoxonium iodide (3.35 g, 15.23 mmol) was dissolved in dimethyl sulfoxide (20 mL), and under nitrogen protection, sodium hydride (609.09 mg, 15.23 mmol, 60% w/w) was added. The mixture was reacted with stirring for 3 hours at 18°C. Under nitrogen protection, 4-bromo-7,7-dimethoxy-7H-cyclopenta[c]pyridine (1.30 g, 5.08 mmol) was added. The mixture was reacted with stirring for 12 hours at 18°C. The reaction liquid was quenched with a saturated aqueous sodium chloride solution (130 mL) and extracted with ethyl acetate (50 mL × 2). The organic phase was separated, washed with a saturated aqueous sodium chloride solution (50mL × 5), dried over anhydrous sodium sulfate, and concentrated under reduced pressure to obtain crude 4-bromo-6,6-dimethoxy-4b,5,5a,6-tetrahydrocyclopropa[3,4]cyclopenta[1,2-c]pyridine (1.1 g). The crude product was directly used for the next step.

MS m/z (ESI): 270.0, 272.0 [M+1].

### Step 4

### 4-Bromo-5,5a-dihydrocyclopropa[3,4]cyclopenta[1,2-c]pyridin-6(4bH)-one

4-Bromo-6,6-dimethoxy-4b,5,5a,6-tetrahydrocyclopropa[3,4]cyclopenta[1,2-c]pyridine (1.1 g, 4.07 mmol) was dissolved in acetone (30 mL), and under nitrogen protection, p-toluenesulfonic acid monohydrate (774.61 mg, 4.07 mmol) was added. The mixture was reacted with stirring for 2 hours at 18°C. The mixture was evaporated to dryness at a low temperature, the reaction was quenched by adding a saturated aqueous sodium chloride solution (50 mL), and the reaction liquid was extracted with dichloromethane (50 mL × 2). The organic phases were combined, sequentially washed with a saturated aqueous sodium chloride solution (50 mL × 2), dried over anhydrous sodium sulfate, and concentrated under reduced pressure. The residue was purified by rapid silica gel chromatography (eluted with petroleum ether:ethyl acetate = 100 : 0 to 75 : 25) to obtain the target product 4-bromo-5,5a-dihydrocyclopropa[3,4]cyclopenta[1,2-c]pyridin-6(4bH)-one (0.55 g, a pale brown solid), yield: 60.28%.

MS m/z (ESI): 224.0, 226.0 [M+1].

### Step 5

### 4-Bromo-4b,5,5a,6-tetrahydrocyclopropa[3,4]cyclopenta[1,2-c]pyridin-6-amine

4-Bromo-5,5a-dihydrocyclopropa[3,4]cyclopenta[1,2-c]pyridin-6(4bH)-one (0.55 g, 2.45 mmol) was dissolved in a solution of ammonia in methanol (2 M, 20 mL), and under nitrogen protection, tetraisopropyl titanate (2.16 g, 4.91 mmol) was added. The mixture was reacted with stirring for 3 hours at 60°C. The reaction liquid was cooled to room temperature, and sodium borohydride (185.74 mg, 4.91 mmol) was then added. The mixture was reacted with stirring for hours at 20°C. The reaction liquid was evaporated to dryness, and the crude product was dissolved with dichloromethane (60 mL) and washed with a saturated aqueous sodium chloride solution (30 mL × 2). The organic phase was separated, dried over anhydrous sodium sulfate, and concentrated under reduced pressure to obtain crude 4-bromo-4b,5,5a,6-tetrahydrocyclopropa[3,4]cyclopenta[1,2-c]pyridin-6-amine (0.44 g). The crude product was directly used for the next step.

MS m/z (ESI): 225.0, 227.0 [M+1].

### Step 6

### N-(4-bromo-4b,5,5a,6-tetrahydrocyclopropa[3,4]cyclopenta[1,2-c]pyridin-6-yl)propanamide

With 4-bromo-4b,5,5a,6-tetrahydrocyclopropa[3,4]cyclopenta[1,2-c]pyridin-6-amine and propionyl chloride as raw materials, N-(4-bromo-4b,5,5a,6-tetrahydrocyclopropa[3,4]cyclopenta[1,2-c]pyridin-6-yl)propanamide was synthesized according to step 3 in Example 1.

MS m/z (ESI): 281.0, 283.0 [M+1].

### Step 7

### N-(4-(1-methyl-2-carbonyl-1,2,3,4-tetrahydroisoquinolin-6-yl)-4b,5,5a,6-tetrahydrocyclopropa[3,4]cyclopenta[1,2-c]pyridin-6-yl)propanamide

With N-(4-bromo-4b,5,5a,6-tetrahydrocyclopropa[3,4]cyclopenta[1,2-c]pyridin-6-yl)propanamide and 1-methyl-6-(4,4,5,5-tetramethyl-1,3,2-dioxaborolan-2-yl)-3,4-dihydroquinolin-2(1H)-one as raw materials, N-(4-(1-methyl-2-carbonyl-1,2,3,4-tetrahydroisoquinolin-6-yl)-4b,5,5a,6-tetrahydrocyclopropa[3,4]cyclopenta[1,2-c]pyridin-6-yl)propanamide was synthesized according to step 4 in Example 1.

MS m/z (ESI): 362.2 [M+1].

The sample was subjected to chiral separation as follows to obtain products P1 and P2.

| Column type | Mobile phase | Flow rate | Detection wavelength | Temper ature | Retention time of P1 | Retention time of P2 | Linear gradient |
|---|---|---|---|---|---|---|---|
| CHIRALCEL AS-H, 4.6 × 150 mm, 5HI | Hexane : EtOH = 70 : 30 | 1.0 ml/min | 214 nm | 35°C | 3.7 min | 5.5 min | Isometric |

The product P1 was subjected to chiral resolution under the following conditions to obtain P1A and P1B

| Column type | Mobile phase | Flow rate | Detection wavelength | Temper ature | Retention time of P1A | Retention time of P1B | Linear gradient |
|---|---|---|---|---|---|---|---|
| CHIRALPAK AD-H, 4.6 × 250 mm, 5RA | EtOH : MeOH = 50 : 50 | 0.5 ml/min | 214 nm | 35°C | 7.7 min | 9.8 min | Isometric |

The product P2 was subjected to chiral resolution under the following conditions to obtain P2A and P2B

| Column type | Mobile phase | Flow rate | Detection wavelength | Temper ature | Retention time of P2A | Retention time of P2B | Linear gradient |
|---|---|---|---|---|---|---|---|
| CHIRALPAK AD-H, 4.6 × 250 mm, 5 µm | EtOH : MeOH = 50 : 50 | 0.5 ml/min | 214 nm | 35°C | 10.1 min | 13.8 min | Isometric |

LCMS and HNMR of a P2B sample was as follows.

¹H NMR (400 MHz, CDCl₃) δ 8.48 (s, 1H), 8.40 (s, 1H), 7.50 (dd, J = 8.4, 2.0 Hz, 1H), 7.44 - 7.34 (m, 1H), 7.11 (d, J = 8.4 Hz, 1H), 6.04 - 5.88 (m, 1H), 5.77 - 5.58 (m, 1H), 3.41 (s, 3H), 3.00 (t, J = 7.4 Hz, 2H), 2.79 - 2.64 (m, 2H), 2.60 - 2.48 (m, 1H), 2.40 - 2.21 (m, 3H), 1.37 - 1.15 (m, 5H).

MS m/z (ESI): 362.2 [M+1].

### Example 57

### 1-Methyl-N-(4-(1-methyl-2-carbonyl-1,4-dihydro-2H-benzo[d][1,3]oxazin-6-yl)-5,6,7,8-tetrahydroisoquinolin-8-yl)-1H-pyrazole-4-carboxamide

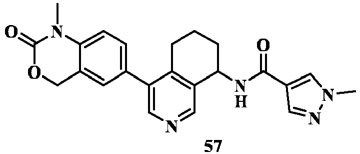

Referring to the synthesis route of Example 1, propionyl chloride was replaced with 1-methyl-1H-pyrazole-4-carbonyl chloride to obtain the title product 1-methyl-N-(4-(1-methyl-2-carbonyl-1,4-dihydro-2H-benzo[d][1,3]oxazin-6-yl)-5,6,7,8-tetrahydroisoquinolin-8-yl)-1H-pyrazole-4-carboxamide 57.

MS m/z (ESI): 418.2 [M+1].

### Example 58

### 6-(5-((1-(Ethylsulfonyl)azetidin-3-yl)oxy)pyridin-3-yl)-1-methyl-1,4-dihydro-2H-benzo[d][1,3]oxazin-2-one

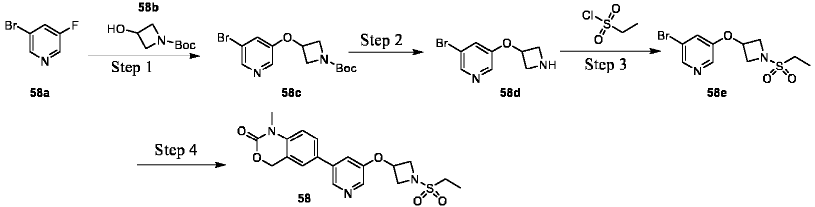

### Step 1

### Tert-butyl 3-((5-bromopyridin-3-yl)oxy)azetidine-1-carboxylate

Sodium hydride (693 mg, 17.34 mmol, 60%) was added to a solution of tert-butyl 3-hydroxyazetidine-1-carboxylate 58b (2 g, 11.56 mmol) in tetrahydrofuran (25 mL) and stirred at room temperature for 30 minutes, and a solution of 3-bromo-5-fluoropyridine 58a (1.6 g,9.25 mmol) in tetrahydrofuran (10 mL) was then dropwise added and stirred at room temperature overnight. Water was added. The mixture was extracted with dichloromethane (30 mL * 3). The organic phases were washed with a saturated aqueous sodium chloride solution, dried over anhydrous sodium sulfate, filtered, spun dry, and then separated by column chromatography to obtain the title product tert-butyl 3-((5-bromopyridin-3-yl)oxy)azetidine-1-carboxylate **58c** (1 g, a pale yellow solid), yield: 33%

MS m/z (ESI): 329.0 [M+1].

### Step 2

### 3-(Azetidin-3-yloxy)-5-bromopyridine

Trifluoroacetic acid (5 mL) was dropwise added to a solution of tert-butyl 3-((5-bromopyridin-3-yl)oxy)azetidine-1-carboxylate **58c** (1 g, 3.04 mmol) in dichloromethane (15 mL), and the mixture was stirred at room temperature for 1 hour, concentrated under reduced pressure, and dried to obtain the title product 3-(azetidin-3-yloxy)-5-bromopyridine **58d** (1.1 g, crude).

MS m/z (ESI): 229.0 [M+1].

### Step 3

### 3-Bromo-5-((1-(ethylsulfonyl)azetidin-3-yl)oxy)pyridine

Referring to step 3 of the synthesis route of Example 1, propionyl chloride was replaced with ethanesulfonyl chloride to obtain the title product 3-bromo-5-((1-(ethylsulfonyl)azetidin-3-yl)oxy)pyridine **58e**

MS m/z (ESI): 321.0 [M+1].

### Step 4

### 6-(5-((1-(Ethylsulfonyl)azetidin-3-yl)oxy)pyridin-3-yl)-1-methyl-1,4-dihydro-2H-benzo[d][1,3]oxazin-2-one

Referring to step 4 of the synthesis route of Example 1, N-(4-bromo-5,6,7,8-tetrahydroisoquinolin-8-yl)propanamide was replaced with 3-bromo-5-((1-(ethylsulfonyl)azetidin-3-yl)oxy)pyridine to obtain the title product 6-(5-((1-(ethylsulfonyl)azetidin-3-yl)oxy)pyridin-3-yl)-1-methyl-1,4-dihydro-2H-benzo[d][1,3]oxazin-2-one **58**

MS m/z (ESI): 404.1 [M+1].

### Example 59

### N-(4-(1-methyl-2-carbonyl-1,4-dihydro-2H-benzo[d][1,3]oxazin-6-yl)-5,6,7,8-tetrahydroisoquinolin-8-yl)acetamide

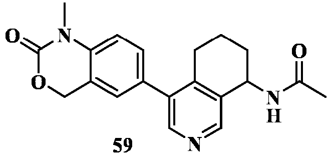

Referring to the synthesis route of Example 1, propionyl chloride was replaced with acetyl chloride to obtain the title product N-(4-(1-methyl-2-carbonyl-1,4-dihydro-2H-benzo[d][1,3]oxazin-6-yl)-5,6,7,8-tetrahydroisoquinolin-8-yl)acetamide **59**

MS m/z (ESI): 352.2 [M+1].

### Example 60

### 1-Methyl-6-(5-(6-(1-methyl-1H-pyrazole-4-carbonyl)-2,6-diazaspiro[3.3]heptan-2-yl)pyridin-3-yl)-1,4-dihydro-2H-benzo[d][1,3]oxazin-2-one

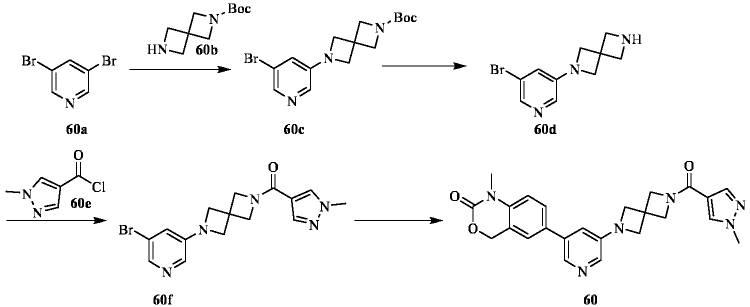

### Step 1

### Tert-butyl 6-(5-bromopyridin-3-yl)-2,6-diazaspiro[3.3]heptane-2-carboxylate

3,5-Dibromopyridine **60a** (3 g, 12.66 mmol) and tert-butyl 2,6-diazaspiro[3.3]heptane-2-carboxylate **60b** (2.51 g, 12.66 mmol) were dissolved in 1,4-dioxane (50 mL), and sodium tert-butoxide (2.43 g, 25.33 mmol), 4,5-bis(diphenylphosphino)-9,9-dimethylxanthene (733 mg, 1.27 mmol), and tris(dibenzylideneacetone)palladium (580 mg,633µmol) were separately added thereto. After displacement three times with nitrogen, the reaction system was placed in an oil bath at 100°C and stirred for 16 hours. After the reaction was complete, the reaction liquid was cooled to room temperature, diluted by adding water, extracted with ethyl acetate, washed with a saturated aqueous sodium chloride solution, dried over anhydrous sodium sulfate, filtered, and concentrated, and the crude product was separated and purified by column chromatography to obtain the title product tert-butyl 6-(5-bromopyridin-3-yl)-2,6-diazaspiro[3.3]heptane-2-carboxylate **60c** (2 g, a beige solid), yield: 44.6%

MS m/z (ESI): 354.1 [M+1].

### Step 2

### 2-(5-Bromopyridin-3-yl)-2,6-diazaspiro[3.3]heptane

Referring to step 2 of the synthesis route of Example 58, the title product 2-(5-bromopyridin-3-yl)-2,6-diazaspiro[3.3]heptane **60d** was obtained.

MS m/z (ESI): 254.1 [M+1].

### Step 3

### (6-(5-Bromopyridin-3-yl)-2,6-diazaspiro[3.3]heptan-2-yl)(1-methyl-1H-pyrazol-4-yl)methanone

Referring to step 3 of the synthesis route of Example 1, the title product 2-(5-bromopyridin-3-yl)-2,6-diazaspiro[3.3]heptane **60f** was obtained.

MS m/z (ESI): 362.1 [M+1].

### Step 4

1-Methyl-6-(5-(6-(1-methyl-1H-pyrazole-4-carbonyl)-2,6-diazaspiro[3.3]heptan-2-yl)pyridin-3-yl)-1,4-dihydro-2H-benzo[d][1,3]oxazin-2-one

Referring to step 4 of the synthesis route of Example 1, the title product 1-methyl-6-(5-(6-(1-methyl-1H-pyrazole-4-carbonyl)-2,6-diazaspiro[3.3]heptan-2-yl)pyridin-3-yl)-1,4-dihydro-2H-benzo[d][1,3]oxazin-2-one **60** was obtained.

MS m/z (ESI): 445.2 [M+1].

### Example 61

### 1-Methyl-N-(4-(4-methyl-3-carbonyl-3,4-dihydro-2H-benzo[b][1,4]oxazin-7-yl)-5,6,7,8-tetrahydroisoquinolin-8-yl)-1H-pyrazole-4-carboxamide

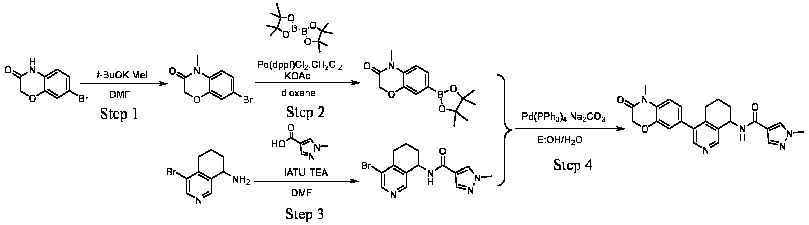

### Step 1

### 7-Bromo-4-methyl-2H-benzo[b][1,4]oxazin-3(4H)-one

7-Bromo-2H-benzo[b][1,4]oxazin-3(4H)-one (5 g, 21.93 mmol) was dissolved in N,N-dimethylformamide (50 mL), and in an ice-water bath, potassium tert-butoxide (4.92 g, 43.85 mmol) was slowly added thereto. After stirring for 0.5 hours, iodomethane (4.67 g, 32.89 mmol, 2.05 mL) was dropwise added thereto. The reaction system was further stirred at room temperature 20°C for 5.5 hours. After the reaction was complete, the reaction was quenched by slowly dropwise adding H₂O (20 mL) under ice-water bath condition, and the reaction liquid was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 3 : 1, uv = 254 nm) to finally obtain 7-bromo-4-methyl-2H-benzo[b][1,4]oxazin-3(4H)-one as a pale yellow solid (4.5 g, yield 84.7%).

MS m/z (ESI): 241.9 [M+1].

### Step 2

### 4-Methyl-7-(4,4,5,5-tetramethyl-1,3,2-dioxaborolan-2-yl)-2H-benzo[b][1,4]oxazin-3(4H)-one

7-Bromo-4-methyl-2H-benzo[b][1,4]oxazin-3(4H)-one (4.5 g, 18.59 mmol) was dissolved in dioxane (50 mL), and bis(pinacolato)diboron (5.66 g, 22.31 mmol), potassium acetate (1.95 g, 19.91 mmol), and [1,1'-bis(diphenylphosphino)ferrocene]palladium dichloride dichloromethane complex (1.35 g, 1.86 mmol) were separately added thereto. After displacement multiple times with nitrogen, the reaction system was placed in an oil bath at 100°C and stirred for 16 hours. After the reaction was complete, the reaction liquid was cooled to room temperature and filtered through diatomite, H₂O (20 mL) was slowly added, and the mixture was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 5 : 1, uv = 254 nm) to finally obtain 4-methyl-7-(4,4,5,5-tetramethyl-1,3,2-dioxaborolan-2-yl)-2H-benzo[b][1,4]oxazin-3(4H)-one as a white solid (5 g, 17.29 mmol, 93.02% yield).

MS m/z (ESI): 290.1 [M+1].

### Step 3

### N-(4-bromo-5,6,7,8-tetrahydroisoquinolin-8-yl)-1-methyl-1H-pyrazole-4-carboxamide

4-Bromo-5,6,7,8-tetrahydroisoquinolin-8-amine (500 mg, 2.20 mmol) and 1-methyl-1H-pyrazole-4-carboxylic acid (333 mg, 2.64 mmol) were dissolved in N,N-dimethylformamide (20 mL), and triethylamine (668 mg, 6.60 mmol) and 2-(7-azabenzotriazole)-N,N,N',N'-tetramethyluronium hexafluorophosphate (1.26 g, 3.30 mmol) were separately added thereto. The reaction liquid was stirred at 25°C for 4 hours. After the reaction was stopped, the reaction was quenched by slowly dropwise adding water (10 mL), and the reaction liquid was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 1 : 1, UV = 254 nm) to obtain the target product N-(4-bromo-5,6,7,8-tetrahydroisoquinolin-8-yl)-1-methyl-1H-pyrazole-4-carboxamide (400 mg, yield: 52.1%).

MS m/z (ESI): 335.0 [M+1].

### Step 4

### 1-Methyl-N-(4-(4-methyl-3-carbonyl-3,4-dihydro-2H-benzo[b][1,4]oxazin-7-yl)-5,6,7,8-tetrahydroisoquinolin-8-yl)-1H-pyrazole-4-carboxamide

N-(4-bromo-5,6,7,8-tetrahydroisoquinolin-8-yl)-1-methyl-1H-pyrazole-4-carboxamide (100 mg, 285.6 µmol) and 4-methyl-7-(4,4,5,5-tetramethyl-1,3,2-dioxaborolan-2-yl)-2H-benzo[b][1,4]oxazin-3(4H)-one (99.6 mg, 342.7 µmol) were dissolved in a mixed solvent of H₂O (2 mL) and EtOH (10 mL), and sodium carbonate (91 mg, 857.0 µmol) and tetrakis(triphenylphosphine)palladium (33.0 mg, 28.6 µmol) were sequentially added thereto. After displacement multiple times with nitrogen, the reaction system was placed in an oil bath at 100°C and stirred for 16 hours. After the reaction was complete, the reaction liquid was cooled to room temperature, diluted by adding water (5 mL), extracted with ethyl acetate (10 mL × 2), washed with a saturated aqueous sodium chloride solution (10 mL × 2), dried over anhydrous sodium sulfate, filtered, and concentrated to obtain a crude product. The crude product was purified by a column (DCM/MeOH = 10 : 1, UV = 254 nm) to finally obtain 1-methyl-N-(4-(4-methyl-3-carbonyl-3,4-dihydro-2H-benzo[b][1,4]oxazin-7-yl)-5,6,7,8-tetrahydroisoquinolin-8-yl)-1H-parazole-4-carboxamide (50 mg, 41.7% yield).

MS m/z (ESI): 418.1 [M+1].

### Example 62

### 7-(5-((1-(Ethylsulfonyl)azetidin-3-yl)oxy)pyridin-3-yl)-4-methyl-2H-benzo[b][1,4]oxazin-3(4H)-one

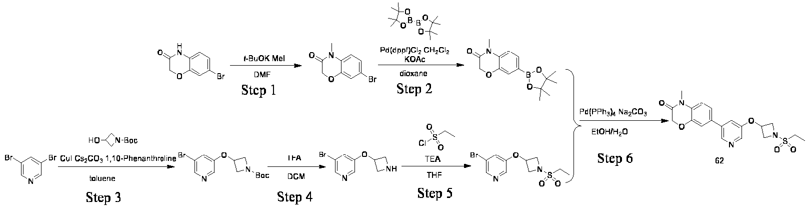

### Step 1

### 7-Bromo-4-methyl-2H-benzo[b][1,4]oxazin-3(4H)-one

7-Bromo-2H-benzo[b][1,4]oxazin-3(4H)-one (5 g, 21.93 mmol) was dissolved in N,N-dimethylformamide (50 mL), and in an ice-water bath, potassium tert-butoxide (4.92 g, 43.85 mmol) was slowly added thereto. After stirring for 0.5 hours, iodomethane (4.67 g, 32.89 mmol, 2.05 mL) was dropwise added thereto. The reaction system was further stirred at room temperature 20°C for 5.5 hours. After the reaction was complete, the reaction was quenched by slowly dropwise adding H₂O (20 mL) under ice-water bath condition, and the reaction liquid was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 3 : 1, uv = 254 nm) to finally obtain 7-bromo-4-methyl-2H-benzo[b][1,4]oxazin-3(4H)-one as a pale yellow solid (4.5 g, yield 84.7%).

MS m/z (ESI): 241.9 [M+1].

### Step 2

### 4-Methyl-7-(4,4,5,5-tetramethyl-1,3,2-dioxaborolan-2-yl)-2H-benzo[b][1,4]oxazin-3(4H)-one

7-Bromo-4-methyl-2H-benzo[b][1,4]oxazin-3(4H)-one (4.5 g, 18.59 mmol) was dissolved in dioxane (50 mL), and bis(pinacolato)diboron (5.66 g, 22.31 mmol), potassium acetate (1.95 g, 19.91 mmol), and [1,1'-bis(diphenylphosphino)ferrocene]palladium dichloride dichloromethane complex (1.35 g, 1.86 mmol) were separately added thereto. After displacement multiple times with nitrogen, the reaction system was placed in an oil bath at 100°C and stirred for 16 hours. After the reaction was complete, the reaction liquid was cooled to room temperature and filtered through diatomite, H₂O (20 mL) was slowly added, and the mixture was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 5 : 1, uv = 254 nm) to finally obtain 4-methyl-7-(4,4,5,5-tetramethyl-1,3,2-dioxaborolan-2-yl)-2H-benzo[b][1,4]oxazin-3(4H)-one as a white solid (5 g, 17.29 mmol, 93.02% yield).

MS m/z (ESI): 290.1 [M+1].

### Step 3

### Tert-butyl 3-((5-bromopyridin-3-yl)oxy)azetidine-1-carboxylate

3,5-Dibromopyridine (3 g, 12.8 mmol) and tert-butyl 3-hydroxyazetidine-1-carboxylate (2.66 g, 15.36 mmol) were dissolved in toluene (30 mL), and cuprous iodide (244 mg, 1.28 mmol), cesium carbonate (12.6 g, 38.4 mmol), and 1,10-phenanthroline (460 mg, 2.56 mmol) were separately added thereto. After displacement multiple times with nitrogen, the reaction system was placed in an oil bath at 100°C and stirred for 16 hours. After the reaction was complete, the reaction liquid was cooled to room temperature. The reaction was quenched by adding water (20 mL), and the reaction liquid was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 5 : 1, uv = 254 nm) to finally obtain the target product tert-butyl 3-((5-bromopyridin-3-yl)oxy)azetidine-1-carboxylate (3.0 g, yield: 71.4%).

MS m/z (ESI): 329.0 [M+1].

### Step 4

### 3-(Azetidin-3-yloxy)-5-bromopyridine

Tert-butyl 3-((5-bromopyridin-3-yl)oxy)azetidine-1-carboxylate (500 mg, 1.52 mmol) was dissolved in dichloromethane (10 mL), and trifluoroacetic acid (1 mL) was added thereto. The reaction system was stirred for 4 hours at room temperature. After the raw material was completely reacted, the excess solvent was directly removed by concentration to obtain crude 3-(azetidin-3-yloxy)-5-bromopyridine (350 mg, crude). The crude product was directly dissolved for the next reaction.

MS m/z (ESI): 228.9 [M+1].

### Step 5

### 3-Bromo-5-((1-(ethylsulfonyl)azetidin-3-yl)oxy)pyridine

3-(Azetidin-3-yloxy)-5-bromopyridine (350 mg, crude) was dissolved in tetrahydrofuran (10 mL), and triethylamine (462 mg, 4.56 mmol) and ethylsulfonyl chloride (292 mg, 2.28 mmol) were separately added thereto. The reaction system was stirred for 4 hours at room temperature. After the reaction was complete, the reaction was quenched by adding water (5 mL), extracted with ethyl acetate (10 mL × 2), washed with a saturated aqueous sodium chloride solution (10 mL × 2), dried over anhydrous sodium sulfate, filtered, and concentrated to obtain a crude product. The crude product was purified by a column (PE/EtOAc = 3 : 1, uv = 254 nm) to finally obtain the target product 3-bromo-5-((1-(ethylsulfonyl)azetidin-3-yl)oxy)pyridine (300 mg, yield: 61.7%).

MS m/z (ESI): 320.9 [M+1].

### Step 6

### 7-(5-((1-(Ethylsulfonyl)azetidin-3-yl)oxy)pyridin-3-yl)-4-methyl-2H-benzo[b][1,4]oxazin-3(4H)-one

3-Bromo-5-((1-(ethylsulfonyl)azetidin-3-yl)oxy)pyridine (100 mg, 311.6 µmol) and 4-methyl-7-(4,4,5,5-tetramethyl-1,3,2-dioxaborolan-2-yl)-2H-benzo[b][1,4]oxazin-3(4H)-one (109.1 mg, 374.0 µmol) were dissolved in a mixed solvent of H₂O (2 mL) and EtOH (10 mL), and sodium carbonate (99 mg, 935.1 µmol) and tetrakis(triphenylphosphine)palladium (36.0 mg, 31.2 µmol) were sequentially added thereto. After displacement multiple times with nitrogen, the reaction system was placed in an oil bath at 100°C and stirred for 16 hours. After the reaction was complete, the reaction liquid was cooled to room temperature, diluted by adding water (5 mL), extracted with ethyl acetate (10 mL × 2), washed with a saturated aqueous sodium chloride solution (10 mL × 2), dried over anhydrous sodium sulfate, filtered, and concentrated to obtain a crude product. The crude product was purified by a column (DCM/MeOH = 10 : 1, UV=254 nm) to finally obtain 7-(5-((1-(ethylsulfonyl)azetidin-3-yl)oxy)pyridin-3-yl)-4-methyl-2H-benzo[b][1,4]oxazin-3(4H)-one (55 mg, 43.7% yield).

MS m/z (ESI): 404.1 [M+1].

### Example 63

### N-(4-(4-methyl-3-carbonyl-3,4-dihydro-2H-benzo[b][1,4]oxazin-7-yl)-5,6,7,8-tetrahydroisoquinolin-8-yl)acetamide

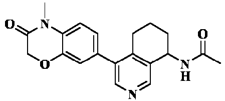

Referring to Example 10, with 7-bromo-2H-benzo[b][1,4]oxazin-3(4H)-one and 4-bromo-5,6,7,8-tetrahydroisoquinolin-8-amine as starting raw materials, the target product N-(4-(4-methyl-3-carbonyl-3,4-dihydro-2H-benzo[b][1,4]oxazin-7-yl)-5,6,7,8-tetrahydroisoquinolin-8-yl)acetamide was finally obtained.

MS m/z (ESI): 352.1 [M+1].

### Example 64

### 4-Methyl-7-(5-(6-(1-methyl-1H-pyrazole-4-carbonyl)-2,6-diazaspiro[3.3]heptan-2-yl)pyridin-3-yl)-2H-benzo[b][1,4]oxazin-3(4H)-one

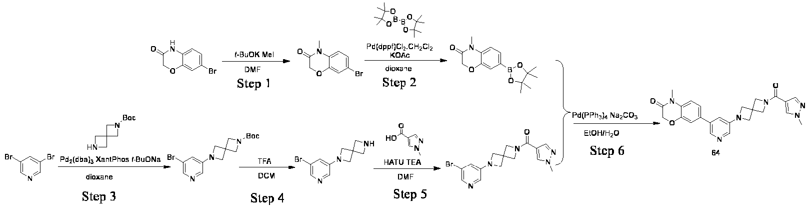

### Step 1

### 7-Bromo-4-methyl-2H-benzo[b][1,4]oxazin-3(4H)-one

7-Bromo-2H-benzo[b][1,4]oxazin-3(4H)-one (5 g, 21.93 mmol) was dissolved in N,N-dimethylformamide (50 mL), and in an ice-water bath, potassium tert-butoxide (4.92 g, 43.85 mmol) was slowly added thereto. After stirring for 0.5 hours, iodomethane (4.67 g, 32.89 mmol, 2.05 mL) was dropwise added thereto. The reaction system was further stirred at room temperature 20°C for 5.5 hours. After the reaction was complete, the reaction was quenched by slowly dropwise adding H₂O (20 mL) under ice-water bath condition, and the reaction liquid was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 3 : 1, uv = 254 nm) to finally obtain 7-bromo-4-methyl-2H-benzo[b][1,4]oxazin-3(4H)-one as a pale yellow solid (4.5 g, yield 84.7%).

MS m/z (ESI): 241.9 [M+1].

### Step 2

### 4-Methyl-7-(4,4,5,5-tetramethyl-1,3,2-dioxaborolan-2-yl)-2H-benzo[b][1,4]oxazin-3(4H)-one

7-Bromo-4-methyl-2H-benzo[b][1,4]oxazin-3(4H)-one (4.5 g, 18.59 mmol) was dissolved in dioxane (50 mL), and bis(pinacolato)diboron (5.66 g, 22.31 mmol), potassium acetate (1.95 g, 19.91 mmol), and [1,1'-bis(diphenylphosphino)ferrocene]palladium dichloride dichloromethane complex (1.35 g, 1.86 mmol) were separately added thereto. After displacement multiple times with nitrogen, the reaction system was placed in an oil bath at 100°C and stirred for 16 hours. After the reaction was complete, the reaction liquid was cooled to room temperature and filtered through diatomite, H₂O (20 mL) was slowly added, and the mixture was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 5 : 1, uv = 254 nm) to finally obtain 4-methyl-7-(4,4,5,5-tetramethyl-1,3,2-dioxaborolan-2-yl)-2H-benzo[b][1,4]oxazin-3(4H)-one as a white solid (5 g, 17.29 mmol, 93.02% yield).

MS m/z (ESI): 290.1 [M+1].

### Step 3

### Tert-butyl 6-(5-bromopyridin-3-yl)-2,6-diazaspiro[3.3]heptane-2-carboxylate

3,5-Dibromopyridine (3 g, 12.66 mmol) and tert-butyl 2,6-diazaspiro[3.3]heptane-2-carboxylate (2.51 g, 12.66 mmol) were dissolved in dioxane (40 mL), and sodium tert-butoxide (2.43 g, 25.33 mmol), 4,5-bis(diphenylphosphino)-9,9-dimethylxanthene (732.76 mg, 1.27 mmol), and tris(dibenzylideneacetone)dipalladium (579.83 mg, 633.20 µmol) were separately added thereto. After displacement multiple times with nitrogen, the reaction system was placed in an oil bath at 100°C and stirred for 16 hours. After the reaction was complete, the reaction liquid was cooled to room temperature, and the reaction was quenched by slowly dropwise adding water (10 mL). The reaction liquid was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 5 : 1, uv = 254 nm) to finally obtain tert-butyl 6-(5-bromopyridin-3-yl)-2,6-diazaspiro[3.3]heptane-2-carboxylate as a beige solid (2 g, 5.65 mmol, 44.58% yield).

MS m/z (ESI): 354.0 [M+1].

### Step 4

### 2-(5-Bromopyridin-3-yl)-2,6-diazaspiro[3.3]heptane

Tert-butyl 6-(5-bromopyridin-3-yl)-2,6-diazaspiro[3.3]heptane-2-carboxylate (500 mg, 1.41 mmol) was dissolved in dichloromethane (10 mL), and trifluoroacetic acid (1 mL) was added thereto. The reaction system was stirred for 4 hours at room temperature. After the raw material was completely reacted, the excess solvent was directly removed by concentration to obtain crude 2-(5-bromopyridin-3-yl)-2,6-diazaspiro[3.3]heptane (360 mg, crude). The crude product was directly dissolved for the next reaction.

MS m/z (ESI): 254.0 [M+1].

### Step 5

### (6-(5-Bromopyridin-3-yl)-2,6-diazaspiro[3.3]heptan-2-yl)(1-methyl-1H-pyrazol-4-yl)methanone

2-(5-Bromopyridin-3-yl)-2,6-diazaspiro[3.3]heptane (360 mg, crude) was dissolved in tetrahydrofuran (10 mL), and triethylamine (430 mg, 4.23 mmol) and 1-methyl-1H-pyrazole-4-carboxylic acid (270 mg, 2.12 mmol) were separately added thereto. The reaction system was stirred for 4 hours at room temperature. After the reaction was complete, the reaction was quenched by adding water (5 mL), extracted with ethyl acetate (10 mL × 2), washed with a saturated aqueous sodium chloride solution (10 mL × 2), dried over anhydrous sodium sulfate, filtered, and concentrated to obtain a crude product. The crude product was purified by passing through a column (PE/EtOAc = 3 : 1, uv = 254 nm) to finally obtain the target product (6-(5-bromopyridin-3-yl)-2,6-diazaspiro[3.3]heptan-2-yl)(1-methyl-1H-pyrazol-4-yl)methanone (350 mg, yield: 68.7%).

MS m/z (ESI): 362.1 [M+1].

### Step 6

### 4-Methyl-7-(5-(6-(1-methyl-1H-pyrazole-4-carbonyl)-2,6-diazaspiro[3.3]heptan-2-yl)pyridin-3-yl)-2H-benzo[b][1,4]oxazin-3(4H)-one

(6-(5-Bromopyridin-3-yl)-2,6-diazaspiro[3.3]heptan-2-yl)(1-methyl-1H-pyrazol-4-yl)methanone (100 mg, 276.2 µmol) and 4-methyl-7-(4,4,5,5-tetramethyl-1,3,2-dioxaborolan-2-yl)-2H-benzo[b][1,4]oxazin-3(4H)-one (96.5 mg, 331.4 µmol) were dissolved in a mixed solvent of H₂O (2 mL) and EtOH (10 mL), and sodium carbonate (88.0 mg, 828.6 µmol) and tetrakis(triphenylphosphine)palladium (32.0 mg, 27.6 µmol) were sequentially added thereto. After displacement multiple times with nitrogen, the reaction system was placed in an oil bath at 100°C and stirred for 16 hours. After the reaction was complete, the reaction liquid was cooled to room temperature, diluted by adding water (5 mL), extracted with ethyl acetate (10 mL × 2), washed with a saturated aqueous sodium chloride solution (10 mL × 2), dried over anhydrous sodium sulfate, filtered, and concentrated to obtain a crude product. The crude product was purified by a column (DCM/MeOH = 10 : 1, UV = 254 nm) to finally obtain 4-methyl-7-(5-(6-(1-methyl-1H-pyrazole-4-carbonyl)-2,6-diazaspiro[3.3]heptan-2-yl)pyridin-3-yl)-2H-benzo[b][1,4]oxazin-3(4H)-one as a pale yellow solid (45 mg, 36.7% yield).

MS m/z (ESI): 445.1 [M+1].

MS m/z (ESI): 444.2 [M+1].

### Example 65

### 5-Methyl-N-(4-(1-methyl-2-oxo-1,4-dihydro-2H-benzo[d][1,3]oxazin-6-yl)-5,6,7,8-tetrahydroisoquinolin-8-yl)isoxazole-4-carboxamide

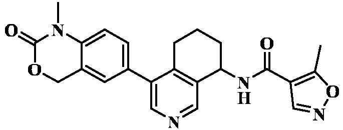

Referring to Example 57, the product 5-methyl-N-(4-(1-methyl-2-oxo-1,4-dihydro-2H-benzo[d][1,3]oxazin-6-yl)-5,6,7,8-tetrahydroisoquinolin-8-yl)isoxazole-4-carboxamide was obtained.

MS m/z (ESI): 419.2 [M+1].

### Example 66

### N-(4-(7-fluoro-1-methyl-2-carbonyl-1,4-dihydro-2H-benzo[d][1,3]oxazin-6-yl)-5,6,7,8-tetrahydroisoquinolin-8-yl)propanamide

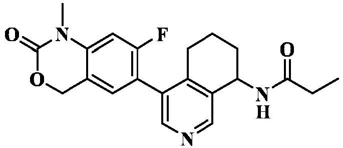

Referring to Example 1, the product N-(4-(7-fluoro-1-methyl-2-carbonyl-1,4-dihydro-2H-benzo[d][1,3]oxazin-6-yl)-5,6,7,8-tetrahydroisoquinolin-8-yl)propanamide was obtained.

MS m/z (ESI): 384.1 [M+1].

### Example 67

### N-((4bR,5aS)-4-(1-methyl-2-carbonyl-1,4-dihydro-2H-benzo[d][1,3]oxazin-6-yl)-4b,5,5a,6-tetrahydrocyclopropa[3,4]cyclopenta[1,2-c]pyridin-6-yl)propanamide

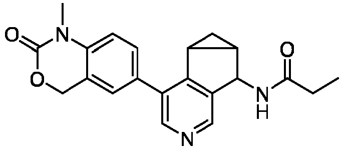

Referring to step 7 of Example 48, with 1-methyl-6-(4,4,5,5-tetramethyl-1,3,2-dioxaborolan-2-yl)-1,4-dihydro-2H-benzo[d][1,3]oxazin-2-one and N-(4-bromo-4b,5,5a,6-tetrahydrocyclopropa[3,4]cyclopenta[1,2-c]pyridin-6-yl)propanamide as raw materials, N-((4bR,5aS)-4-(1-methyl-2-carbonyl-1,4-dihydro-2H-benzo[d][1,3]oxazin-6-yl)-4b,5,5a,6-tetrahydrocyclopropa[3,4]cyclopenta[1,2-c]pyridin-6-yl)propanamide was synthesized.

MS m/z (ESI): 364.2 [M+1].

| Column type | Mobile phase | Flow rate | Detection wavelength | Temper ature | Retention time of P1 | Retention time of P2 | Linear gradient |
|---|---|---|---|---|---|---|---|
| CHIRALCE L OJ-H, 4.6 × 150 mm, 5 µm | Hexane: EtOH: MeOH = 70 : 15 : 15 | 1.0 ml/min | 214 nm | 35°C | 5.6 min | 8.2 min | Isometric |

The sample was subjected to chiral resolution to obtain P1 and P2. The chiral resolution conditions were as follows.

| Column type | Mobile phase | Flow rate | Detection wavelength | Temper ature | Retention time of P1A | Retention time of P1B | Retention time of P1C |
|---|---|---|---|---|---|---|---|
| CHIRALCE L AS-H, 4.6 × 150 mm, 5 µm | Hexane: EtOH: MeOH = 70: 15 : 15 | 1.0 ml/min | 214 nm | 35°C | 4.7 min | 5.4 min | Isometric |

### Example 68

### 6-(1'-(Cyclopropylsulfonyl)-1',2',3',6'-tetrahydro-[3,4'-bipyridine]-5-yl)-1-methyl-1,4-dihydro-2H-benzo[d][1,3]oxazin-2-one

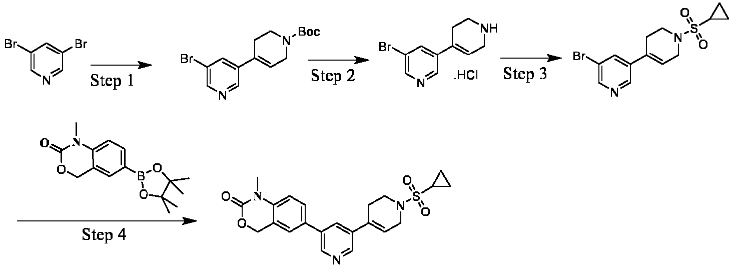

### Step 1

### Tert-butyl 5-bromo-3',6'-dihydro-[3,4'-bipyridine]-1'(2'H)-carboxylate

3,5-Dibromopyridine (0.38 g, 1.60 mmol), tert-butyl 4-(4,4,5,5-tetramethyl-1,3,2-dioxaborolan-2-yl)-3,6-dihydropyridin-1(2H)-carboxylate (496.00 mg, 1.60 mmol), and sodium carbonate (510.05 mg, 4.81 mmol) were dissolved in 1'4-dioxane (12 mL) and water (3 mL), and under nitrogen protection, bis(triphenyl)palladium dichloride (56.30 mg, 80.21 µmol) was added. The mixture was reacted with stirring for 5 hours at 90°C. The reaction was quenched by adding a saturated aqueous sodium chloride solution (50 mL) to the mixture, and the reaction liquid was extracted with ethyl acetate (30 mL × 3). The organic phases were combined, sequentially washed with a saturated aqueous sodium chloride solution (50 mL × 2), dried over anhydrous sodium sulfate, and concentrated under reduced pressure. The residue was purified by rapid silica gel chromatography (eluted with petroleum ether : ethyl acetate = 100 : 0 to 70 : 30) to obtain the target product tert-butyl 5-bromo-3',6'-dihydro-[3,4'-bipyridine]-1'(2'H)-carboxylate (0.32 g, a white solid), yield: 58.81%.

MS m/z (ESI): 339.1, 341.1 [M+1].

### Step 2

### 5-Bromo-1',2',3',6'-tetrahydro-3,4'-bipyridine hydrochloride

Tert-butyl 5-bromo-3',6'-dihydro-[3,4'-bipyridine]-1'(2'H)-carboxylate (0.2 g, 589.58 µmol) was dissolved in hydrogen chloride dioxane (4 M, 10 mL), and the mixture was reacted with stirring for 2 hours at 20°C. The reaction liquid was concentrated under reduced pressure to obtain crude 5-bromo-1',2',3',6'-tetrahydro-3,4'-bipyridine hydrochloride (0.16 g). The crude product was directly used for the next step.

MS m/z (ESI): 239.0, 241.0 [M+1].

### Step 3

### 5-Bromo-1'-(cyclopropylsulfonyl)-1',2',3',6'-tetrahydro-3,4'-bipyridine

5-Bromo-1',2',3',6'-tetrahydro-3,4'-bipyridine hydrochloride (0.16 g, 580.61 µmol, CL) and triethylamine (293.76 mg, 2.90 mmol, 404.90 µL) were dissolved in dichloromethane (10 mL), and under nitrogen protection, cyclopropylsulfonyl chloride (106.12 mg, 754.79 µmol) was added. The mixture was reacted with stirring for 1 hours at 20°C. The reaction liquid was washed with a saturated aqueous sodium chloride solution (30 mL × 2). The organic phase was separated, dried over anhydrous sodium sulfate, and concentrated under reduced pressure to obtain crude 5-bromo-1'-(cyclopropylsulfonyl)-1',2',3',6'-tetrahydro-3,4'-bipyridine (0.19 g). The crude product was directly used for the next step.

MS m/z (ESI): 343.0, 345.0 [M+1].

### Step 4

### 6-(1'-(Cyclopropylsulfonyl)-1',2',3',6'-tetrahydro-[3,4'-bipyridine]-5-yl)-1-methyl-1,4-dihydro-2H-benzo[d][1,3]oxazin-2-one

Referring to step 4 of Example 1, with 1-methyl-6-(4,4,5,5-tetramethyl-1,3,2-dioxaborolan-2-yl)-1,4-dihydro-2H-benzo[d][1,3]oxazin-2-one and 5-bromo-1'-(cyclopropylsulfonyl)-1',2',3',6'-tetrahydro-3,4'-bipyridine as raw materials, 6-(1'-(cyclopropylsulfonyl)-1',2',3',6'-tetrahydro-[3,4'-bipyridine]-5-yl)-1-methyl-1,4-dihydro-2H-benzo[d][1,3]oxazin-2-one was synthesized.

MS m/z (ESI): 426.1 [M+1].

¹H NMR (400 MHz, CDCl3) δ 8.73 (d, J = 2.1 Hz, 1H), 8.64 (d, J = 2.1 Hz, 1H), 7.87 (s, 1H), 7.64 - 7.56 (m, 1H), 7.42 - 7.31 (m, 1H), 7.11 - 7.03 (m, 1H), 6.32 - 6.19 (m, 1H), 5.29 (s, 2H), 4.17 - 4.02 (m, 2H), 3.70 - 3.55 (m, 2H), 3.44 (s, 3H), 2.79 - 2.63 (m, 2H), 2.42 - 2.26 (m, 1H), 1.31 - 1.17 (m, 2H), 1.10 - 0.97 (m, 2H).

### Example 69

### 6-(5-(1-(Cyclopropylsulfonyl)piperidin-4-yl)pyridin-3-yl)-1-methyl-1,4-dihydro-2H-benzo[d][1,3]oxazin-2-one

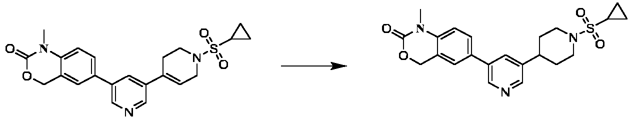

6-(1'-(Cyclopropylsulfonyl)-1',2',3',6'-tetrahydro-[3,4'-bipyridine]-5-yl)-1-methyl-1,4-dihydro-2H-benzo[d][1,3]oxazin-2-one (16 mg, 37.6 µmol) was dissolved in ethanol (5 mL), and under nitrogen protection, palladium on carbon (10% w/w, with 50% of water) (4.0 mg) was added. The mixture was reacted with stirring at 20°C for 12 hours in a hydrogen atmosphere (1 atm). The catalyst was removed by filtration, and the liquid phase was evaporated to dryness to obtain a crude product. The crude product was separated by Prep-HPLC to obtain the target product 6-(5-(1-(cyclopropylsulfonyl)piperidin-4-yl)pyridin-3-yl)-2-methoxyoxazin-2-one (1 mg).

MS m/z (ESI): 428.1 [M+1].

### Example 70

### 6-(1'-(Cyclopropylcarbonyl)-1',2',3',6'-tetrahydro-[3,4'-bipyridine]-5-yl)-1-methyl-1,4-dihydro-2H-benzo[d][1,3]oxazin-2-one

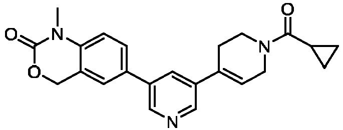

Referring to Example 68, the product 6-(1'-(cyclopropylcarbonyl)-1',2',3',6'-tetrahydro-[3,4'-bipyridine]-5-yl)-1-methyl-1,4-dihydro-2H-benzo[d][1,3]oxazin-2-one was obtained.

MS m/z (ESI): 390.1 [M+1].

### Example 71

### 7-(1'-(Cyclopropylsulfonyl)-1',2',3',6'-tetrahydro-[3,4'-bipyridine]-5-yl)-4-methyl-2H-benzo[b][1,4]oxazin-3(4H)-one

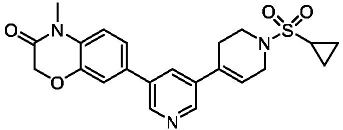

Referring to Example 68, the product 7-(1'-(cyclopropylsulfonyl)-1',2',3',6'-tetrahydro-[3,4'-bipyridine]-5-yl)-4-methyl-2H-benzo[b][1,4]oxazin-3(4H)-one was obtained.

MS m/z (ESI): 426.1 [M+1].

### Example 72

### (R)-2,2,2-trifluoro-N-(4-(1-methyl-2-oxo-1,4-dihydro-2H-benzo[d][1,3]oxazin-6-yl)-5,6,7,8-tetrahydroisoquinolin-8-yl)acetamide

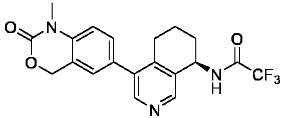

Referring to the synthesis route of Example 1, the title product (R)-2,2,2-trifluoro-N-(4-(1-methyl-2-oxo-1,4-dihydro-2H-benzo[d][1,3]oxazin-6-yl)-5,6,7,8-tetrahydroisoquinolin-8-yl)acetamide 130 was obtained.

MS m/z (ESI): 406.1 [M+1].

### Example 73

### (R)-N-(4-(1-(methyl-d3)-2-carbonyl-1,4-dihydro-2H-benzo[d][1,3]oxazin-6-yl)-5,6,7,8-tetrahydroisoquinolin-8-yl)propanamide

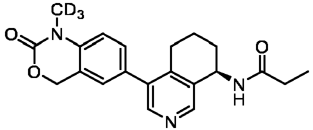

Referring to Example 1, with deuterated iodomethane as a raw material, the product (R)-N-(4-(1-(methyl-d3)-2-carbonyl-1,4-dihydro-2H-benzo[d][1,3]oxazin-6-yl)-5,6,7,8-tetrahydroisoquinolin-8-yl)propanamide was obtained.

MS m/z (ESI): 369.2 [M+1].

### Example 74

### ((R)-N-(4-(1-methyl-2-carbonyl-1,4-dihydro-2H-benzo[d][1,3]oxazin-6-yl)-5,6,7,8-tetrahydroisoquinolin-8-yl)propanamide-d5

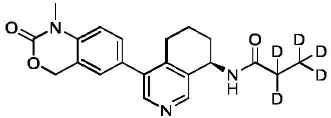

Referring to Example 1, with deuterated propionic acid as a raw material, the product ((R)-N-(4-(1-methyl-2-carbonyl-1,4-dihydro-2H-benzo[d][1,3]oxazin-6-yl)-5,6,7,8-tetrahydroisoquinolin-8-yl)propanamide-d5 was obtained.

MS m/z (ESI): 371.2 [M+1].

### Example 75

### (R)-N-(8-(1-methyl-2-oxo-1,4-dihydro-2H-benzo[d][1,3]oxazin-6-yl)-3,4-dihydro-2H-pyrano[3,2-c]pyridin-4-yl)propanamide

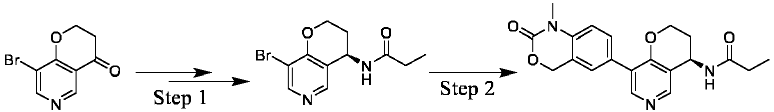

### Step 1

### (R)-N-(8-bromo-3,4-dihydro-2H-pyrano[3,2-c]pyridin-4-yl)propanamide

Referring to steps 4 and 5 of intermediate Im-1 and step 3 of Example 1, with 8-bromo-2,3-dihydro-4H-pyrano[3,2-c]pyridin-4-one as a raw material, the title product (R)-N-(8-bromo-3,4-dihydro-2H-pyrano[3,2-c]pyridin-4-yl)propanamide was synthesized.

MS m/z (ESI): 285.0, 287.0 [M+1].

### Step 2

### (R)-N-(8-(1-methyl-2-oxo-1,4-dihydro-2H-benzo[d][1,3]oxazin-6-yl)-3,4-dihydro-2H-pyrano[3,2-c]pyridin-4-yl)propanamide

Referring to step 4 of Example 1, with (R)-N-(8-bromo-3,4-dihydro-2H-pyrano[3,2-c]pyridin-4-yl)propanamide as a raw material, the target product (R)-N-(8-(1-methyl-2-oxo-1,4-dihydro-2H-benzo[d][1,3]oxazin-6-yl)-3,4-dihydro-2H-pyrano[3,2-c]pyridin-4-yl)propanamide was obtained.

MS m/z (ESI): 368.1 [M+1].

### Example 76

### (S)-N-(7-(1-methyl-2-oxo-1,4-dihydro-2H-benzo[d][1,3]oxazin-6-yl)-2,3-dihydrofuro[3,2-c]pyridin-3-yl)propanamide

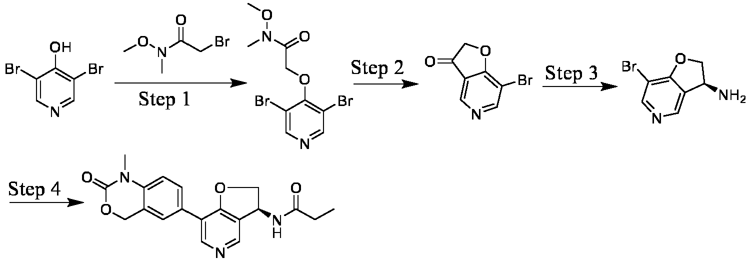

### Step 1

### 2-[(3,5-Dibromo-4-pyridinyl)oxy]-N-methoxy-N-methyl-acetamide

3,5-Dibromopyridin-4-ol (0.5 g, 1.98 mmol) and 2-bromo-N-methoxy-N-methylacetamide (359.87 mg, 1.98 mmol) were dissolved in N,N-dimethylformamide (5 mL), and under nitrogen protection, potassium carbonate (819.75 mg, 5.93 mmol) was added. The mixture was reacted with stirring for 12 hours at 25°C. The reaction was quenched by adding a saturated aqueous sodium chloride solution (50 mL) to a mixture, and the reaction liquid was extracted with ethyl acetate (30 mL × 2). The organic phases were combined, sequentially washed with a saturated aqueous sodium chloride solution (30 mL × 5), dried over anhydrous sodium sulfate, and concentrated under reduced pressure. The residue was purified by rapid silica gel chromatography (eluted with petroleum ether : ethyl acetate = 100 : 0 to 60 : 40) to obtain the target product (0.58 g, yield 83%).

MS m/z (ESI): 352.9 [M+1].

### Step 2

### 7-Bromofuro[3,2-c]pyridin-3-one

2-[(3,5-Dibromo-4-pyridinyl)oxy]-N-methoxy-N-methyl-acetamide (0.58 g, 1.64 mmol) was dissolved in THF (10 mL), and under nitrogen protection and cooling with a dry ice ethanol bath, n-butyllithium (1 M, 1.64 mL) was added. The mixture was reacted with stirring for 1 hours at -78°C. The reaction liquid was slowly warmed to 0°C, and the reaction was quenched by adding a saturated aqueous ammonium chloride solution (1 mL). A saturated aqueous sodium chloride solution (50 mL) was then added to the mixture, and the mixture was extracted with ethyl acetate (30 mL × 2). The organic phases were combined, sequentially washed with a saturated aqueous sodium chloride solution (30mL × 2), dried over anhydrous sodium sulfate, and concentrated under reduced pressure. The residue was purified by rapid silica gel chromatography (eluted with petroleum ether : ethyl acetate = 100 : 0 to 70 : 30) to obtain the target product 7-bromofuro[3,2-c]pyridin-3-one (0.15 g, yield 42.78%).

MS m/z (ESI): 213.9, 215.9 [M+1].

### Step 3

### (S)-7-Bromo-2,3-dihydrofuro[3,2-c]pyridin-3-amine

Referring to steps 4 and 5 of intermediate Im-1, the product (S)-7-bromo-2,3-dihydrofuro[3,2-c]pyridin-3-amine was obtained.

MS m/z (ESI): 215.0, 217.0 [M+1].

### Step 4

### (S)-N-(7-(1-methyl-2-oxo-1,4-dihydro-2H-benzo[d][1,3]oxazin-6-yl)-2,3-dihydrofuro[3,2-c]pyridin-3-yl)propanamide

Referring to the synthesis of Example 1, the product (S)-N-(7-(1-methyl-2-oxo-1,4-dihydro-2H-benzo[d][1,3]oxazin-6-yl)-2,3-dihydrofuro[3,2-c]pyridin-3-yl)propanamide was obtained.

MS m/z (ESI): 354.1 [M+1].

### Example 77

### 1-Methyl-N-(1-(6-(1-methyl-2-carbonyl-1,4-dihydro-2H-benzo[d][1,3]oxazin-6-yl)pyrazin-2-yl)piperidin-4-yl)-1H-pyrazole-4-carboxamide

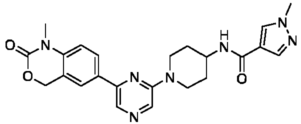

Referring to Example 60, tert-butyl 3,5-dibromopyrazine and piperidin-4-ylcarbamate as a raw material, the title product 1-methyl-N-(1-(6-(1-methyl-2-carbonyl-1,4-dihydro-2H-benzo[d][1,3]oxazin-6-yl)pyrazin-2-yl)piperidin-4-yl)-1H-pyrazole-4-carboxamide was obtained.

MS m/z (ESI): 448.2 [M+1].

### Biological assay and evaluation

The present invention will be further described and explained below in conjunction with test examples, and these examples are not meant to limit the scope of the present invention.

### Test Example 1

G-402 cell line was used as host cells to express (by transient or stable transfection) an enzyme of the human CYP 11 family. Specifically, G-402 cell lines stably expressing human CYP11B1 and human CYP11B2 were established respectively. It has been confirmed that the G-402 cell line can express cofactors (adrenodoxin and adrenodoxin reductase) quite essential for the activity of the CYP11 family, and compared to H295R cells, this cell line itself does not exhibit any enzymatic activity associated with the CYP11 family. Therefore, the G-402 cell line is very suitable as a host cell for the ectopic expression of an enzyme of the CYP11 family.

The G-402 cell line, originally derived from renal myoblastoma, can be obtained from ATCC (CRL-1440). The main elements of an expression plasmid include ORF of human CYP11B1 or human CYP11B2, a suitable promoter (CMV promoter), and a suitable resistance marker (neomycin). The expression plasmid was transfected into G-402 cells by standard technology, and a specific antibiotic was then given for screening. The activity of the enzyme expressed by the screened monoclonal cells was assessed by 11-deoxycorticosterone (substrate of CYP11B2) and 11-deoxycortisol (substrate of CYP11B1).

The G-402 cells expressing CYP11 plasmid, as established in the above solution, were cultured in McCoy's 5a Modified Medium (ATCC Catalog No. 30-2007) containing 10% FCS and 400 µg/ml G418 in an incubator at 37°C and 5% CO₂. A cell enzyme experiment was carried out using DMEM/F12 medium containing 2.5% charcoal-treated FBS and an appropriate concentration of a substrate (1 µM 11-deoxycorticosterone or 1 µM 11-deoxycortisol). In order to detect the activity of the enzyme in the cells, the cells were plated in a 96-well plate and incubated for 16 h. The supernatant was then transfered and analyzed for the concentration of the expected product (the product of CYP11B2: aldosterone; the product of CYP11B1: cortisol). The concentrations of these products were determined by an HTRF experiment of CisBio.

In the cell enzyme experiment, the inhibitory effect of the test compound on the generated product can reflect the inhibitory effect thereof on the enzyme. The dose-dependent inhibition of the enzyme activity by the compound was calculated by plotting the concentration of the test compound (x axis) versus the measured product level (y axis). Then, the original data was fitted to a 4-parameter sigmoidal function (Morgan-Mercer-Flodin, MMF model) by least square method: y = (AB + Cx^{D}) / (B + x^{D})
in which A is the maximum y value, B is the EC50 determined by XLFit, C is the minimum y value, and D is the slope value. The maximum value A corresponds to the amount of the product detected without an inhibitor, and C corresponds to the amount of the product detected in the G402 cells expressing an empty plasmid.

The EC₅₀ value of the compound of the present patent was determined by the above G-402 experiment system. The activity of the CYP11B2 enzyme was determined under the conditions of 1 µM 11-deoxycorticosterone and a variable amount of the inhibitor, and the activity of the CYP11B1 enzyme was determined under the conditions of 1 µM 11-deoxycortisol and a variable amount of the inhibitor.

| **Example** | **Human CYP11B2 EC₅₀ (nM)** | **Example** | **Human CYP11B2 EC₅₀ (nM)** |
|---|---|---|---|
| 1-A | 70 | 37 | 35 |
| 58 | 48 | 68 | 7 |

| **Example** | **hsF** | **Example** | **hsF** |
|---|---|---|---|
| 1-A | 144x | 10-A | 275x |
| 2-A | 174x | 48-P2B | 326x |
| 60 | > 1279x | 68 | 394x |

Note: hSF, the abbreviation of human selective factor, refers to the selectivity of the compound for CYP11B2 over CYP11B1. The numerical value of hSF is the ratio of CYP11B1 EC50 to CYP11B2 EC50.

As can be seen from the above data, the compound of the present invention has better CYP11B2 activity and also higher selectivity compared with CYP11B1 enzyme.

### Test Example 2. Pharmacokinetic assay in rats

### 1. Study objective:

SD rats were used as test animals to study the pharmacokinetic behavior of the compounds of the present invention in rats (plasma) after oral administration.

### 2. Test scheme

### 2.1 Test drugs:

Compounds of the present invention, made in house.

### 2.2 Test animals:

3 SD rats per group, male.

### 2.3 Drug formulation:

Formulation of drug for oral administration: 0.5% CMC-Na (1% Tween 80)
5 g of sodium carboxymethyl cellulose (CMC-Na, viscosity: 800-1200 Cps) was weighed and dissolved in 1000 mL of purified water, and 10 g of Tween 80 was added. These materials were uniformly mixed to form a clear solution.

The example compounds were weighed and dissolved in the solution and the mixture was shaken well and ultrasonicated for 15 min to obtain a colorless clear solution with a concentration of 0.5 mg/mL.
Formulation of drug for intravenous administration: 5% DMSO + 10% Solutol HS15 + 85% PBS

The example compounds were weighed, and 5% DMSO was added based on the total administration volume; the mixture was vortexed and ultrasonicated for 2 min to ensure complete dissolution; then, 10% Solutol HS15 was added, and the mixture was vortexed and ultrasonicated for 2 min to achieve complete dissolution; finally, 85% PBS was added, and the mixture was vortexed and ultrasonicated for 5 min; the resulting mixture was filtered through a 0.22 µM filter membrane to obtain a colorless, transparent, and clear solution with a concentration of 0.2 mg/mL.

### 2.4 Administration:

After overnight fasting, 3 male SD rats per group were administered orally (PO) at a dose of 5 mg/kg with an administration volume of 10 mL/kg.

After overnight fasting, 3 male SD rats per group were administered intravenously (IV) at a dose of 1 mg/kg with an administration volume of 5 mL/kg.

### 2.5 Sample collection:

0.2 mL of blood was collected from the jugular vein of the experimental animals before administration and at 0.25 h, 0.5 h, 1.0 h, 2.0 h, 4.0 h, 6.0 h, 8.0 h, and 24.0 h after administration and placed in an EDTA-2K test tube, and the test tube was centrifuged at 8000 rpm for 6 min at 4°C to separate the plasma, which was stored at -80°C; and the animals were fed 4 h after administration.

3 Experimental results: The final determination results were obtained by using LCMS/MS method.

| Example | tₘₐₓ (h) | Cₘₐₓ (ng/mL) | AUC₀₋ₜ (ng/mL*h) | AUC_{0-∞} (ng/mL*h) | t_{1/2} (h) | MRT_{0-∞} (h) |
|---|---|---|---|---|---|---|
| 1-A | 1.0 | 6333 | 27618 | 27652 | 2.6 | 3.7 |
| 10-A | 2.0 | 2823 | 19245 | 19355 | 3.3 | 4.8 |

As can be seen from the experimental results, the compounds of the present invention have a very good exposure amount in rats.

### Test Example 3. Pharmacokinetic assay in mice

### 1. Study objective:

Balb/c mice were used as test animals to study the pharmacokinetic behavior of the compounds of the present invention in mice (plasma) after oral administration.

### 2. Test scheme

### 2.1 Test drugs:

Compounds of the present invention, made in house.

### 2.2 Test animals:

3 Balb/c mice per group, male; from Shanghai Bk Lab Animal Co., Ltd., with animal production license number (SCXK (Shanghai) 2013-0006 N0.311620400001794).

### 2.3 Drug formulation:

Formulation of drug for oral administration: 0.5% CMC-Na (1% Tween 80)

0.5 g of sodium carboxymethyl cellulose (CMC-Na, viscosity: 800-1200 Cps) was weighed and dissolved in 99 mL of purified water, and 1 ml of Tween 80 was added. These materials were uniformly mixed to form a clear solution.

Examples 1 and 2 were weighed and dissolved in the solution, and the mixtures were shaken well and ultrasonicated for 15 min to obtain colorless clear solutions with a concentration of 0.5 mg/mL.

Formulation of drug for intravenous administration: 5% DMSO + 10% Solutol HS15 + 85% PBS

Examples 1 and 2 were weighed, and 5% of DMSO was added based on the total administration volume; the mixture was vortexed and ultrasonicated for 2 min to ensure complete dissolution; then, 10% Solutol HS15 was added, and the mixture was vortexed and ultrasonicated for 2 min to achieve complete dissolution; finally, 85% PBS was added, and the mixture was vortexed and ultrasonicated for 5 min; the resulting mixture was filtered through a 0.22 µM filter membrane to obtain a colorless, transparent, and clear solution with a concentration of 0.2 mg/mL.

### 2.4 Administration:

After overnight fasting, 3 male Balb/c mice per group were administered orally (PO) at a dose of 5 mg/kg with an administration volume of 10 mL/kg.

After overnight fasting, 3 male Balb/c mice per group were administered intravenously (IV) at a dose of 1 mg/kg with an administration volume of 5 mL/kg.

### 2.5 Sample collection:

0.04 mL of blood was collected from the orbit of the experimental animals before administration and at 0.25 h, 0.5 h, 1.0 h, 2.0 h, 4.0 h, 6.0 h, 8.0 h, and 24.0 h after administration and placed in an EDTA-2K test tube, and the test tube was centrifuged at 8000 rpm for 6 min at 4°C to separate the plasma, which was stored at -80°C; and the animals were fed 4 h after administration.

3 Experimental results: The final determination results were obtained by using LCMS/MS method.

| Example | tₘₐₓ (h) | Cₘₐₓ (ng/mL) | AUC₀₋ₜ (ng/mL*h) | AUC_{0-∞} (ng/mL*h) | t_{1/2} (h) | MRT_{0-∞} (h) |
|---|---|---|---|---|---|---|
| 1-A | 0.3 | 3770 | 20796 | 20843 | 2.7 | 4.7 |

As can be seen from the experimental results, the compounds of the present invention have a very good exposure amount in mice.

### Test Example 4. In vivo efficacy assay

Male cynomolgus monkeys were gavaged (P.O.) with the compounds to be tested and divided into 0 mpk (vehicle group), 0.1 mpk, 0.3 mpk, 1 mpk, and 3 mpk dose groups. Each group was given 5 ug/kg ACTH intravenous injection (I.V.) after one hour. Blood samples were collected at -1 hour, 0 hour, 0.5 hours, 1.0 hour, 1.5 hours, 2 hours, and 3 hours after intravenous injection of ACTH. The volume was about 0.5 mL at each time point. The samples were centrifuged (3200 g, at 2-8°C for 10 minutes) within one hour after collection. The contents of the plasma aldosterone, cortisol, corticosterone, 11-deoxycortisol, and 11-deoxycorticosterone at each time point were then analyzed by LC/MS-MS method. The experimental results were as follows:

| | Dose | Aldosterone (ng/mL) | | Cortisol (ng/mL) | |
|---|---|---|---|---|---|
| | | 0 | 0.5 h | 0 | 1 h |
| Vehicle | 0 | 0.57 | 0.82 | 194 | 301 |
| Example 1-A | 0.1 mpk | 0.38 | 0.33 | 246 | 360 |
| | 0.3 mpk | 0.23 | 0.17 | 230 | 355 |
| | 1 mpk | 0.12 | 0.15 | 215 | 315 |
| | 3 mpk | 0.13 | 0.09 | 225 | 326 |

As can be seen from the experimental results, the compounds of the present invention can effectively reduce the content of aldosterone without causing significant changes in hormones such as cortisol.

## Claims

1. A compound represented by general formula (I), or a stereoisomer or a pharmaceutically acceptable salt thereof: 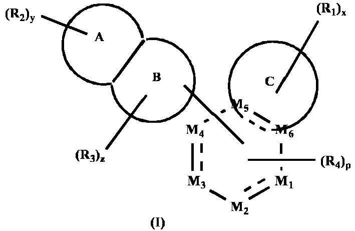 **characterized in that**: 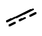
is a single bond or a double bond;
ring A is 5- to 7-membered heterocyclyl containing 2-3 heteroatoms selected from N, O, or S, and ring A contains at least one oxygen atom;
ring B is 5- to 6-membered heteroaryl or phenyl;
ring C is cycloalkyl, heteroaryl, heterocyclyl, or absent;
M₁, M₂, M₄, M₅, and M₆ are each independently selected from N, C, NH, or CH;
M₃ is a bond, N, or CH;
each R₁ is independently selected from cycloalkyl, heterocyclyl, cycloalkyloxy, heterocyclyloxy, cycloalkylamino, heterocyclylamino, cycloalkylthio, heterocyclylthio, -C(O)(CH₂)ₙR_{b}, -NRₐC(O)(CH₂)ₙR_{b}, -O(CH₂)ₙR_{d}, -NH(CH₂)ₙR_{b}, - S(CH₂)ₙR_{b}, -S(O)₂(CH₂)ₙR_{b}, -S(O)(NH)(CH₂)ₙR_{b}, -NRₐS(O)₂(CH₂)ₙR_{b}, - NRₐS(O)(NH)(CH₂)ₙR_{b}, or -NS(O)(CH₂)ₙRₐR_{b}, wherein optionally the cycloalkyl, heterocyclyl, cycloalkyloxy, heterocyclyloxy, cycloalkylamino, heterocyclylamino, cycloalkylthio, or heterocyclylthio is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, hydroxyalkyl, - C(O)(CH₂)ₙR_{b}, -S(O)₂(CH₂)ₙR_{b}, -S(O)(NH)(CH₂)ₙR_{b}, -S(O)₂(CH₂)ₙR_{b}, or - NRₐS(O)(NH)(CH₂)ₙR_{b};
or any two R₁, together with adjacent carbon atoms, form 3- to 8-membered cycloalkyl or 4- to 7-membered heterocyclyl, wherein optionally the 3- to 8-membered cycloalkyl or 4- to 7-membered heterocyclyl is further substituted with oxo, -C(O)R_{b}, -NRₐC(O)R_{b}, -S(O)₂R_{b}, -S(O)(NH)R_{b}, -NRₐS(O)₂R_{b}, or - NRₐS(O)(NH)R_{b};
Rₐ or R_{b} is each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, aryl, heteroaryl, or heterocyclyl, wherein optionally the cycloalkyl, aryl, heteroaryl, or heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, or -S(O)₂alkyl;
R₂, R₃, or R₄ is each independently selected from hydrogen, deuterium, oxo, halogen, amino, hydroxyl, cyano, nitro, alkyl, alkenyl, alkynyl, deuteroalkyl, haloalkyl, alkoxy, haloalkoxy, hydroxyalkyl, cycloalkyl, heterocyclyl, aryl, or heteroaryl, wherein the alkyl, alkenyl, alkynyl, deuteroalkyl, haloalkyl, alkoxy, haloalkoxy, hydroxyalkyl, cycloalkyl, heterocyclyl, aryl, heteroaryl, - NRₐC(O)(CH₂)ₙR_{b}, or -C(O)NRₐ(CH₂)ₙR_{b} can be optionally further substituted;
or R₂ and R₃, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
or any two R₂, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
or R₂ and R₄, together with adjacent atoms, form 5- to 14-membered cycloalkyl, 5- to 14-membered heteroaryl, or 5-14-membered heterocyclyl, wherein optionally the 5- to 14-membered cycloalkyl, 5- to 14-membered heteroaryl, or 5-14-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
p, x, y, and z are each independently selected from 1, 2, 3, or 4; and
n is selected from 0, 1, 2, or 3.

2. The compound, or the stereoisomer or the pharmaceutically acceptable salt thereof according to claim 1, **characterized in that** 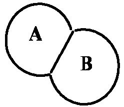 is selected from 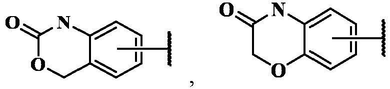 or

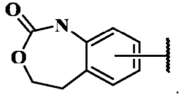

3. The compound, or the stereoisomer or the pharmaceutically acceptable salt thereof according to claim 1, **characterized in that** ring C is absent or selected from 3- to 10-membered cycloalkyl, or 4- to 10-membered heterocyclyl containing 1-3 moieties selected from C(O), N, O, S, SO₂, or SONH;
preferably, ring C is selected from 5- to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, and 7- to 10-membered bicyclic heterocyclyl containing 1-3 moieties selected from C(O), N, O, S, SO₂, or SONH;
more preferably, ring C is selected from the following group:

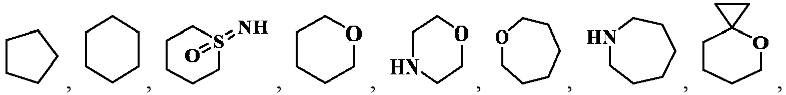

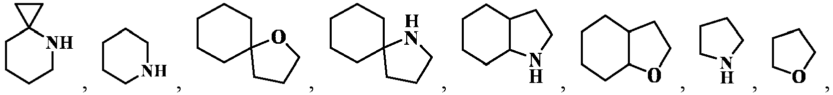

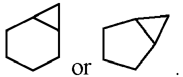

4. The compound, or the stereoisomer or the pharmaceutically acceptable salt thereof according to claim 1, **characterized in that** each R₁ is independently selected from 3- to 10-membered cycloalkyl, 4- to 10-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, 4- to 8-membered heterocyclylthio, -C(O)(CH₂)ₙR_{b}, - NRₐC(O)(CH₂)ₙR_{b}, -O(CH₂)ₙR_{d}, -NH(CH₂)ₙR_{d}, -S(CH₂)ₙR_{b}, -S(O)₂(CH₂)ₙR_{b}, - S(O)(NH)(CH₂)ₙR_{b}, -NRₐS(O)₂(CH₂)ₙR_{b}, or -NRₐS(O)(NH)(CH₂)ₙR_{b}, wherein optionally the 3- to 10-membered cycloalkyl, 4- to 10-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, or 4- to 8-membered heterocyclylthio is further substituted with one or more substituents selected from oxo, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, -C(O)(CH₂)ₙR_{b}, -S(O)₂(CH₂)ₙR_{b}, - S(O)(NH)(CH₂)ₙR_{b}, -S(O)₂(CH₂)ₙR_{b}, or -NRₐS(O)(NH)(CH₂)ₙR_{b};
Rₐ or R_{b} is each independently selected from hydrogen, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋₆hydroxyalkyl, 3- to 8-membered cycloalkyl, 5- to 8-membered heteroaryl containing 1-3 moieties selected from C(O), N, O, or S, or 4- to 8-membered heterocyclyl containing 1-3 moieties selected from C(O), N, O, or S, wherein optionally the 3- to 8-membered cycloalkyl, 5- to 8-membered heteroaryl containing 1-3 moieties selected from C(O), N, O, or S, or 4-to 8-membered heterocyclyl containing 1-3 moieties selected from C(O), N, O, or S is further substituted with one or more substituents selected from oxo, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋₆hydroxyalkyl, 3- to 6-membered cycloalkyl, or -SO₂-C₁₋₆alkyl;
preferably, each R₁ is independently selected from 3- to 10-membered cycloalkyl, 4- to 10-membered heterocyclyl containing 1-3 moieties selected from N, O, S, SO₂, or SONH, 3- to 8-membered cycloalkyloxy, 3- to 8-membered cycloalkylamino, 4- to 8-membered heterocyclyloxy containing 1-3 moieties selected from N, O, S, SO₂, or SONH, 4- to 8-membered heterocyclylamino containing 1-3 moieties selected from N, O, S, SO₂, or SONH, -C(O)(CH₂)ₙR_{b}, - NRₐC(O)(CH₂)ₙR_{b}, -O(CH₂)ₙR_{b}, -S(O)₂(CH₂)ₙR_{b}, -S(O)(NH)(CH₂)ₙR_{b}, - NRₐS(O)₂(CH₂)ₙR_{b}, or -NRₐS(O)(NH)(CH₂)ₙR_{b}, wherein optionally the 3- to 10-membered cycloalkyl, 4- to 10-membered heterocyclyl containing 1-3 moieties selected from N, O, S, SO₂, or SONH, 3- to 8-membered cycloalkyloxy, 3- to 8-membered cycloalkylamino, 4- to 8-membered heterocyclyloxy containing 1-3 moieties selected from N, O, S, SO₂, or SONH, or 4- to 8-membered heterocyclylamino containing 1-3 moieties selected from N, O, S, SO₂, or SONH is further substituted with one or more substituents selected from oxo, -C(O)(CH₂)ₙR_{b}, -S(O)₂(CH₂)ₙR_{b}, -S(O)(NH)(CH₂)ₙR_{b}, -S(O)₂(CH₂)ₙR_{b}, or -NRₐS(O)(NH)(CH₂)ₙR_{b}; and
Rₐ or R_{b} is each independently selected from hydrogen, deuterium, halogen, hydroxyl, cyano, C₁₋₃alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 5- to 8-membered heteroaryl containing 1-3 heteroatoms selected from N, O, or S, or 4- to 8-membered heterocyclyl containing 1-3 moieties selected from C(O), N, O, or S is further substituted with one or more substituents selected from oxo, deuterium, halogen, amino, hydroxyl, cyano, nitro, C₁₋₃alkyl, C₂₋₄alkenyl, C₂₋₄alkynyl, C₁₋₃deuteroalkyl, C₁₋₃haloalkyl, C₁₋₃alkoxy, C₁₋₃haloalkoxy, C₁₋₃hydroxyalkyl, 3- to 6-membered cycloalkyl, or -SO₂-C₁₋₃alkyl.

5. The compound, or the stereoisomer or the pharmaceutically acceptable salt thereof according to claim 1 or 4, **characterized in that** R₂, R₃, or R₄ is each independently selected from hydrogen, deuterium, halogen, amino, hydroxyl, cyano, nitro, C₁₋₆alkyl, C₂₋₆alkenyl, C₂₋₆alkynyl, C₁₋₆deuteroalkyl, C₁₋₆haloalkyl, C₁₋₆alkoxy, C₁₋₆haloalkoxy, C₁₋₆deuteroalkoxy, C₁₋₆hydroxyalkyl, 3- to 8-membered cycloalkyl, 3- to 8-membered cycloalkyloxy, 3- to 8-membered cycloalkylamino, C₆₋₁₀aryl, 5-to 6-membered heteroaryl containing 1-3 heteroatoms selected from N, O, or S or 4-to 8-membered heterocyclyl containing 1-3 moieties selected from C(O), N, O, or S, -NRₐR_{b}, -NRₐC(O)R_{b}, or - C(O)NRₐR_{b};
preferably, R₂, R₃, or R₄ is each independently selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, or C₃₋₆cycloalkyloxy.

6. The compound, or the stereoisomer or the pharmaceutically acceptable salt thereof according to any one of claims 1-5, **characterized in that** the compound, or the stereoisomer or the pharmaceutically acceptable salt thereof is further a compound represented by general formula (II-a), (II-b), or (II-c), or a stereoisomer or a pharmaceutically acceptable salt thereof: 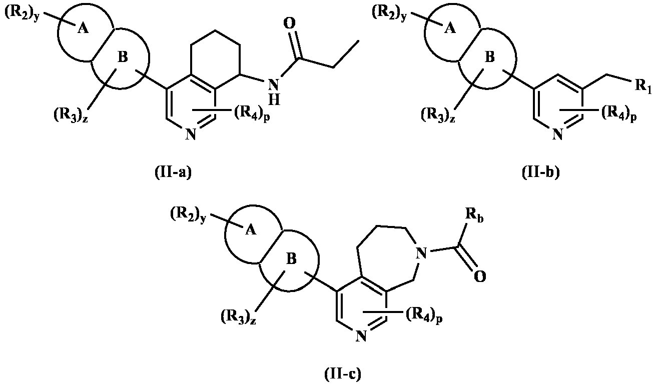 wherein,
ring A is 6-membered heterocyclyl;
ring B is selected from phenyl, or 5- to 6-membered heteroaryl containing 1-3 heteroatoms selected from N, O, or S;
R₂, R₃, or R₄ is each independently selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, -NRₐC(O)R_{b}, or -C(O)NRₐR_{b}; and
Rₐ or R_{b} is each independently selected from hydrogen, deuterium, halogen, hydroxyl, cyano, C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S.

7. The compound, or the stereoisomer or the pharmaceutically acceptable salt thereof according to any one of claims 1-5, **characterized in that** the compound, or the stereoisomer or the pharmaceutically acceptable salt thereof is further a compound represented by general formula (III-b)-(III-c), or a stereoisomer or a pharmaceutically acceptable salt thereof: 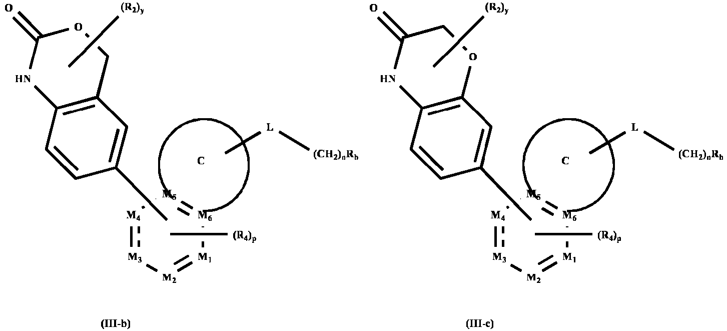
ring C is selected from 5- to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, and 7- to 10-membered bicyclic heterocyclyl containing 1-3 moieties selected from C(O), N, O, S, SO₂, or SONH;
or ring C is absent;
L is selected from a bond, -O-, -R_{c}C(O)-, -R_{c}S(O)NH-, or -R_{c}S(O)₂, preferably -NHC(O)-;
R_{c} is selected from a bond, NH, 3- to 8-membered cycloalkyl, 4- to 8-membered heterocyclyl containing 1-3 moieties selected from N, O, S, SO₂, or SONH, 3- to 8-membered cycloalkyloxy, or 4- to 8-membered heterocyclyloxy containing 1-3 moieties selected from N, O, S, SO₂, or SONH;
R₂, R₃, or R₄ is each independently selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, or C₃₋₆cycloalkyl;
R_{b} is selected from hydrogen, deuterium, C₁₋₃alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂, or SONH; and
y is 1, 2, or 3.

8. The compound, or the stereoisomer or the pharmaceutically acceptable salt thereof according to claim 1 or 7, **characterized in that** 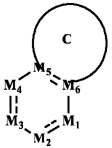 is selected from the following groups: 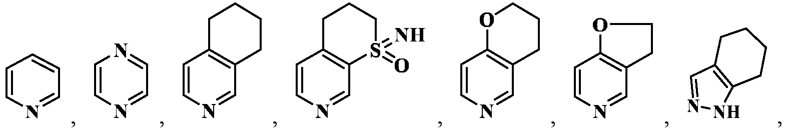 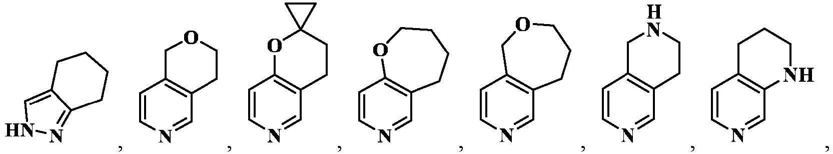 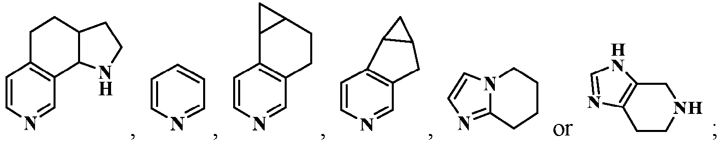 preferably

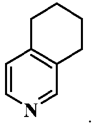

9. The compound, or the stereoisomer or the pharmaceutically acceptable salt thereof according to any one of claims 1-5, **characterized in that** the compound, or the stereoisomer or the pharmaceutically acceptable salt thereof is further a compound represented by general formula (IV), or a stereoisomer or a pharmaceutically acceptable salt thereof: 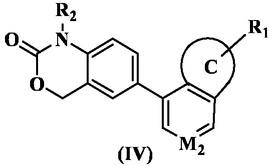 wherein:
M₂ is selected from N or CH;
ring C is selected from 5- to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, 7- to 10-membered bicyclic heterocyclyl containing 1-3 moieties selected from C(O), N, O, S, SO₂, or SONH, or absent; preferably, ring C is selected from 6-membered monocyclic cycloalkyl;
R₁ is selected from -C(O)R_{b}, -NRₐC(O)R_{b}, -OR_{b}, -S(O)₂R_{b}, -S(O)(NH)R_{b}, - NRₐS(O)₂R_{b}, -NRₐS(O)(NH)R_{b}, or 4- to 8-membered heterocyclyl, wherein the 4-to 8-membered nitrogen-containing heterocyclyl is optionally further substituted with -C(O)R_{b} or -S(O)₂R_{b};
Rₐ or R_{b} is each independently selected from hydrogen, deuterium, halogen, hydroxyl, cyano, C₁₋₃alkyl, C₁₋₃deuteroalkyl, C₁₋₃haloalkyl, C₁₋₃alkoxy, C₁₋₃haloalkoxy, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S; and
R₂ is selected from hydrogen, deuterium, halogen, C₁₋₃alkyl, C₁₋₃deuteroalkyl, C₁₋₃haloalkyl, C₁₋₃alkoxy, C₁₋₃haloalkoxy, C₁₋₃hydroxyalkyl, or C₃₋₆cycloalkyloxy.

10. A compound as follows, or a stereoisomer or a pharmaceutically acceptable salt thereof:

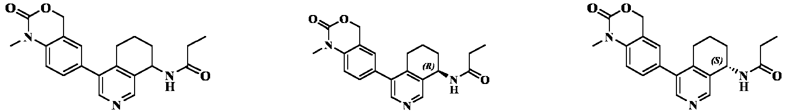

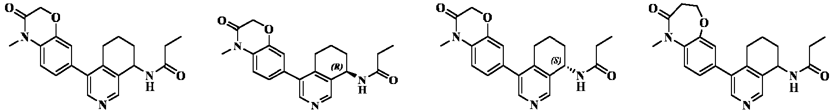

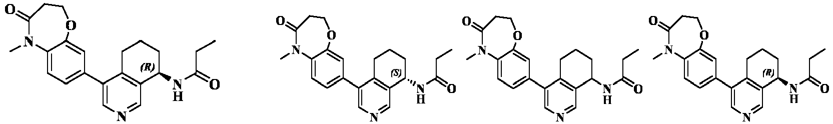

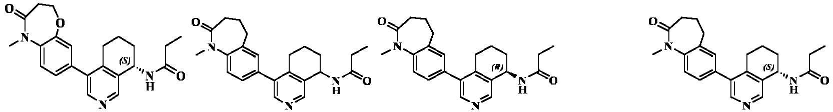

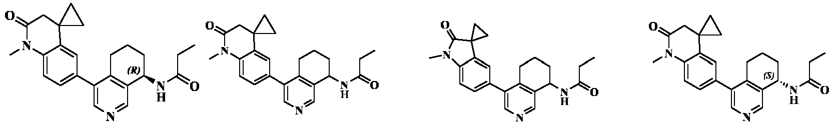

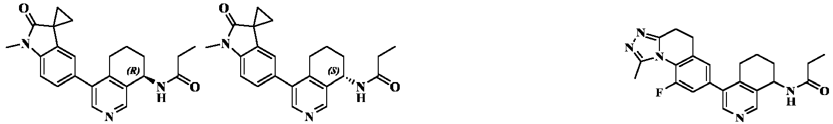

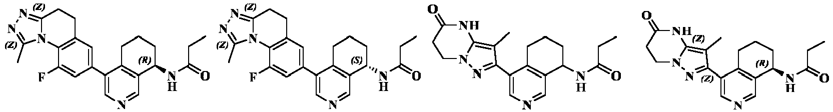

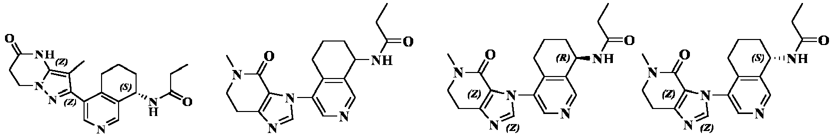

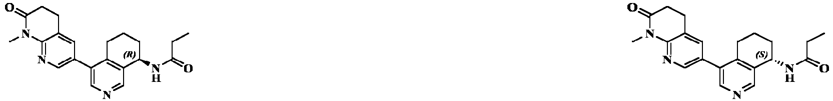

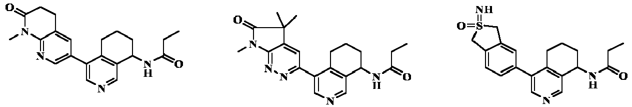

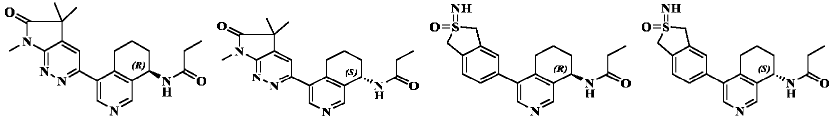

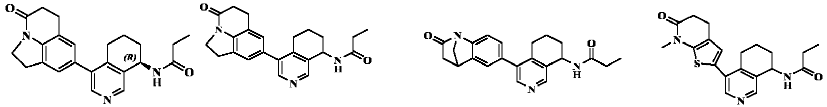

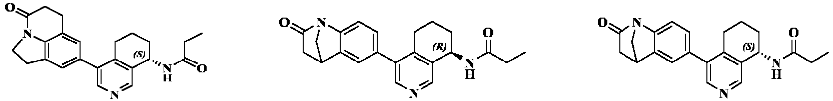

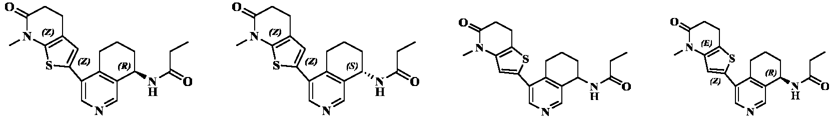

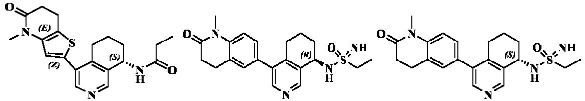

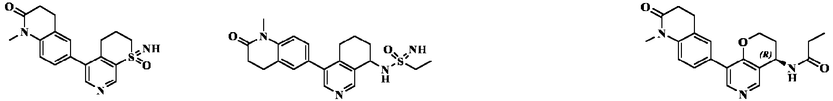

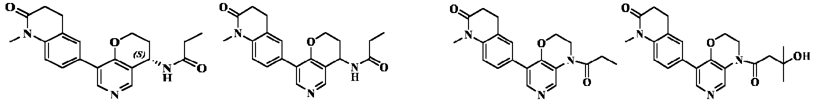

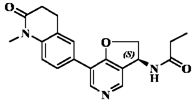

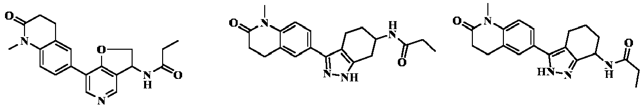

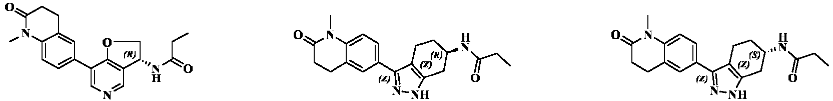

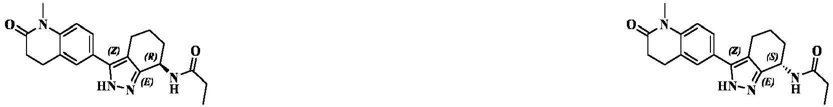

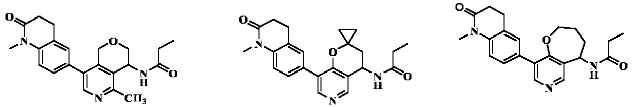

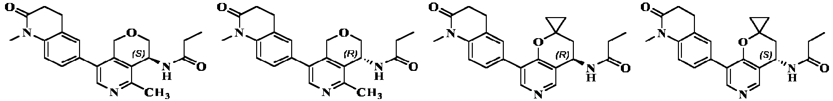

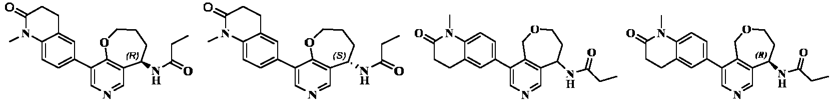

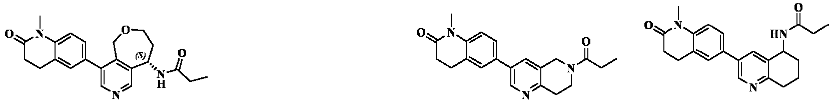

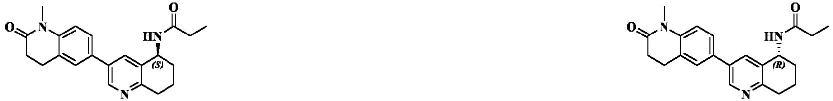

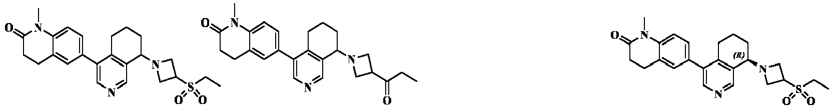

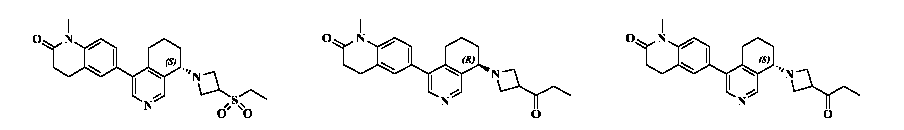

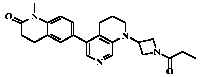

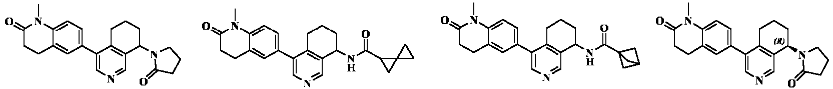

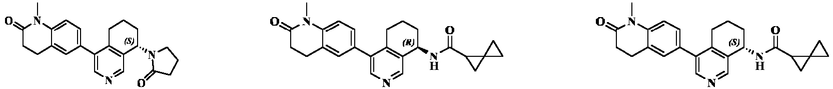

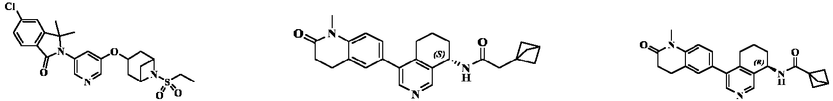

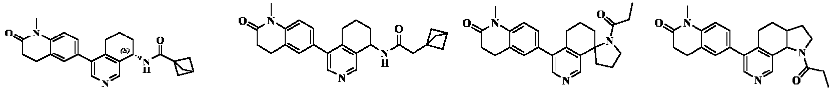

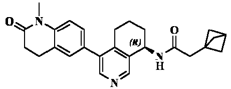

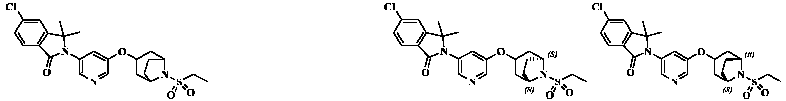

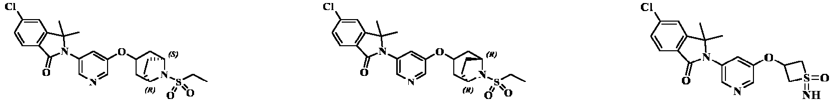

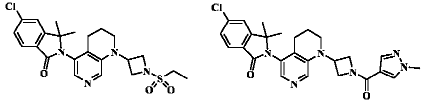

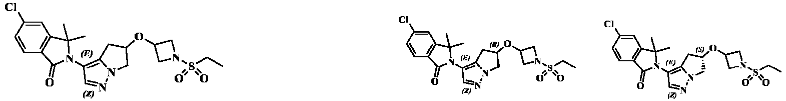

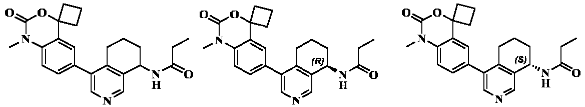

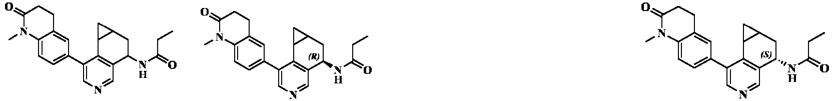

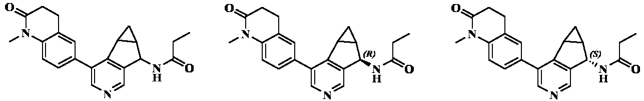

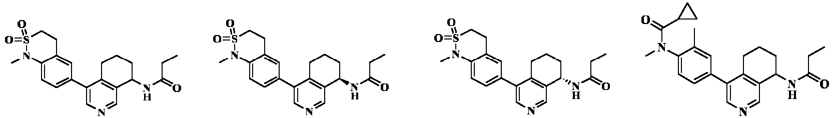

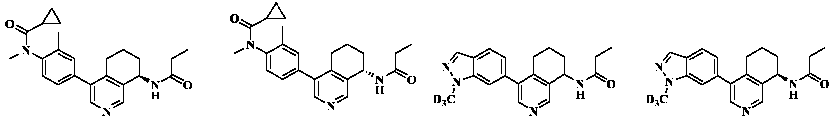

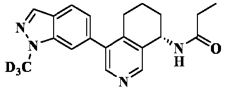

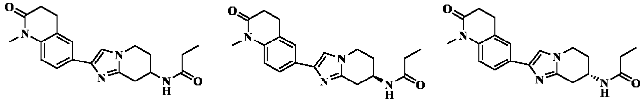

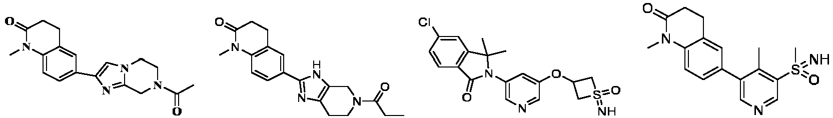

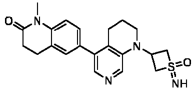

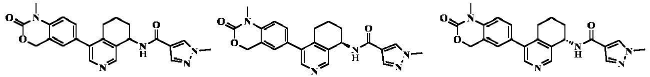

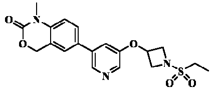

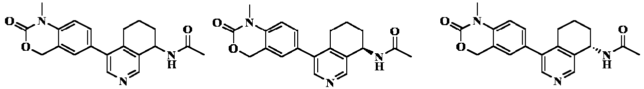

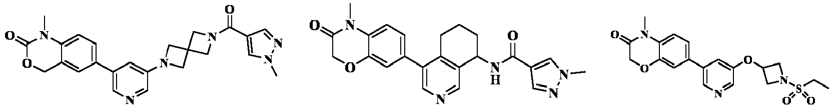

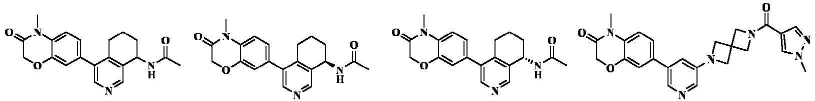

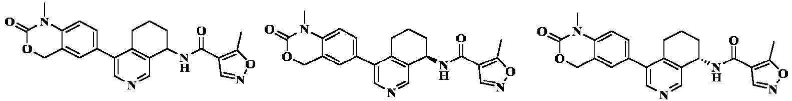

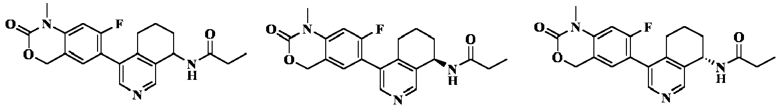

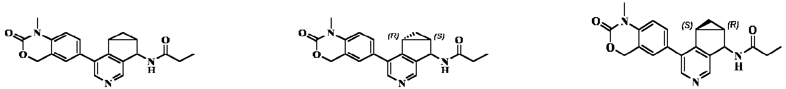

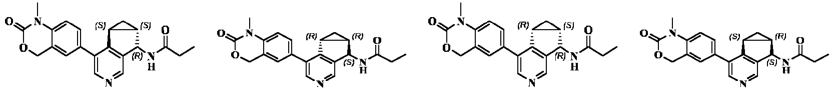

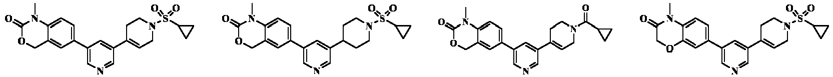

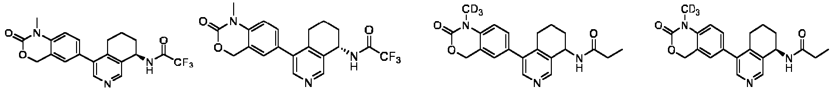

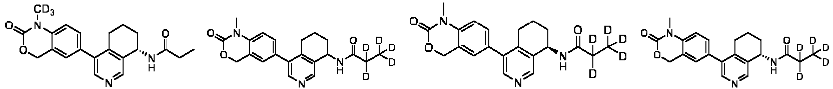

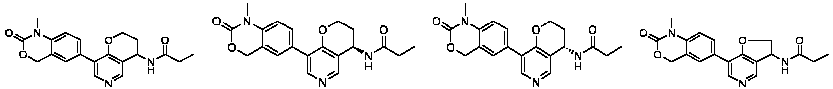

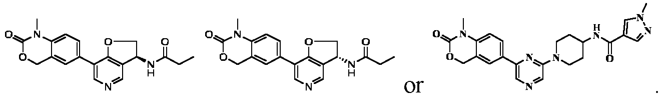

11. A pharmaceutical composition, comprising a therapeutically effective dose of the compound, or the stereoisomer or the pharmaceutically acceptable salt thereof according to any one of claims 1-10, and one or more pharmaceutically acceptable carriers, diluents, or excipients.

12. Use of the compound according to any one of claims 1-10 or the pharmaceutical composition according to claim 11 in the preparation of a medicament for treating or preventing kidney disease, kidney or cardiac fibrosis, diabetic nephropathy, congestive heart failure, hypertension, aldosteronism, and Cushing's syndrome, **characterized in that** preferably, the hypertension is refractory hypertension, the nephropathy is chronic kidney disease, and the aldosteronism is primary aldosteronism.

13. Use of the compound of the general formula, or the stereoisomer or the pharmaceutically acceptable salt thereof according to any one of claims 1-10 or the pharmaceutical composition according to claim 11 in the preparation of a medicament for treating a disease related to CYP11B2.

14. A method for preparing the compound represented by general formula (I), or the stereoisomer or the pharmaceutically acceptable salt thereof according to claim 1, **characterized by** comprising the following steps: 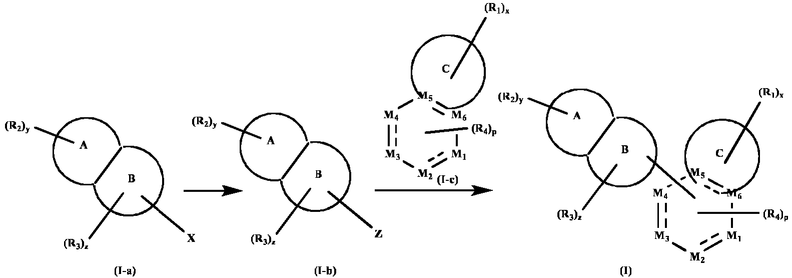
reacting compound (I-a) with a boron compound to prepare compound (I-b), or a stereoisomer or a pharmaceutically acceptable salt thereof, and performing a coupling reaction with compound (I-c), or a stereoisomer or a pharmaceutically acceptable salt thereof to prepare the compound represented by general formula (I), or the stereoisomer or the pharmaceutically acceptable salt thereof, wherein
a catalyst for the coupling reaction is palladium reagent, preferably palladium acetate, tetrakis(triphenylphosphine)palladium, bis(dibenzylideneacetone)palladium, tris(dibenzylideneacetone)dipalladium, or [1,1'-bis(diphenylphosphino)ferrocene]palladium dichloride;
X is halogen, preferably bromine;
Z is a boron-containing compound, preferably dioxaborolanyl; and
ring A, ring B, ring C, R₁, R₂, R₃, R₄, M₁, M₂, M₃, M₄, M₅, M₆, x, y, z, and p are as defined in claim 1.
